# EUROPÄISCHE PATENTSCHRIFT

(11) **EP 1 732 930 B1**
(45) Veröffentlichungstag und Bekanntmachung des Hinweises auf die Patenterteilung: **02.05.2012**
(21) Anmeldenummer: 05728341.8
(22) Anmeldetag: 11.03.2005
(51) Int. Cl.: C07D 491/10, C07D 207/36, C07D 209/54, C07D 309/14, C07C 235/12, C07C 235/10, C07D 307/94, C07C 69/74, C07C 69/612, A01N 43/36, A01N 43/08, A01N 43/12

(54) **2,4,6-PHENYLSUBSTITUIERTE CYCLISCHE KETOENOLE**
2,4,6-PHENYLSUBSTITUTED CYCLIC KETOENOLES
CETOENOLS CYCLIQUES 2,4,6-PHENYLSUBSTITUES

(30) Priorität: 25.03.2004 DE 102004014620
(43) Veröffentlichungstag der Anmeldung: 20.12.2006
(73) Patentinhaber: Bayer CropScience AG, 40789 Monheim (DE)
(72) Erfinder: BRETSCHNEIDER, Thomas, 53797 Lohmar (DE); FISCHER, Reiner, 40789 Monheim (DE); GAERTZEN, Oliver, 50668 Köln (DE); KUNZ, Klaus, 40625 Düsseldorf (DE); LEHR, Stefan, 65835 Liederbach (DE); FEUCHT, Dieter, 65760 Eschborn (DE); LÖSEL, Peter, 51371 Leverkusen (DE); MALSAM, Olga, 51503 Rösrath (DE); BOJACK, Guido, 65207 Wiesbaden (DE); ARNOLD, Christian, 40764 Langenfeld (DE); AULER, Thomas, 42799 Leichlingen (DE); HILLS, Martin, Jeffrey, 65510 Idstein (DE); KEHNE, Heinz, 65719 Hofheim (DE); ROSINGER, Chris, 65719 Hofheim (DE)
(86) Internationale Anmeldenummer: PCT/EP2005/002605
(87) Internationale Veröffentlichungsnummer: WO 2005/092897

(56) Entgegenhaltungen:
- WO-A-01/17972
- US-A- 5 683 965
- US-B1- 6 316 486
- US-B1- 6 358 887
- US-B1- 6 410 480
- CLIVE D L J ET AL: "Synthesis of natural (-)-hamigeran B" TETRAHEDRON LETTERS, ELSEVIER SCIENCE PUBLISHERS, AMSTERDAM, NL, Bd. 44, Nr. 42, 13. Oktober 2003 (2003-10-13), Seiten 7731-7733, XP004457079 ISSN: 0040-4039

## Beschreibung

Die vorliegende Erfindung betrifft neue 2,4,6-phenylsubstituierte cyclische Ketoenole, mehrere Verfahren zu ihrer Herstellung und ihre Verwendung als Schädlingsbekämpfungsmittel und/oder Herbizide.

Die Erfindung betrifft außerdem neue selektiv-herbizide Wirkstoffkombinationen, die 2,4,6-phenylsubstituierte cyclische Ketoenole einerseits und zumindest eine die Kulturpflanzenverträglichkeit verbessernde Verbindung andererseits enthalten und mit besonders gutem Erfolg zur selektiven Unkrautbekämpfung in verschiedenen Nutzpflanzenkulturen verwendet werden können.

Von 3-Acyl-pyrrolidin-2,4-dionen sind pharmazeutische Eigenschaften vorbeschrieben (S. Suzuki et al. Chem. Pharm. Bull. 15 1120 (1967)). Weiterhin wurden N-Phenylpyrrolidin-2,4-dione von R. Schmierer und H. Mildenberger (Liebigs Ann. Chem. 1985, 1095) synthetisiert. Eine biologische Wirksamkeit dieser Verbindungen wurde nicht beschrieben.

In EP-A-0 262 399 und GB-A-2 266 888 werden ähnlich strukturierte Verbindungen (3-Arylpyrrolidin-2,4-dione) offenbart, von denen jedoch keine herbizide, insektizide oder akarizide Wirkung bekannt geworden ist. Bekannt mit herbizider, insektizider oder akarizider Wirkung sind unsubstituierte, bicyclische 3-Aryl-pyrrolidin-2,4-dion-Derivate (EP-A-355 599 und EP-A-415 211 und JP-A-12-053 670) sowie substituierte monocyclische 3-Aryl-pyrrolidin-2,4-dion-Derivate (EP-A-377 893 und EP-A-442 077).

Weiterhin bekannt sind polycyclische 3-Arylpyrrolidin-2,4-dion-Derivate (EP-A-442 073) sowie 1H-Arylpyrrolidin-dion-Derivate (EP-A-456 063, EP-A-521 334, EP-A-596 298, EP-A-613 884, EP-A-613 885, WO 94/01 997, WO 95/26 954, WO 95/20 572, EP-A-0 668 267, WO 96/25 395, WO 96/35 664, WO 97/01 535, WO 97/02 243, WO 97/36 868, WO 97/43275, WO 98/05638, WO 98/06721, WO 98/25928, WO 99/16748, WO 99/24437, WO 99/43649, WO 99/48869, WO 99/55673, WO 01/17972, WO 01/23354, WO 01/74770, WO 03/062244, WO 04/024688, WO 04/007448, WO 04/080962, WO 04/065366, DE-A-10326386)

Es ist bekannt, dass bestimmte substituierte Δ³-Dihydrofuran-2-on-Derivate herbizide Eigenschaften besitzen (vgl. DE-A-4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydro-furanon-(2)) ist ebenfalls in DE-A-4 014 420 beschrieben. Ähnlich strukturierte Verbindungen ohne Angabe einer insektiziden und/oder akariziden Wirksamkeit sind aus der Publikation Campbell et al., J. Chem. Soc., Perkin Trans. 1, 1985, (8) 1567-76 bekannt. Weiterhin sind 3-Aryl-Δ³-dihydrofuranon-Derivate mit herbiziden, akariziden und insektiziden Eigenschaften aus EP-A-528 156, EP-A-0 647 637, WO 95/26 345, WO 96/20 196, WO 96/25 395, WO 96/35 664, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354, WO 01/74770, WO 03/062244, WO 04/024688, WO 04/080962, DE-A-10326386 bekannt. Auch 3-Aryl-Δ³-dihydrothiphen-on-Derivate sind bekannt (WO 95/26 345, 96/25 395, WO 97/01 535, WO 97/02 243, WO 97/36 868, WO 98/05638, WO 98/25928, WO 99/16748, WO 99/43649, WO 99/48869, WO 99/55673, WO 01/17972, WO 01/23354, WO 01/74770, WO 03/062244, WO 04/080962, DE-A-10326386).

Bestimmte, im Phenylring unsubstituierte Phenyl-pyron-Derivate sind bereits bekannt geworden (vgl. A.M. Chirazi, T. Kappe und E. Ziegler, Arch. Pharm. 309, 558 (1976) und K.-H. Boltze und K. Heidenbluth, Chem. Ber. 91, 2849), wobei für diese Verbindungen eine mögliche Verwendbarkeit als Schädlingsbekämpfungsmittel nicht angegeben wird. Im Phenylring substituierte Phenyl-pyron-Derivate mit herbiziden, akariziden und insektiziden Eigenschaften sind in EP-A-588 137, WO 96/25 395, WO 96/35 664, WO 97/01 535, WO 97/02 243, WO 97/16 436, WO 97/19 941, WO 97/36 868, WO 98/05638, WO 99/43649, WO 99/48869, WO 99/55673, WO 01/17972 und WO 01/74770, WO 03/062244, WO 04/080962, DE-A-10326386 beschrieben.

Bestimmte, im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird. Im Phenylring substituierte 5-Phenyl-1,3-thiazin-Derivate mit herbizider, akarizider und insektizider Wirkung sind in WO 94/14 785, WO 96/02 539, WO 96/35 664, WO 97/01 535, WO 97/02 243, WO 97/02 243, WO 97/36 868, WO 99/05638, WO 99/43649, WO 99/48869, WO 99/55673, WO 01/17972 und WO 01/74770, WO 03/062244, WO 04/080962, DE-A-10326386 beschrieben.

Es ist bekannt, dass bestimmte substituierte 2-Arylcyclopentandione herbizide, insektizide und akarizide Eigenschaften besitzen (vgl. z.B. US-4 283 348; 4 338 122; 4 436 666; 4 526 723; 4 551 547; 4 632 698; WO 96/01 798; WO 96/03 366, WO 97/14 667 sowie WO 98/39281, WO 99/43649, WO 99/48869, WO 99/55673, WO 01/17972, WO 01/74770, WO 03/062244, WO 04/080962, DE-A-10326386. Außerdem sind ähnlich substituierte Verbindungen bekannt; 3-Hydroxy-5,5-dimethyl-2-phenylcyclopent-2-en-l-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-en-one aus der Publikation Edwards et al., J. Chem. Soc. S, (1967), 405-9. Eine insektizide oder akarizide Wirkung wird nicht beschrieben. Außerdem ist 2-(2,4,6-Trimethylphenyl)-1,3-indandion aus der Publikation J. Economic Entomology, 66, (1973), 584 und der Offenlegungsschrift DE-A 2 361 084 bekannt, mit Angabe von herbiziden und akariziden Wirkungen.

Es ist bekannt, dass bestimmte substituierte 2-Arylcyclohexandione herbizide, insektizide und akarizide Eigenschaften besitzen (US-4 175 135, 4 209 432, 4 256 657, 4 256 658, 4 256 659, 4 257 858, 4 283 348, 4 303 669, 4 351 666, 4 409 153, 4 436 666, 4 526 723, 4 613 617, 4 659 372, DE-A 2 813 341, sowie Wheeler, T.N., J. Org. Chem. 44, 4906 (1979)), WO 99/43649, WO 99/48869, WO 99/55673, WO 01/17972, WO 01/74770, WO 03/062244, WO 04/080962, DE-A-103263 86.

Es ist bekannt, dass bestimmte substituierte 4-Aryl-pyrazolidin-3,5-dione akarizide, insektizide und herbizide Eigenschaften besitzen (vgl. z.B. WO 92/16 510, EP-A-508 126, WO 96/11 574, WO 96/21 652, WO 99/47525, WO 0/17 351, WO 01/17 352, WO 01/17 353, WO 01/17 972, WO 01/17 973, WO 03/06 2244, WO 03/028 466, WO 04/080962, DE-A-10326386, DE-A-10331675.

Die Wirksamkeit und Wirkungsbreite dieser Verbindungen ist jedoch insbesondere bei niedrigen Aufwandmengen und Konzentrationen nicht immer voll zufriedenstellend. Weiterhin ist die Pflanzenverträglichkeit dieser Verbindungen nicht immer ausreichend.

Es wurden nun neue Verbindungen der Formel (I) gefunden 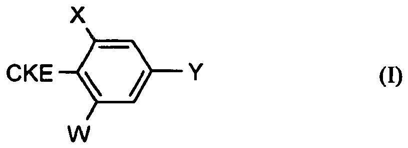 in welcher
- W: steht hervorgehoben für Methoxy, Ethoxy, n-Propoxy, Methoxy-ethoxy, Cyclopropyl-methoxy,
- X: steht hervorgehoben für Chlor,
- Y: steht hervorgehoben für Methyl,
- CKE: steht hervorgehoben für eine der Gruppen

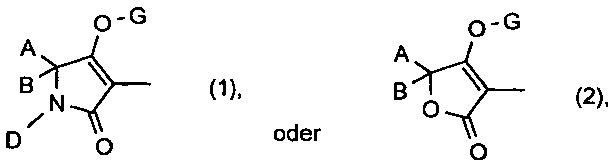

- A: steht hervorgehoben für Methyl, Ethyl, i-Propyl, i-Butyl oder Cyclopropyl,
- B: steht hervorgehoben für Wasserstoff, Methyl oder Ethyl oder
- A, B und: das Kohlenstoffatom an das sie gebunden sind stehen hervorgehoben für gesättigtes C₅-C₆-Cycloalkyl, in welchem gegebenenfalls ein Ringatom durch Sauerstoff ersetzt ist und welches gegebenenfalls einfach durch Methyl oder Methoxy substituiert ist,
- D: steht hervorgehoben für Wasserstoff, Methyl oder Ethyl,
- G: steht hervorgehoben für Wasserstoff (a) oder für eine der Gruppen

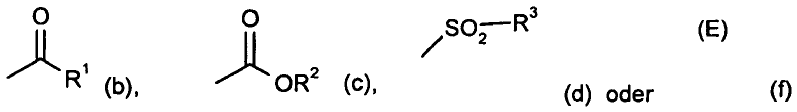

- E: steht für ein Ammoniumion,
- R¹: steht hervorgehoben für C₁-C₆-Alkyl, C₁-C₂-Alkoxy-C₁-alkyl, C₃-C₆-Cycloalkyl, (insbesondere für Cyclopropyl oder Cyclohexyl), einfach durch Chlor substituiertes C₁-C₄-Alkyl oder für gegebenenfalls einfach durch Chlor substituiertes Phenyl,
- R²: steht hervorgehoben für C₁-C₈-Alkyl, C₃-C₆-Alkenyl oder Benzyl,
- R³: steht hervorgehoben für C₁-C₆-Alkyl.

Die Verbindungen der Formel (I) können, auch in Abhängigkeit von der Art der Substituenten, als geometrische und/oder optische Isomere oder Isomerengemische, in unterschiedlicher Zusammensetzung vorliegen, die gegebenenfalls in üblicher Art und Weise getrennt werden können. Sowohl die reinen Isomeren als auch die Isomerengemische, deren Herstellung und Verwendung sowie diese enthaltende Mittel sind Gegenstand der vorliegenden Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Unter Einbeziehung der Bedeutungen (1) bis (2) der Gruppe CKE ergeben sich folgende hauptsächliche Strukturen (I-1) bis (I-2): 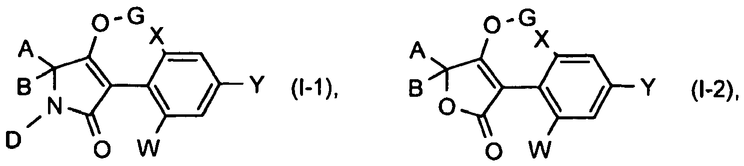 worin
A, B, D, G, W, X und Y die oben angegebene Bedeutung haben.

Unter Einbeziehung der verschiedenen Bedeutungen (a), (b), (c), (d), (f) der Gruppe G ergeben sich folgende hauptsächliche Strukturen (I-1-a) bis (I-1-d), und (I-1-f), wenn CKE für die Gruppe (I) steht, 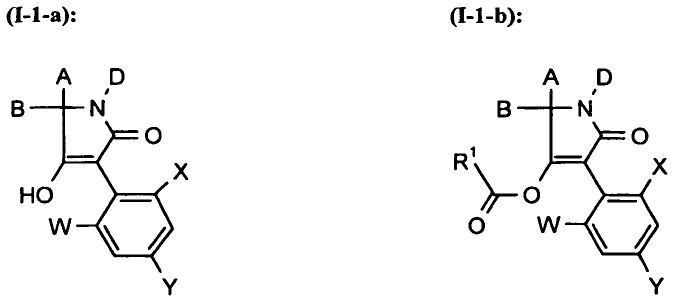 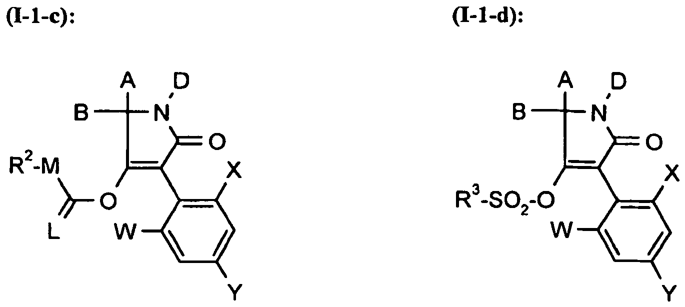 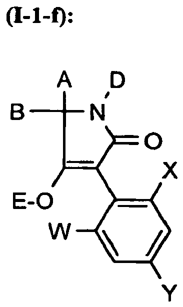 worin

A, B, D, E, L, M, W, X, Y, R¹, R², R³ die oben angegebenen Bedeutungen besitzen.

Unter Einbeziehung der verschiedenen Bedeutungen (a), (b), (c), (d), (f) der Gruppe G ergeben sich folgende hauptsächliche Strukturen (I-2-a) bis (I-2-d und (I-2-f), wenn CKE für die Gruppe (2) steht, 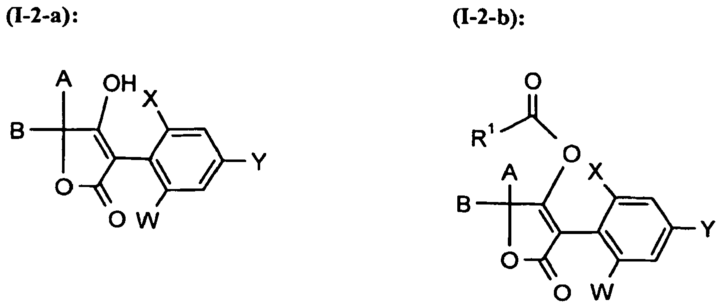 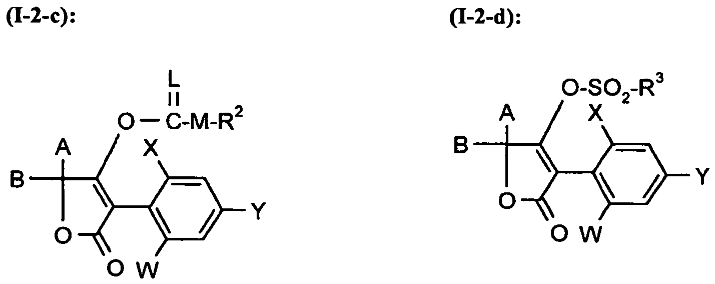 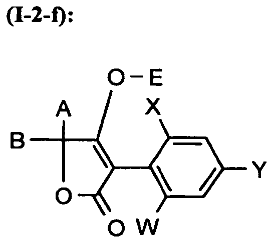 worin
A, B, E, L, M, W, X, Y, R¹, R², R³ die oben angegebene Bedeutung haben.

Weiterhin wurde gefunden, dass man die neuen Verbindungen der Formel (I) nach einem der im folgenden beschriebenen Verfahren erhält:
(A) Man erhält substituierte 3-Phenylpyrrolidin-2,4-dione bzw. deren Enole der Formel (I-1-a) 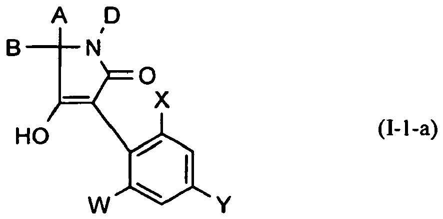 in welcher
   - A, B, D, W, X und Y: die oben angegebenen Bedeutungen haben,
   wenn man
   N-Acylaminosäureester der Formel (II) 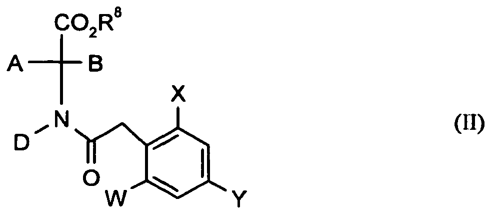 in welcher
   - A, B, D, W, X und Y: die oben angegebenen Bedeutungen haben,
   und
   - R⁸: für Alkyl (bevorzugt C₁-C₆-Alkyl) steht,
   in Gegenwart eines Verdünnungsmittels und in Gegenwart einer Base intramolekular kondensiert.
(B) Außerdem wurde gefunden, dass man substituierte 3-Phenyl-4-hydroxy-Δ³-dihydrofuranon-Derivate der Formel (I-2-a) 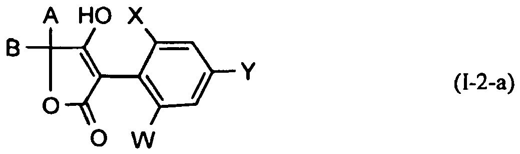 in welcher
   A, B, W, X und Y die oben angegebenen Bedeutungen haben,
   erhält, wenn man
   Carbonsäureester der Formel (III) 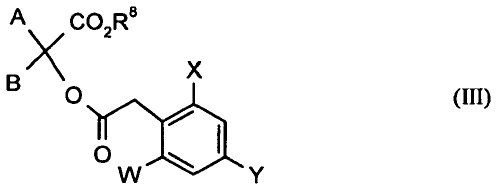 in welcher
   A, B, W, X , Y und R⁸ die oben angegebenen Bedeutungen haben,
   in Gegenwart eines Verdünnungsmittels und in Gegenwart einer Base intramolekular kondensiert.
   Außerdem wurde gefunden
   (I) dass man die Verbindungen der oben gezeigten Formeln (1-I-b) bis (I-2-b), in welchen A, B, D, R¹, W, X, und Y die oben angegebenen Bedeutungen haben, erhält, wenn man Verbindungen der oben gezeigten Formeln (I-1-b) bis (I-2-b), in welchen A, B, D, W, X und Y die oben angegebenen Bedeutungen haben, jeweils
   (α) mit Säurehalogeniden der Formel (XIII) 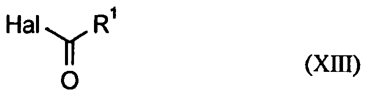 in welcher
      - R¹: die oben angegebene Bedeutung hat und
      - Hal: für Halogen (insbesondere Chlor oder Brom) steht
      oder
   (ß) mit Carbonsäureanhydriden der Formel (XIV)

      R¹-CO-O-CO-R¹ (XIV)

      in welcher
      - R¹: die oben angegebene Bedeutung hat,
      gegebenenfalls in Gegenwart eines Verdünnungsmittels und gegebenenfalls in Gegenwart eines Säurebindemittels umsetzt;
   (J) dass man die Verbindungen der oben gezeigten Formeln (I-1-c) bis (I-2-c), in welchen A, B, D, R², M, W, X und Y die oben angegebenen Bedeutungen haben und L für Sauerstoff steht, erhält, wenn man Verbindungen der oben gezeigten Formeln (I-1-a) bis (1-2-a), in welchen A, B, D, W, X und Y die oben angegebenen Bedeutungen haben, jeweils
      mit Chlorameisensäureestenn der Formel (XV)

      R²-M-CO-Cl (XV)

      in welcher
      - R²: die oben angegebenen Bedeutungen haben,
      - M: steht für Sauerstoff,
      gegebenenfalls in Gegenwart eines Verdünnungsmittels und gegebenenfalls in Gegenwart eines Säurebindemittels umsetzt;
   (L) dass man Verbindungen der oben gezeigten Formeln (1-1-d) bis (1-2-d), in welchen A, B, D, R³, W, X und Y die oben angegebenen Bedeutungen haben, erhält, wenn man Verbindungen der oben gezeigten Formeln (I-1-a) bis (I-2-a), in welchen A, B, D, W, X und Y die oben angegebenen Bedeutungen haben, jeweils
      mit Sulfonsäurechloriden der Formel (XVII)

      R³-SO₂-Cl (XVII)

      in welcher
      - R³: die oben angegebene Bedeutung hat,
      gegebenenfalls in Gegenwart eines Verdünnungsmittels und gegebenenfalls in Gegenwart eines Säurebindemittels umsetzt,
   (N) dass man Verbindungen der oben gezeigten Formeln (I-1-f) bis (I-2-f), in welchen A, B, D, E, W, X und Y die oben angegebenen Bedeutungen haben, erhält, wenn man Verbindungen der Formeln (I-1-a) bis (1-2-a), in welchen A, B, D, W, X und Y die oben angegebenen Bedeutungen haben, jeweils
      mit Metallverbindungen oder Aminen der Formeln (XIX) oder (XX)

      Me(OR¹⁰)ₜ (XIX)

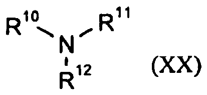

      in welchen
      - Me: für ein ein- oder zweiwertiges Metall (bevorzugt ein Alkali- oder Erdalkalimetall wie Lithium, Natrium, Kalium, Magnesium oder Calcium),
      - t: für die Zahl 1 oder 2 und

      - R¹⁰, R¹¹, R¹²: unabhängig voneinander für Wasserstoff oder Alkyl (bevorzugt C₁-C₈-Alkyl) stehen,
      gegebenenfalls in Gegenwart eines Verdünnungsmittels umsetzt,
   (P) dass man Verbindungen der oben gezeigten Formeln (I-1-a) bis (I-2-a), in welchen A, B, D, W, X und Y die oben angegebene Bedeutung haben, erhält, wenn man Verbindungen der Formeln (I-1-a') bis (1-2-a'), in welchen A, B, D, X und Y die oben genannte Bedeutung haben und W' bevorzugt für Brom steht 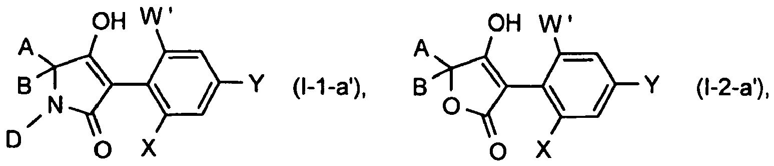 mit Alkoholen der Formel

      W-OH

      in welcher
      - W: die oben angegebene Bedeutung hat, gegebenenfalls in Gegenwart eines Verdünnungsmittel, eines Cu-I-Salzes (z.B. CuBr, CuJ) und einer starken Base (z.B. Natriumhydrid, Kalium-tert.-butylat) umsetzt.

Weiterhin wurde gefunden, dass die neuen Verbindungen der Formel (I) eine sehr gute Wirksamkeit als Schädlingsbekämpfungsmittel, vorzugsweise als Insektizide, Akarizide und Herbizide aufweisen.

Überraschenderweise wurde nun auch gefunden, dass bestimmte substituierte, cyclische Ketoenole bei gemeinsamer Anwendung mit den im weiteren beschriebenen, die Kulturpflanzen-Verträglichkeit verbessernden Verbindungen (Safenem/Antidots) ausgesprochen gut die Schädigung der Kulturpflanzen verhindert und besonders vorteilhaft als breit wirksame Kombinationspräparate zur selektiven Bekämpfung von unerwünschten Pflanzen in Nutzpflanzenkulturen, wie z.B. in Getreide aber auch Mais, Soja und Reis, verwendet werden können.

Gegenstand der Erfindung sind selektiv-herbizide Mittel enthaltend einen wirksamen Gehalt an einer Wirkstoffkombination umfassend als Komponenten
a') mindestens ein substituiertes, cyclisches Ketoenol der Formel (I), in welcher CKE, W, X und Y die oben angegebene Bedeutung haben
   und
(b') zumindest eine die Kulturpflanzen-Verträglichkeit verbessernde Verbindung aus der folgenden Gruppe von Verbindungen:

4-Dichloracetyl-1-oxa-4-aza-spiro[4.5]-decan (AD-67, MON-4660), 1-Dichloracetyl-hexahydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlor-benzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlor-phenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazolcarboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyloxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-di-carboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxa-zolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-Dichlor-phenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chlor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoylsulfamoyl)-phenyl]-3-methyl-harnstoff (alias N-(2-Methoxybenzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methylharnstoff, 1-[4-(N-Naphthylsulfamoyl)-phenyl]-3,3-dimethyl-harnstoff, N-(2-Methoxy-5-methylbenzoyl)-4-(cyclopropylaminocarbonyl)-benzolsulfonamid,
und/oder eine der folgenden durch allgemeine Formeln definierten Verbindungen
der allgemeinen Formel (IIa) 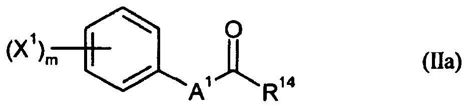 oder der allgemeinen Formel (IIb) 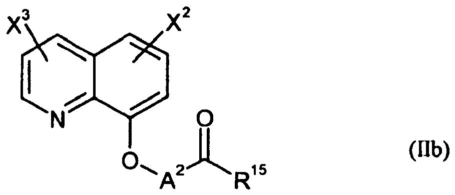 oder der Formel (IIc) 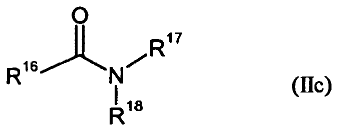 wobei
- m: für eine Zahl 0, 1, 2, 3, 4 oder 5 steht,
- A¹: für eine der nachstehend skizzierten divalenten heterocyclischen Gruppierungen steht,

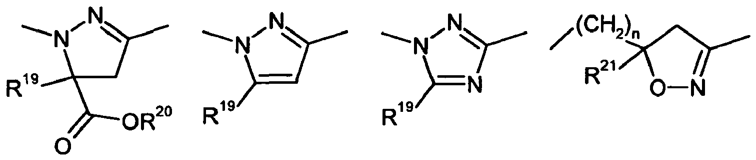

- n: für eine Zahl 0, 1, 2, 3, 4 oder 5 steht,
- A²: für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
- R¹⁴: für Hydroxy, Mercapto, Amino, C₁-C₆-Alkoxy, C₁-C₆-Alkylthio, C₁-C₆-Alkylamino oder Di-(C₁-C₄-alkyl)-amino steht,
- R¹⁵: für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylarnino oder Di-(C₁-C₄-alkyl)-amino steht,
- R¹⁶: für gegebenenfalls durch Fluor, Chlor und/oder Brom substituiertes C₁-C₄-Alkyl steht,
- R¹⁷: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄- alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
- R¹⁸: für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl,
- R¹ und R¹⁸: auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl stehen,
- R¹⁹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- R²⁰: für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
- R²¹: für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
- X¹: für Nitro, Cyano, Halogen, C₁-C₄-Alkyl, C₁-C₄-Halogenalkyl, C₁-C₄-Alkoxy oder C₁-C₄-Halogenalkoxy steht,
- X²: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
- X³: für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen der allgemeinen Formel (IId) 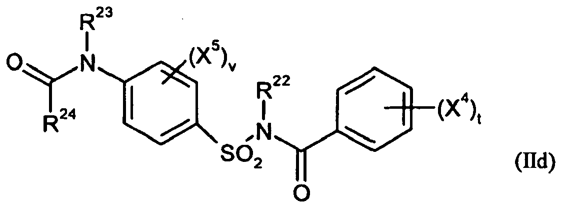 oder der allgemeinen Formel (IIe) 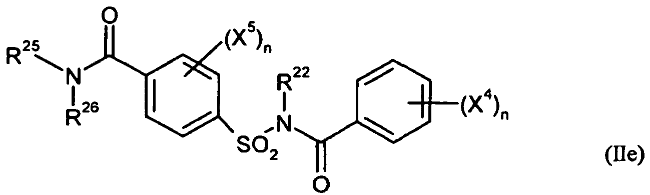 wobei
- t: für eine Zahl 0, 1, 2, 3, 4 oder 5 steht,
- v: für eine Zahl 0, 1, 2, 3, 4 oder 5 steht,
- R²²: für Wasserstoffoder C₁-C₄-Alkyl steht,
- R²³: für Wasserstoff oder C₁-C₄-Alkyl steht,
- R²⁴: für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁-C₄-Alkyl substituiertes C₃-C₆-Cycloalkyl, C₃-C₆-Cycloalkyloxy, C₃-C₆-Cycloalkylthio oder C₃-C₆-Cycloalkylamino steht,
- R²⁵: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
- R²⁶: für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxaalkandiyl steht,
- X⁴: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht, und

- X⁵: für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Erfindungsgemäß hervorgehoben werden die Verbindungen der Formel (I), in welchen eine Kombination der vorstehend als hervorgehoben aufgeführten Bedeutungen vorliegt.

Gesättigte oder ungesättigte Kohlenwasserstoffreste wie Alkyl, Alkandiyl oder Alkenyl können, auch in Verbindung mit Heteroatomen, wie z.B. in Alkoxy, soweit möglich, jeweils geradkettig oder verzweigt sein.

Gegebenenfalls substituierte Reste können, sofern nichts anderes angegeben ist, einfach oder mehrfach substituiert sein, wobei bei Mehrfachsubstitutionen die Substituenten gleich oder verschieden sein können.

Im einzelnen seien außer den bei den Herstellungsbeispielen genannten Verbindungen die folgenden Verbindungen der Formel (I-1-a) genannt:

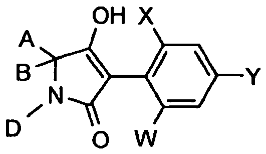

**Tabelle 1: W = OCH₃, X = Cl, Y = CH₃.**

| A | B | D |
|---|---|---|
| CH₃ | H | H |
| C₂H₅ | H | H |
| C₃H₇ | H | H |
| i-C₃H₇ | H | H |
| C₄H₉ | H | H |
| i-C₄H₉ | H | H |
| s-C₄H₉ | H | H |
| t-C₄H₉ | H | H |
| CH₃ | CH₃ | H |
| C₂H₅ | CH₃ | H |
| C₃H₇ | CH₃ | H |
| i-C₃H₇ | CH₃ | H |
| C₄H₉ | CH₃ | H |
| i-C₄H₉ | CH₃ | H |
| s-C₄H₉ | CH₃ | H |
| t-C₄H₉ | CH₃ | H |
| C₂H₅ | C₂H₅ | H |
| C₃H₇ | C₃H₇ | H |
| 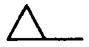 | CH₃ | H |
| 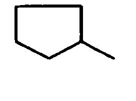 | CH₃ | H |
| 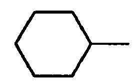 | CH₃ | H |
| -(CH2)2- | | H |
| -(CH₂)₄- | | H |
| -(CH₂)₅- | | H |
| -(CH₂)₆- | | H |
| -(CH₂)₇- | | H |
| -(CH₂)₂-O-(CH₂)₂- | | H |
| -CH₂-O-(CH₂)₃- | | H |
| -(CH₂)₂-S-(CH₂)₂- | | H |
| -CH₂-CHCH₃-(CH₂)₃- | | H |
| -(CH₂)₂-CHCH₃-(CH₂)₂- | | H |
| -(CH₂)₂-CHC₂H₅-(CH₂)₂- | | H |
| -(CH₂)₂-CHC₃H₇-(CH₂)₂- | | H |
| -(CH₂)₂-CHi-C₃H₇-(CH₂)₂- | | H |
| -(CH₂)₂-CHOCH₃-(CH₂)₂- | | H |
| -(CH₂)₂-CHOC₂H₅-(CH₂)₂- | | H |
| -(CH₂)₂-CHOC₃H_{?}-(CH₂)₂- | | H |
| -(CH₂)₂-CHOi-C₃H₇-(CH₂)₂- | | H |
| -(CH₂)₂-C(CH₃)₂-(CH2)₂- | | H |
| -CH₂-(CHCH₃)₂-(CH₂)₂- | | H |
| 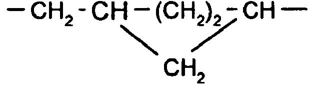 | | H |
| 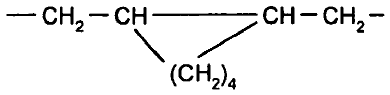 | | H |
| 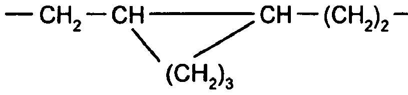 | | H |
| 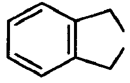 | | H |
| 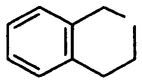 | | H |
| | | |

| A | D | B |
|---|---|---|
| -(CH₂)₃- | | H |
| -(CH₂)₄- | | H |
| -CH₂-CHCH₃-CH₂- | | H |
| -CH₂-CH₂-CHCH₃- | | H |
| -CH₂-CHCH₃-CHCH₃- | | H |
| -CH₂-S-CH₂- | | H |
| -CH₂-S-(CH₂)₂- | | H |
| -(CH₂)₂-S-CH₂- | | H |
| 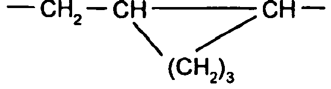 | | H |
| H | CH₃ | H |
| H | C₂H₅ | H |
| H | C₃H₇ | H |
| H | i-C₃H₇ | H |
| H | 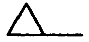 | H |
| H | 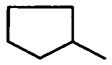 | H |
| H | 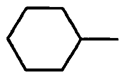 | H |
| CH₃ | CH₃ | H |
| CH₃ | C₂H₅ | H |
| CH₃ | C₃H₇ | H |
| CH₃ | i-C₃H₇ | H |
| CH₃ | 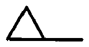 | H |
| CH₃ | 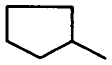 | H |
| CH₃ | 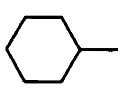 | H |
| C₂H₅ | CH₃ | H |
| C₂H₅ | C₂H₅ | H |

- **Tabelle 2:**: A, B und D wie in Tabelle 1 angegeben W = OCH₃; X = Br; Y = CH₃
- **Tabelle 3:**: A, B und D wie in Tabelle 1 angegeben W = OC₂H₅; X = Cl; Y = CH₃.
- **Tabelle 4:**: A, B und D wie in Tabelle 1 angegeben W = OC₂H₅; X = Br; Y = CH₃.

Im einzelnen seien außer den bei den Herstellungsbeispielen genannten Verbindungen die folgenden Verbindungen der Formel (I-2-a) genannt:

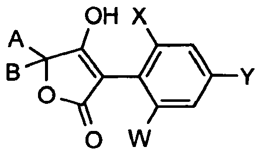

**Tabelle 5: W = OCH₃, X = Cl, Y = CH₃.**

| A | B |
|---|---|
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| C₂H₅ | C₂H₅ |
| C₃H₇ | C₃H₇ |
| 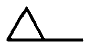 | CH₃ |
| 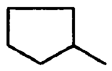 | CH₃ |
| 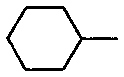 | CH₃ |
| -(CH2)2- | |
| -(CH₂)₄- | |
| -(CH₂)₅- | |
| -(CH₂)₆- | |
| -(CH₂)₇- | |
| -(CH₂)₂-O-(CH₂)₂- | |
| -CH₂-O-(CH₂)₃- | |
| -(CH₂)₂-S-(CH₂)₂- | |
| -CH₂-CHCH₃-(CH2)₃- | |
| -(CH₂)₂-CHCH₃-(CH₂)₂- | |
| (CH₂)₂-CHC₂H₅-(CH₂)₂- | |
| -(CH₂)₂-CHC₃H₇-(CH₂)₂- | |
| -(CH₂)₂-CHOi-C₃H₇-(CH₂)₂- | |
| -(CH₂)₂-CHOCH₃-(CH₂)₂- | |
| -(CH₂)₂-CHOC₂H₅-(CH₂)₂- | |
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| -(CH₂)₂-CHOC₃H₇-(CH₂)₂- | |
| -(CH₂)₂-CHi-C₃H₇-(CH₂)₂- | |
| -(CH₂)₂-C(CH₃)₂-(CH₂)₂- | |
| -CH₂-(CHCH₃)₂-(CH₂)₂- | |
| 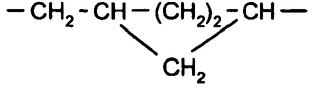 | |
| 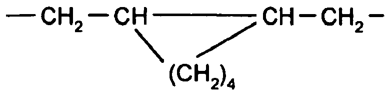 | |
| CH₃ | H |
| C₂H₅ | H |
| C₃H₇ | H |
| i-C₃H₇ | H |
| C₄H₉ | H |
| i-C₄H₉ | H |
| s-C₄H₉ | H |
| t-C₄H₉ | H |
| CH₃ | CH₃ |
| C₂H₅ | CH₃ |
| C₃H₇ | CH₃ |
| i-C₃H₇ | CH₃ |
| C₄H₉ | CH₃ |
| i-C₄H₉ | CH₃ |
| s-C₄H₉ | CH₃ |
| t-C₄H₉ | CH₃ |
| 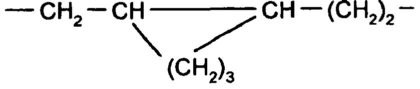 | |
| 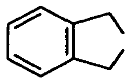 | |
| 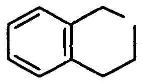 | |

| | |
|---|---|
| **Tabelle 6:** | A und B wie in Tabelle 5 angegeben |
| | W = OCH₃; X = Br; Y = CH₃. |

| | |
|---|---|
| **Tabelle 7:** | A und B wie in Tabelle 5 angegeben |
| | W = OC₂H₅; X = Cl; Y = CH₃. |

| | |
|---|---|
| **Tabelle 8:** | A und B wie in Tabelle 5 angegeben |
| | W = OC₂H₅; X = Br; Y = CH₃. |

Bevorzugte Bedeutungen der oben in Zusammenhang mit den die Kulturpflanzen-Verträglichkeit verbessernden Verbindungen ("Herbizid-Safenern") der Formeln (IIa), (IIb), (IIc), (IId) und (IIe) aufgeführten Gruppen werden im Folgenden definiert.
- m: steht bevorzugt für die Zahlen 0, 1, 2, 3 oder 4.
- A¹: steht bevorzugt für eine der nachstehend skizzierten divalenten heterocyclischen Gruppierungen

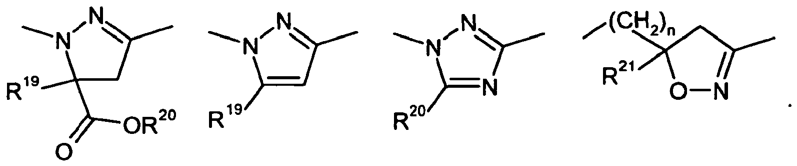

- n: steht bevorzugt für die Zahlen 0, 1, 2, 3 oder 4.
- A²: steht bevorzugt für jeweils gegebenenfalls durch Methyl, Ethyl, Methoxycarbonyl oder Ethoxycarbonyl substituiertes Methylen oder Ethylen.
- R¹⁴: steht bevorzugt für Hydroxy, Mercapto, Amino, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁵: steht bevorzugt für Hydroxy, Mercapto, Amino, Methoxy, Ethoxy, n- oder i-Propoxy, n-, i-, s- oder t-Butoxy, 1-Methylhexyloxy, Allyloxy, 1-Allyloxymethyl-ethoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino.
- R¹⁶: steht bevorzugt für jeweils gegebenenfalls durch Fluor, Chlor und/oder Brom substituiertes Methyl, Ethyl, n- oder i-Propyl.

- R¹⁷: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl.
- R¹⁸: steht bevorzugt für Wasserstoff, jeweils gegebenenfalls durch Fluor und/oder Chlor substituiertes Methyl, Ethyl, n- oder i-Propyl, n-, i-, s- oder t-Butyl, Propenyl, Butenyl, Propinyl oder Butinyl, Methoxymethyl, Ethoxymethyl, Methoxyethyl, Ethoxyethyl, Dioxolanylmethyl, Furyl, Furylmethyl, Thienyl, Thiazolyl, Piperidinyl, oder gegebenenfalls durch Fluor, Chlor, Methyl, Ethyl, n- oder i-Propyl, n-, i-, s- oder t-Butyl substituiertes Phenyl, oder R¹⁷ und R¹⁸ stehen auch gemeinsam für einen der Reste -CH₂-O-CH₂-CH₂- und -CH₂-CH₂-O-CH₂-CH₂-, die gegebenenfalls substituiert sind durch Methyl, Ethyl, Furyl, Phenyl, einen annellierten Benzolring oder durch zwei Substituenten, die gemeinsam mit dem C-Atom, an das sie gebunden sind, einen 5- oder 6-gliedrigen Carbocyclus bilden.
- R¹⁹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, Cyclopropyl, Cyclobutyl, Cyclopentyl, Cyclohexyl oder Phenyl.
- R²⁰: steht bevorzugt für Wasserstoff, gegebenenfalls durch Hydroxy, Cyano, Fluor, Chlor, Methoxy, Ethoxy, n- oder i-Propoxy substituiertes Methyl, Ethyl, n- oder i-Propyl, n-, i-, s-oder t-Butyl.
- R²¹: steht bevorzugt für Wasserstoff, Cyano, Fluor, Chlor, Brom, oder für jeweils gegebenenfalls durch Fluor, Chlor und/oder Brom substituiertes Methyl, Ethyl, n- oder i-Propyl, n-, i-, s-oder t-Butyl, Cyclopropyl, Cyclobutyl, Cyclopentyl, Cyclohexyl oder Phenyl.
- X¹: steht bevorzugt für Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- X²: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.

- X³: steht bevorzugt für Wasserstoff, Nitro, Cyano, Fluor, Chlor, Brom, Methyl, Ethyl, n- oder i-Propyl, n-, i-, s- oder t-Butyl, Difluormethyl, Dichlormethyl, Trifluormethyl, Trichlormethyl, Chlordifluormethyl, Fluordichlormethyl, Methoxy, Ethoxy, n- oder i-Propoxy, Difluormethoxy oder Trifluormethoxy.
- t: steht bevorzugt für die Zahlen 0, 1, 2, 3 oder 4.
- v: steht bevorzugt für die Zahlen 0, 1, 2, 3 oder 4.
- R²²: steht bevorzugt für Wasserstoff, Methyl, Ethyl, n- oder i-Propyl.
- R²³: steht bevorzugt für Wasserstoff, Methyl, Ethyl, n- oder i-Propyl.
- R²⁴: steht bevorzugt für Wasserstoff, jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, , Methylamino, Ethylamino, n- oder i-Propylamino, n-, i-, s- oder t-Butylamino, Dimethylamino oder Diethylamino, oder jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylthio, Cyclobutylthio, Cyclopentylthio, Cyclohexylthio, Cyclopropylamino, Cyclobutylamino, Cyclopentylamino oder Cyclohexylamino.
- R²⁵: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, oder jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl.
- R²⁶: steht bevorzugt für Wasserstoff, jeweils gegebenenfalls durch Cyano, Hydroxy, Fluor, Chlor, Methoxy, Ethoxy, n- oder i-Propoxy substituiertes Methyl, Ethyl, n- oder i-Propyl, n-, i- oder s-Butyl, jeweils gegebenenfalls durch Cyano, Fluor, Chlor oder Brom substituiertes Propenyl, Butenyl, Propinyl oder Butinyl, jeweils gegebenenfalls durch Cyano, Fluor, Chlor, Brom, Methyl, Ethyl, n- oder i-Propyl substituiertes Cyclopropyl, Cyclobutyl, Cyclopentyl oder Cyclohexyl, oder gegebenenfalls durch Nitro, Cyano, Fluor, Chlor, Brom, Methyl, Ethyl, n-oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, oder zusammen mit R²⁵ für jeweils gegebenenfalls durch Methyl oder Ethyl substituiertes Butan-1,4-diyl (Trimethylen), Pentan-1,5-diyl, 1-Oxa-butan-1,4-diyl oder 3-Oxa-pentan-1,5-diyl.
- X⁴: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.
- X⁵: steht bevorzugt für Nitro, Cyano, Carboxy, Carbamoyl, Formyl, Sulfamoyl, Hydroxy, Amino, Fluor, Chlor, Brom, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy.

Beispiele für die als erfindungsgemäße Herbizid-Safener ganz besonders bevorzugten Verbindungen der Formel (IIa) sind in der nachstehenden Tabelle aufgeführt.

**Tabelle : Beispiele für die Verbindungen der Formel (IIa)**

| | | | |
|---|---|---|---|
| 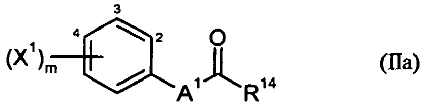 | | | |

| **Beispiel-Nr.** | **(Positionen) (X¹)ₘ** | **A¹** | **R¹⁴** |
|---|---|---|---|
| IIa-1 | (2) Cl, (4) Cl | 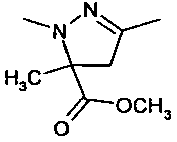 | OCH₃ |
| IIa-2 | (2) Cl, (4) Cl | 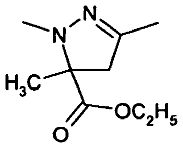 | OCH₃ |
| IIa-3 | (2) Cl, (4) Cl | 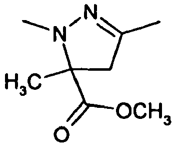 | OC₂H₅ |
| IIa-4 | (2) Cl, (4) Cl | 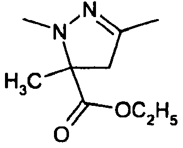 | OC₂H₃ |
| IIa-5 | (2) Cl | 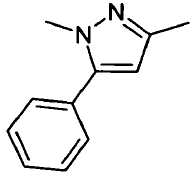 | OCH₃ |
| IIa-6 | (2) Cl, (4) Cl | 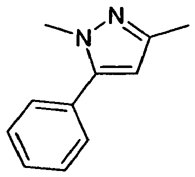 | OCH₃ |
| IIa-7 | (2) F | 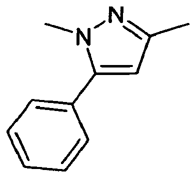 | OCH₃ |
| IIa-8 | (2) F | 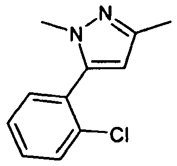 | OCH₃ |
| IIa-9 | (2) Cl, (4) Cl | 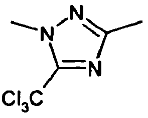 | OC₂H₅ |
| IIa- 10 | (2) Cl, (4) CF₃ | 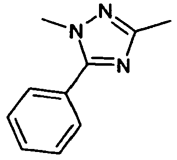 | OCH₃ |
| IIa-11 | (2) Cl | 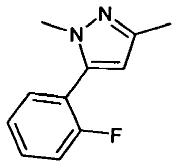 | OCH₃ |
| IIa-12 | - | 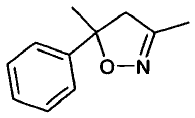 | OC₂H₅ |
| IIa-13 | (2) Cl, (4) Cl | 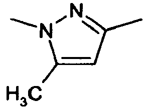 | OC₂H₅ |
| IIa-14 | (2) Cl, (4) Cl | 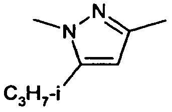 | OC₂H₅ |
| IIa-15 | (2) Cl, (4) Cl | 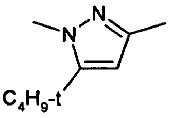 | OC₂H₅ |
| IIa-16 | (2) Cl, (4) Cl | 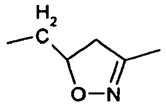 | OC₂H₅ |
| IIa-17 | (2) Cl, (4) Cl | 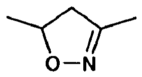 | OC₂H₅ |
| IIa-18 | - | 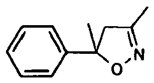 | OH |

Beispiele für die als erfindungsgemäße Herbizid-Safener ganz besonders bevorzugten Verbindungen der Formel (IIb) sind in der nachstehenden Tabelle aufgeführt.

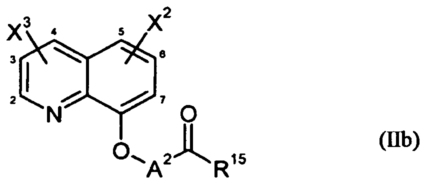

**Tabelle: Beispiele für die Verbindungen der Formel (IIb)**

| **Beispiel-Nr.** | **(Position) X²** | **(Position) X³** | **A²** | **R¹⁵** |
|---|---|---|---|---|
| IIb-1 | (5) Cl | - | CH₂ | OH |
| IIb-2 | (5) Cl | - | CH₂ | OCH₃ |
| IIb-3 | (5) Cl | - | CH₂ | OC₂H₅ |
| IIb-4 | (5) Cl | - | CH₂ | OC₃H₇-n |
| IIb-5 | (5) Cl | - | CH₂ | OC₃H₇-i |
| IIb-6 | (5) Cl | - | CH₂ | OC₄H₉-n |
| IIb-7 | (5) Cl | - | CH₂ | OCH(CH₃)C₅H₁₁-n |
| IIb-8 | (5) Cl | (2) F | CH₂ | OH |
| IIb-9 | (5) Cl | (2) Cl | CH₂ | OH |
| IIb-10 | (5) Cl | - | CH₂ | OCH₂CH=CH₂ |
| IIb-11 | (5) Cl | - | CH₂ | OC₄H₉-i |
| IIb-12 | (5) Cl | - | CH₂ | 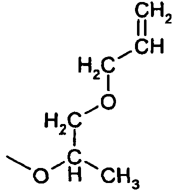 |
| IIb-13 | (5) Cl | - | 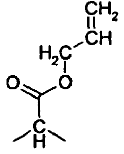 | OCH₂CH=CH₂ |
| IIb-14 | (5) Cl | - | 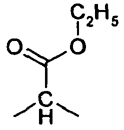 | OC₂H₅ |
| IIb-15 | (5) Cl | - | 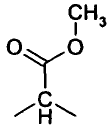 | OCH3 |

Beispiele für die als erfindungsgemäße Herbizid-Safener ganz besonders bevorzugten Verbindungen der Formel (IIc) sind in der nachstehenden Tabelle aufgeführt.

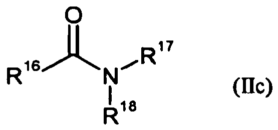

**Tabelle: Beispiele für die Verbindungen der Formel (IIc)**

| **Beispiel-Nr.** | **R¹⁶** | **N(R¹⁷,R¹⁸)** |
|---|---|---|
| IIc-1 | CHCl₂ | N(CH₂CH=CH₂)₂ |
| IIc-2 | CHCl₂ | 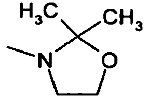 |
| IIc-3 | CHCl₂ | 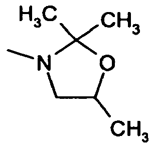 |
| IIc-4 | CHCl₂ | 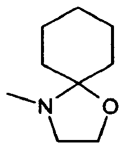 |
| IIc-5 | CHCl₂ | 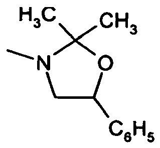 |
| IIc-6 | CHCl₂ | 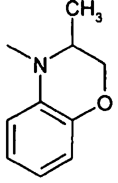 |
| IIc-7 | CHCl₂ | 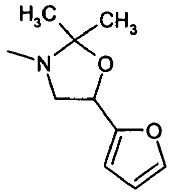 |

Beispiele für die als erfindungsgemäße Herbizid-Safener ganz besonders bevorzugten Verbindungen der Formel (IId) sind in der nachstehenden Tabelle aufgeführt.

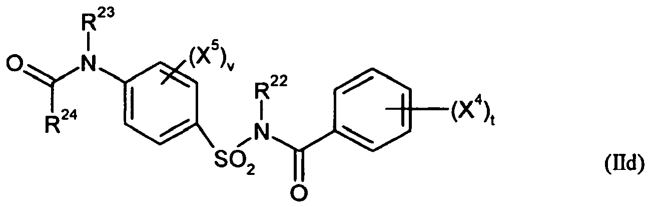

**Tabelle : Beispiele für die Verbindungen der Formel (IId)**

| **Beispiel-Nr.** | **R²²** | **R²³** | **R²⁴** | **(Positionen) (X⁴)ₜ** | **(Positionen) (X⁵)ᵥ** |
|---|---|---|---|---|---|
| IId-1 | H | H | CH₃ | (2) OCH₃ | - |
| IId-2 | H | H | C₂H₅ | (2) OCH₃ | - |
| IId-3 | H | H | C₃H₇-n | (2) OCH3 | - |
| IId-4 | H | H | C₃H₇-i | (2) OCH₃ | - |
| IId-5 | H | H | 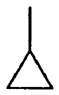 | (2) OCH₃ | - |
| IId-6 | H | H | CH₃ | (2) OCH₃ | - |
| | | | | (5) CH₃ | |
| IId-7 | H | H | C₂H₅ | (2) OCH₃ | - |
| | | | | (5) CH₃ | |
| IId-8 | H | H | C₃H₇-n | (2) OCH₃ | - |
| | | | | (5) CH₃ | |
| IId-9 | H | H | C₃H₇-i | (2) OCH₃ | - |
| | | | | (5) CH₃ | |
| IId-10 | H | H | 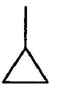 | (2) OCH₃ | - |
| | | | | (5) CH₃ | |
| IId-11 | H | H | OCH₃ | (2) OCH₃ | - |
| | | | | (5) CH₃ | |
| IId-12 | H | H | OC₂H₅ | (2) OCH₃ | - |
| | | | | (5) CH₃ | |
| IId-13 | H | H | OC₃H₇-i | (2) OCH₃ | - |
| | | | | (5) CH₃ | |
| IId-14 | H | H | SCH₃ | (2) OCH₃ | - |
| | | | | (5) CH₃ | |
| IId-15 | H | H | SC₂H₅ | (2) OCH₃ | - |
| | | | | (5) CH₃ | |
| IId-16 | H | H | SC₃H₇-i | (2) OCH₃ | - |
| | | | | (5) CH₃ | |
| IId-17 | H | H | NHCH₃ | (2) OCH₃ | - |
| | | | | (5) CH₃ | |
| IId-18 | H | H | NHC₂H₅ | (2) OCH₃ | - |
| | | | | (5) CH₃ | |
| IId-19 | H | H | NHC₃H₇-i | (2) OCH₃ | - |
| | | | | (5) CH₃ | |
| IId-20 | H | H | 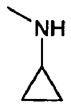 | (2) OCH₃ | - |
| | | | | (5) CH₃ | |
| IId-21 | H | H | NHCH₃ | (2) OCH₃ | - |
| IId-22 | H | H | NHC₃H₇-i | (2) OCH₃ | - |
| IId-23 | H | H | N(CH₃)₂ | (2) OCH₃ | - |
| IId-24 | H | H | N(CH₃)₂ | (3) CH₃ | - |
| | | | | (4) CH₃ | |
| IId-25 | H | H | CH₂-O-CH₃ | (2) OCH₃ | - |

Beispiele für die als erfindungsgemäße Herbizid-Safener ganz besonders bevorzugten Verbindungen der Formel (IIe) sind in der nachstehenden Tabelle aufgeführt.

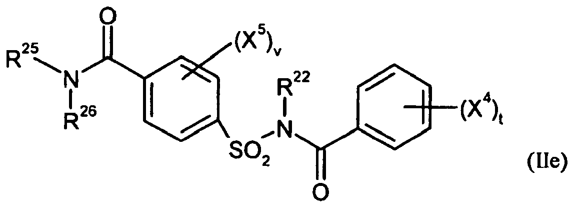

**Tabelle: Beispiele für die Verbindungen der Formel (IIe)**

| **Beispiel-Nr.** | **R²²** | **R²⁵** | **R²⁶** | **(Positionen) (X⁴)ₜ** | **(Positionen) (X⁵)ᵥ** |
|---|---|---|---|---|---|
| IIe-1 | H | H | CH₃ | (2) OCH₃ | - |
| IIe-2 | H | H | C₂H₅ | (2) OCH₃ | - |
| IIe-3 | H | H | C₃H₇-n | (2) OCH₃ | - |
| IIe-4 | H | H | C₃H₇-i | (2) OCH₃ | - |
| IIe-5 | H | H | 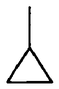 | (2) OCH₃ | - |
| IIe-6 | H | CH₃ | CH₃ | (2) OCH₃ | - |
| IIe-7 | H | H | CH₃ | (2) OCH₃ | - |
| | | | | (5) CH₃ | |
| IIe-8 | H | H | C₂H₅ | (2) OCH₃ | - |
| | | | | (5) CH₃ | |
| IIe-9 | H | H | C₃H₇-n | (2) OCH₃ | - |
| | | | | (5) CH₃ | |
| IIe-10 | H | H | C₃H₇-i | (2) OCH₃ | - |
| | | | | (5) CH₃ | |
| IIe-11 | H | H | 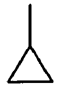 | (2) OCH₃ | - |
| | | | | (5) CH₃ | |
| IIe-12 | H | CH₃ | CH₃ | (2) OCH₃ | - |
| | | | | (5) CH₃ | |

Als die die Kulturpflanzen-Verträglichkeit verbessernde Verbindung [Komponente (b')] sind Cloquintocet-mexyl, Fenchlorazol-ethyl, Isoxadifen-ethyl, Mefenpyr-diethyl, Furilazole, Fenclorim, Cumyluron, Dymron, Dimepiperate und die Verbindungen IIe-5 und IIe-11 am meisten bevorzugt, wobei Cloquintocet-mexyl und Mefenpyr-diethyl besonders hervorgehoben seien.

Beispiele für die erfindungsgemäßen selektiv herbiziden Kombinationen aus jeweils einem Wirkstoff der Formel (I) und jeweils einem der oben definierten Safener sind in der nachstehenden Tabelle aufgeführt.

**Tabelle: Beispiele für die erfindungsgemäßen Kombinationen**

| **Wirkstoffe der Formel (I)** | **Safener** |
|---|---|
| I-1 | Cloquintocet-mexyl |
| I-1 | Fenchlorazole-ethyl |
| I-1 | Isoxadifen-ethyl |
| I-1 | Mefenpyr-diethyl |
| I-1 | Furilazole |
| I-1 | Fenclorim |
| I-1 | Cumyluron |
| I-1 | Daimuron /Dymron |
| I-1 | Dimepiperate |
| I-1 | IIe-11 |
| I-1 | IIe-5 |
| I-2 | Cloquintocet-mexyl |
| I-2 | Fenchlorazole-ethyl |
| I-2 | Isoxadifen-ethyl |
| I-2 | Mefenpyr-diethyl |
| I-2 | Furilazole |
| I-2 | Fenclorim |
| I-2 | Cumyluron |
| I-2 | Daimuron /Dymron |
| I-2 | Dimepiperate |
| I-2 | IIe-11 |
| I-2 | IIe-5 |

Die als Safener erfindungsgemäß zu verwendenden Verbindungen der allgemeinen Formel (IIa) sind bekannt und/oder können nach an sich bekannten Verfahren hergestellt werden (vgl. WO-A-91/07874, WO-A-95/07897).

Die als Safener erfindungsgemäß zu verwendenden Verbindungen der allgemeinen Formel (IIb) sind bekannt und/oder können nach an sich bekannten Verfahren hergestellt werden (vgl. EP-A-191736).

Die als Safener erfindungsgemäß zu verwendenden Verbindungen der allgemeinen Formel (IIc) sind bekannt und/oder können nach an sich bekannten Verfahren hergestellt werden (vgl. DE-A-2218097, DE-A-2350547).

Die als Safener erfindungsgemäß zu verwendenden Verbindungen der allgemeinen Formel (IId) sind bekannt und/oder können nach an sich bekannten Verfahren hergestellt werden (vgl. DE-A-19621522 / US-A-6235680).

Die als Safener erfindungsgemäß zu verwendenden Verbindungen der allgemeinen Formel (IIe) sind bekannt und/oder können nach an sich bekannten Verfahren hergestellt werden (vgl. WO-A-99/66795 / US-A-6251827).

Es wurde nun überraschend gefunden, dass die oben definierten Wirkstoffkombinationen aus substituierten Ketoenolen der allgemeinen Formel (I) und Safenern (Antidots) aus der oben aufgeführten Gruppe (b') bei sehr guter Nutzpflanzen-Verträglichkeit eine besonders hohe herbizide Wirksamkeit aufweisen und in verschiedenen Kulturen, insbesondere in Getreide (vor allem Weizen), aber auch in Soja, Kartoffeln, Mais und Reis zur selektiven Unkrautbekämpfung verwendet werden können.

Dabei ist es als überraschend anzusehen, dass aus einer Vielzahl von bekannten Safenern oder Antidots, die befähigt sind, die schädigende Wirkung eines Herbizids auf die Kulturpflanzen zu antagonisieren, gerade die oben aufgeführten Verbindungen der Gruppe (b') geeignet sind, die schädigende Wirkung von substituierten cyclischen Ketoenolen der Formel (I) auf die Kulturpflanzen annähernd vollständig aufzuheben, ohne dabei die herbizide Wirksamkeit gegenüber den Unkräutern maßgeblich zu beeinträchtigen.

Hervorgehoben sei hierbei die besonders vorteilhafte Wirkung der besonders und am meisten bevorzugten Kombinationspartner aus der Gruppe (b'), insbesondere hinsichtlich der Schonung von Getreidepflanzen, wie z.B. Weizen, Gerste und Roggen, aber auch Mais und Reis, als Kulturpflanzen.

Verwendet man beispielsweise gemäß Verfahren (A) N-(2-Chlor-4-methyl-6-methoxy-phenylacetyl)-1-amino-cyclohexan-carbonsäureethylester als Ausgangsstoff, so kann der Verlauf des erfindungsgemäßen Verfahrens durch folgendes Reaktionsschema wiedergegeben werden:

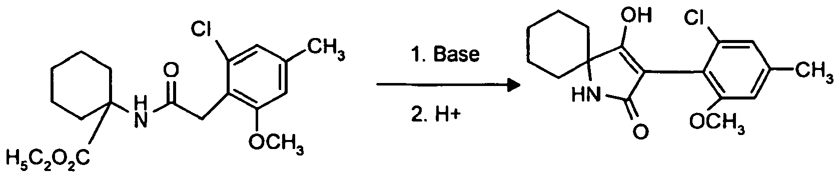

Verwendet man beispielsweise gemäß Verfahren (B) O-(2-Chlor-4-methyl-6-methoxy-phenylacetyl)-2-hydroxyisobuttersäureethylester, so kann der Verlauf des erfindungsgemäßen Verfahrens durch folgendes Reaktionsschema wiedergegeben werden:

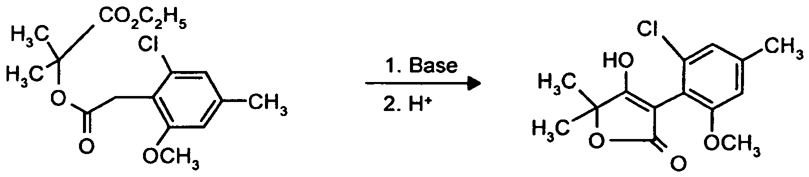

Verwendet man beispielsweise gemäß Verfahren (Iα) 3-(2-Chlor-4-methyl-6-methoxy-phenyl)-5,5-dimethylpyrrolidin-2,4-dion und Pivaloylchlorid als Ausgangsstoffe, so kann der Verlauf des erfindungsgemäßen Verfahrens durch folgendes Reaktionsschema wiedergegeben werden:

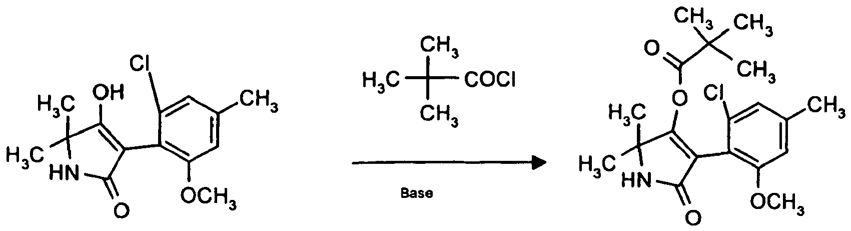

Verwendet man beispielsweise gemäß Verfahren (Iβ) 3-(2-Chlor-4-methyl-6-methoxy-phenyl)-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden:

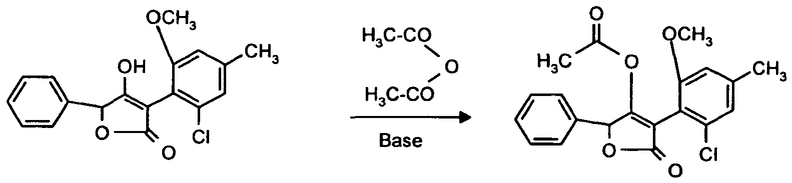

Verwendet man beispielsweise gemäß Verfahren (J) 8-[(2-Chlor-4-methyl-6-methoxy)-phenyl]-1-aza-bicyclo-(4,3,0^{1,6})-nonan-7,9-dion und Chlorameisensäureethoxyethylester als Ausgangsverbindungen, so kann der Verlauf des erfindungsgemäßen Verfahrens durch folgendes Reaktionsschema wiedergegeben werden:

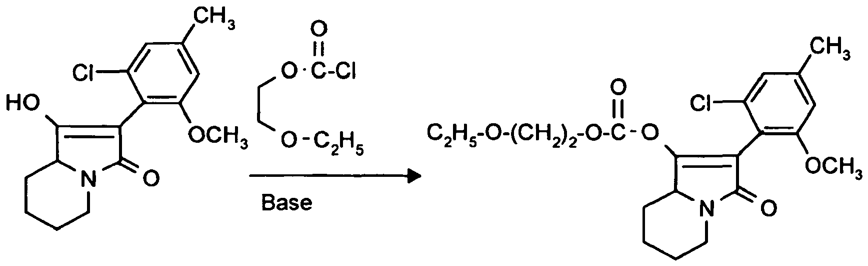

Verwendet man beispielsweise gemäß Verfahren (L) 3-(2-Chlor-4-methyl-6-methoxy-phenyl)-5,5-pentamethylen-pyrrolidin-2,4-dion und Methansulfonsäurechlorid als Ausgangsprodukt, so kann der Reaktionsverlauf durch folgendes Reaktionsschema wiedergegeben werden:

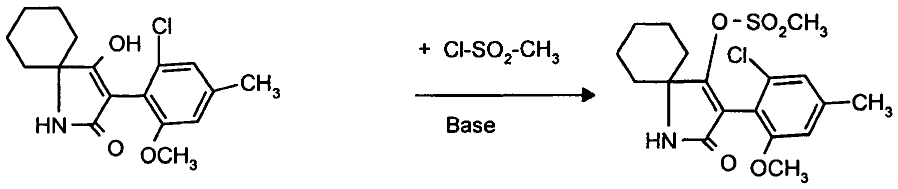

Verwendet man beispielsweise gemäß Verfahren (N) 3-(2-Chlor-4-methyl-6-methoxy-phenyl]-5-cyclopropyl-5-methyl-pyrrolidin-2,4-dion und NaOH als Komponenten, so kann der Verlauf des erfindungsgemäßen Verfahrens durch folgendes Reaktionsschema wiedergegeben werden:

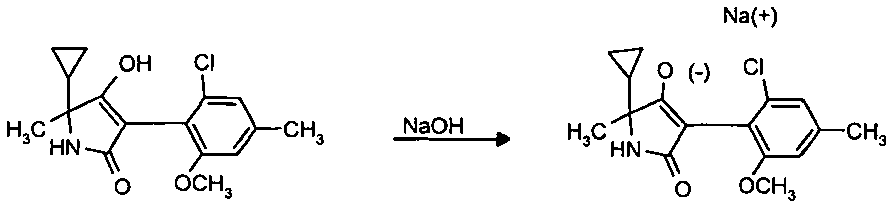

Die beim erfindungsgemäßen Verfahren (a) als Ausgangsstoffe benötigten Verbindungen der Formel (II) 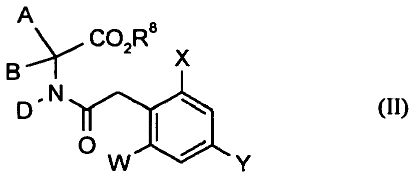 in welcher
A, B, D, W, X, Y und R⁸ die oben angegebenen Bedeutungen haben,
sind neu.

Man erhält die Acylaminosäureester der Formel (II) beispielsweise, wenn man Aminosäurederivate der Formel (XXIII) 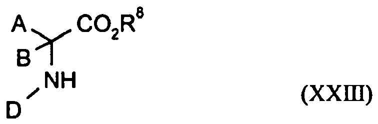 in welcher
A, B, R⁸ und D die oben angegebenen Bedeutungen haben,
mit substituierten Phenylessigsäurederivaten Formel (XXIV) 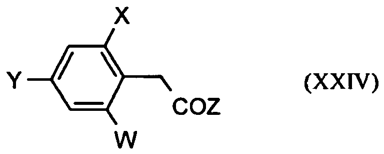 in welcher
- W, X und Y: die oben angegebenen Bedeutungen haben und
- Z: für eine durch Carbonsäureaktivierungsreagenzien wie Carbonyldiimidazol, Carbonyldiimide (wie z.B. Dicyclohexylcarbonyldiimid), Phosphorylierungsreagenzen (wie z.B. POCl₃, BOP-Cl), Halogenierungsmittel z.B. Thionylchlorid, Oxalylchlorid, Phosgen oder Chlorameisensäsureester eingeführte Abgangsgruppe steht,
acyliert (Chem. Reviews 52, 237-416 (1953); Bhattacharya, Indian J. Chem. 6, 341-5, 1968) oder wenn man Acylaminosäuren der Formel (XXV) 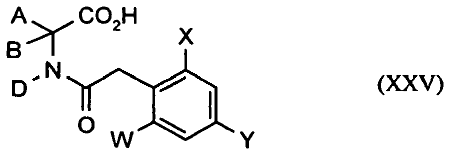 in welcher
A, B, D, W, X und Y die oben angegebenen Bedeutungen haben,
verestert (Chem. Ind. (London) 1568 (1968)).

Die Verbindungen der Formel (XXV) 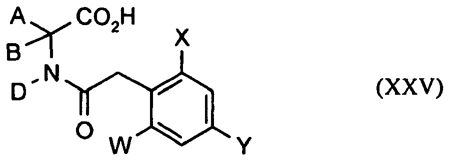 in welcher
A, B, D, W, X und Y die oben angegebenen Bedeutungen haben,
sind neu.

Man erhält die Verbindungen der Formel (XXV), wenn man Aminosäuren der Formel (XXVI) 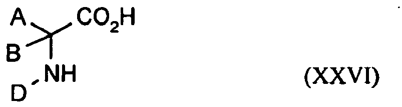 in welcher
A, B und D die oben angegebenen Bedeutungen haben,
mit substituierten Phenylessigsäurederivaten der Formel (XXIV) 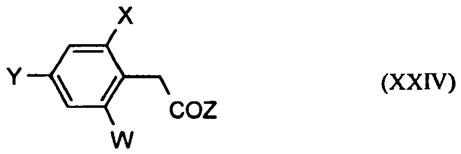 in welcher
- W, X und Y: die oben angegebenen Bedeutungen haben und
- Z: die oben angegebene Bedeutung hat,
beispielsweise nach Schotten-Baumann acyliert (Organikum, VEB Deutscher Verlag der Wissenschaften, Berlin 1977, S. 505).

Die Verbindungen der Formel (XXIV) sind neu. Sie lassen sich nach im Prinzip bekannten Verfahren und wie aus den Beispielen ersichtlich darstellen (s. z.B. H. Henecka, Houben-Weyl, Methoden der Organischen Chemie, Bd. 8, S. 467-469 (1952)).

Man erhält die Verbindungen der Formel (XXIV) beispielsweise, indem man substituierte Phenylessigsäuren der Formel (XXVII) 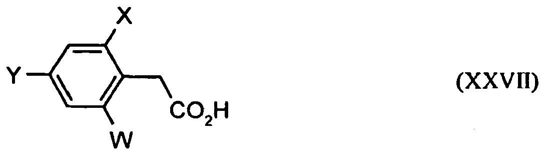 in welcher
W, X und Y die oben angegebene Bedeutung haben,
mit Halogenierungsmitteln (z.B. Thionylchlorid, Thionylbromid, Oxalylchlorid, Phosgen, Phosphortrichlorid, Phosphortribromid oder Phosphorpentachlorid), Phosphonylierungsreagenzien wie (z.B. POCl₃, BOP-Cl), Carbonyldiimidazol, Carbonyldiimide (z.B. Dicyclohexylcarbonyldiimid) gegebenenfalls in Gegenwart eines Verdünnungsmittels (z.B. gegebenenfalls chlorierten aliphatischen oder aromatischen Kohlenwasserstoffen wie Toluol oder Methylenchlorid oder Ethern, z.B. Tetrahydrofuran, Dioxan, Methyl-tert.-butylether) bei Temperaturen von -20°C bis 150°C, bevorzugt von 10°C bis 100°C, umsetzt.

Die Verbindungen der Formel (XXIII) und (XXVI) sind teilweise bekannt und/oder lassen sich nach bekannten Verfahren darstellen (siehe z.B. Compagnon, Miocque Ann. Chim. (Paris) [14] 5, S. 11-22, 23-27 (1970)).

Die substituierten cyclischen Aminocarbonsäuren der Formel (XXVI), in der A und B einen Ring bilden, sind im Allgemeinen nach der Bucherer-Bergs-Synthese oder nach der Strecker-Synthese erhältlich und fallen dabei jeweils in unterschiedlichen Isomerenformen an. So erhält man unter den Bedingungen der Bucherer-Bergs-Synthese vorwiegend die Isomeren (im Folgenden der Einfachheit halber als β bezeichnet), in welchen die Reste R und die Carboxylgruppe äquatorial stehen, während nach den Bedingungen der Strecker-Synthese vorwiegend die Isomeren (im folgenden der Einfachheit halber als α bezeichnet) anfallen, bei denen die Aminogruppe und die Reste R äquatorial stehen.

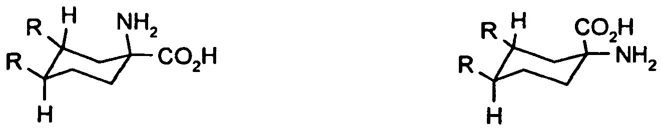

| | |
|---|---|
| Bucherer-Bergs-Synthese | Strecker-Synthese |
| (β-Isomeres) | (α-Isomeres) |

(L. Munday, J. Chem. Soc. 4372 (1961); J.T. Eward, C. Jitrangeri, Can. J. Chem. 53, 3339 (1975).

Weiterhin lassen sich die bei dem obigen Verfahren (A) verwendeten Ausgangsstoffe der Formel (II) 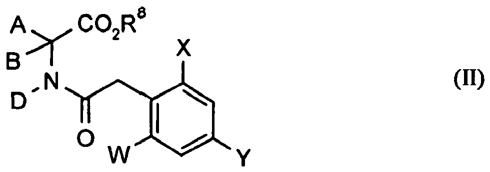 in welcher
A, B, D, W, X, Y und R⁸ die oben angegebenen Bedeutungen haben,
herstellen, wenn man Aminonitrile der Formel (XXVIII) 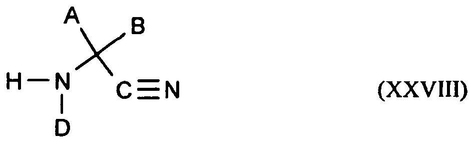 in welcher
A, B und D die oben angegebenen Bedeutungen haben,
mit substituierten Phenylessigsäurederivaten der Formel (XXIV) 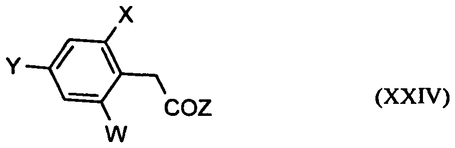 in welcher
W, X, Y und Z die oben angegebenen Bedeutungen haben,
zu Verbindungen der Formel (XXIX) 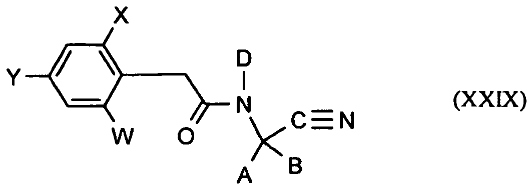 in welcher
A, B, D, W, X und Y die oben angegebenen Bedeutungen haben,
umsetzt,
und diese anschließend einer sauren Alkoholyse unterwirft.

Die Verbindungen der Formel (XXIX) sind ebenfalls neu.

Die bei dem erfindungsgemäßen Verfahren (B) als Ausgangstoffe benötigten Verbindungen der Formel (III) 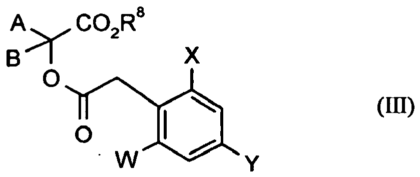 in welcher
A, B, W, X, Y und R⁸ die oben angegebenen Bedeutungen haben,
sind neu.

Sie lassen sich nach im Prinzip bekannten Methoden herstellen.

So erhält man die Verbindungen der Formel (III) beispielsweise, wenn man 2-Hydroxycarbonsäureester der Formel (XXX-A) 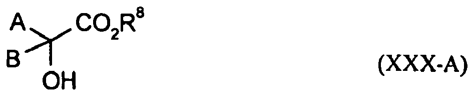 in welcher
- A, B und R⁸: die oben angegebenen Bedeutungen haben,
mit substituierten Phenylessigsäurederivaten der Formel (XXIV) 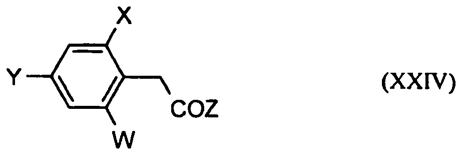 in welcher
- W, X und Y: die oben angegebenen Bedeutungen haben,
acyliert (Chem. Reviews 52, 237-416 (1953)).
Weiterhin erhält man Verbindungen der Formel (III), wenn man substituierte Phenylessigsäuren der Formel (XXVII) 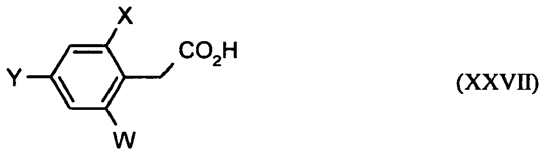 in welcher
- W, X und Y: die oben angegebenen Bedeutungen haben,
mit α-Halogencarbonsäureestern der Formel (XXX-B) 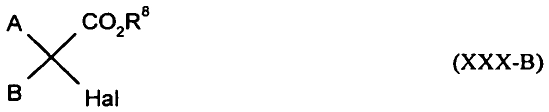 in welcher
- A, B und R⁸: die oben angegebenen Bedeutungen haben und
- Hal: für Chlor oder Brom steht,
alkyliert.

Die Verbindungen der Formel (XXVII) sind neu.

Die Verbindungen der Formel (XXX-B) sind käuflich.

Beispielsweise erhält man die Verbindungen der Formel (XXVII), 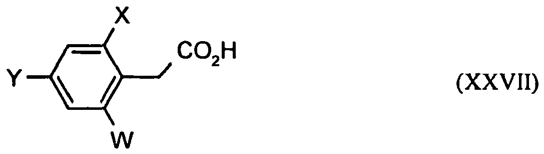 in welcher
- W, X und Y: die oben angegebenen Bedeutungen haben,
wenn man Phenylessigsäureester der Formel (XXXI) 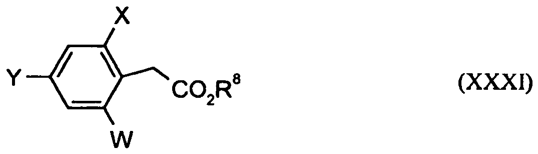 in welcher
- W, X, Y und R⁸: die oben angegebene Bedeutung haben,
in Gegenwart von Säuren oder Basen, in Gegenwart eines Lösungsmittels unter allgemein bekannten Standardbedingungen verseift. Weiterhin erhält man Phenylessigsäuren der Formel (XXVII) nach Verfahren (Q).

Die Verbindungen der Formel (XXXI) sind neu.

Die Verbindungen der Formel (XXXI) 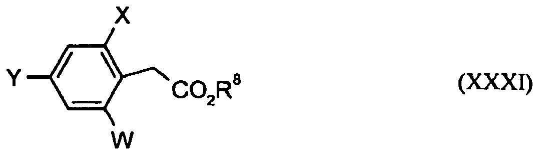 in welcher
- W, X, Y und R⁸: die oben angegebene Bedeutung haben,
erhält man beispielsweise nach dem in den Beispielen beschriebenen Verfahren (R),
wenn man Phenylessigsäureester der Formel (XXXI-a) 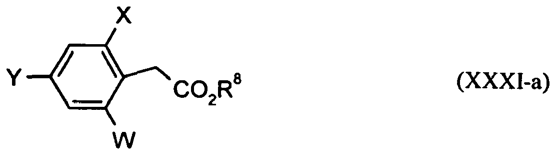 in welcher
- R⁸, X und Y: die oben angegebene Bedeutung haben, und
- W: für Halogen (insbesondere für Brom) steht,
in Gegenwart eines Alkohols, in Gegenwart einer Base und gegebenenfalls in Gegenwart eines Katalysators (bevorzugt Kupfersalze wie z.B. Kupfer(I)bromid) umsetzt.

Die Phenylessigsäureester der Formel (XXXI-a) sind aus der Anmeldung WO 96/35 664 bekannt oder lassen sich nach den dort beschriebenen Verfahren herstellen.

Weiterhin erhält man Phenylessigsäureester der Formel (XXXI) nach dem weiter hinten beschriebenen Verfahren (Q), in dem man die dort erhaltenen Phenylessigsäuren der Formel (XXVII) nach Standardmethoden verestert.

Die Verbindungen der Formel (XXVII) wurden bereits bei den Vorstufen für das Verfahren (A) beschrieben oder sind als Beispiele in den nachfolgenden Verfahren (Q) explizit beschrieben.
(Q) So erhält man weiterhin Phenylessigsäuren der Formel (XXVII), 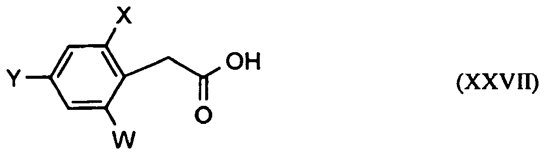 in welcher
   - W, X und Y: die oben angegebene Bedeutung haben,
   wenn man Phenylacetaldehyde der Formel (XLII) 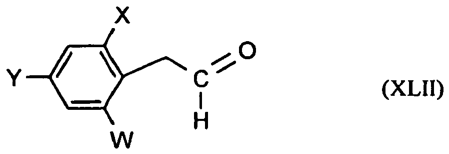 in welcher
   - W, X und Y: die oben angegebene Bedeutung haben,
   gegebenenfalls in Gegenwart eines Lösungsmittels mit geeigneten Oxidationsmitteln (wie z.B. NaOCl) oxidiert.
   Die Verbindungen der Formel (XLII) sind neu.
   Man erhält Verbindungen der Formel (XLII) 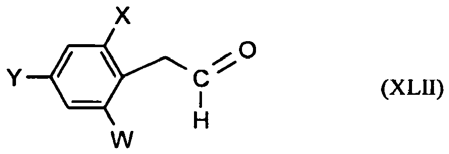 in welcher
   - W, X und Y: die oben angegebene Bedeutung haben,
   wenn man 3-Phenylpropene der Formel (XLIII) 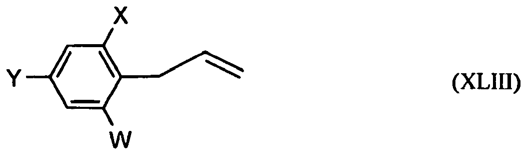 in welcher
   - W, X und Y: die oben angegebene Bedeutung haben
   in Gegenwart eines Lösungsmittels ozonolysiert und das erhaltene Ozonid beispielsweise mit Dimethylsulfid reduktiv aufarbeitet.

Die zur Herstellung der Verbindungen der Formel (XLII) benötigten 2-Alkoxy-substituierte 3-Phenyl-propene sind im Prinzip bekannte Verbindungen in der organischen Chemie und lassen sich nach Standardverfahren durch Alkylierung von Phenolen mit Allylhalogeniden, gefolgt von einer Claisen-Umlagerung und anschließender Alkylierung herstellen (WO 96/25 395).

Die zur Durchführung der erfindungsgemäßen Verfahren (I), (J), (L), (N) außerdem als Ausgangsstoffe benötigten Säurehalogenide der Formel (XIII), Carbonsäureanhydride der Formel (XIV), Chlorameisensäureester der Formel (XV), Sulfonsäurechloride der Formel (XVII), und Metallhydroxide, Metallalkoxide oder Amine der Formel (XIX) und (XX) sind allgemein bekannte Verbindungen der Organischen bzw. Anorganischen Chemie.

Die Verbindungen der Formeln (XIII) bis (XXII), (XXIII), (XXVI), (XXVIII), und sind darüber hinaus aus den eingangs zitierten Patentanmeldungen bekannt und/oder lassen sich nach den dort angegebenen Methoden herstellen.

Das Verfahren (A) ist dadurch gekennzeichnet, dass man Verbindungen der Formel (II), in welcher A, B, D, W, X, Y und R⁸ die oben angegebenen Bedeutungen haben, in Gegenwart einer Base einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon, sowie Alkohole wie Methanol, Ethanol, Propanol, Iso-Propanol, Butanol, Iso-Butanol und tert.-Butanol.

Als Base (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (A) wird im Allgemeinen unter Normaldruck durchgeführt.

Bei der Durchführung des erfindungsgemäßen Verfahrens (A) setzt man die Reaktionskomponenten der Formel (II) und die deprotonierenden Basen im Allgemeinen in etwa doppeläquimolaren Mengen ein. Es ist jedoch auch möglich, die eine oder andere Komponente in einem größeren Überschuss (bis zu 3 Mol) zu verwenden.

Das Verfahren (B) ist dadurch gekennzeichnet, dass Verbindungen der Formel (III), in welcher A, B, W, X, Y und R⁸ die oben angegebenen Bedeutungen haben, in Gegenwart eines Verdünnungsmittels und in Gegenwart einer Base einer intramolekularen Kondensation unterwirft.

Als Verdünnungsmittel können bei dem erfindungsgemäßen Verfahren (B) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, Iso-Propanol, Butanol, Iso-Butanol und tert.-Butanol eingesetzt werden.

Als Base (Deprotonierungsmittel) können bei der Durchführung des erfindungsgemäßen Verfahrens (B) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (= Methyltrialkyl(C₈-C₁₀)ammoniumchlorid) oder TDA 1 (= Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetallalkoholate, wie Natrium-methylat, Natrium-ethylat und Kalium-tert.-butylat einsetzbar.

Die Reaktionstemperaturen können bei der Durchführung des erfindungsgemäßen Verfahrens (B) innerhalb eines größeren Bereiches variiert werden. Im Allgemeinen arbeitet man bei Temperaturen zwischen 0°C und 250°C, vorzugsweise zwischen 50°C und 150°C.

Das erfindungsgemäße Verfahren (B) wird im Allgemeinen unter Normaldruck durchgeführt.

Bei der Durchführung des erfindungsgemäßen Verfahrens (B) setzt man die Reaktionskomponenten der Formel (III) und die deprotonierenden Basen im Allgemeinen in etwa äquimolaren Mengen ein. Es ist jedoch auch möglich, die eine oder andere Komponente in einem größeren Überschuss (bis zu 3 Mol) zu verwenden.

Das Verfahren (I-α) ist dadurch gekennzeichnet, dass man Verbindungen der Formeln (I-1-a) bis (I-2-a) jeweils mit Carbonsäurehalogeniden der Formel (XIII) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-α) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zulässt, kann die Umsetzung auch in Gegenwart von Wasser durchgeführt werden.

Als Säurebindemittel kommen bei der Umsetzung nach dem erfindungsgemäßen Verfahren (I-α) alle üblichen Säureakzeptoren in Betracht. Vorzugsweise verwendbar sind tertiäre Amine, wie Triethylamin, Pyridin, Diazabicyclooctan (DABCO), Diazabicycloundecen (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide wie Natriumhydroxid und Kaliumhydroxid.

Die Reaktionstemperaturen können bei dem erfindungsgemäßen Verfahren (I-α) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (I-α) werden die Ausgangsstoffe der Formeln (I-1-a) bis (I-2-a) und das Carbonsäurehalogenid der Formel (XIII) im Allgemeinen jeweils in angenähert äquivalenten Mengen verwendet. Es ist jedoch auch möglich, das Carbonsäurehalogenid in einem größeren Überschuss (bis zu 5 Mol) einzusetzen. Die Aufarbeitung erfolgt nach üblichen Methoden.

Das Verfahren (I-β) ist dadurch gekennzeichnet, dass man Verbindungen der Formeln (I-1-a) bis (I-2-a) mit Carbonsäureanhydriden der Formel (XIV) gegebenenfalls in Gegenwart eines Verdünnungsmittels und gegebenenfalls in Gegenwart eines Säurebindemittels umsetzt.

Als Verdünnungsmittel können bei dem erfindungsgemäßen Verfahren (I-β) vorzugsweise diejenigen Verdünnungsmittel verwendet werden, die auch bei der Verwendung von Säurehalogeniden vorzugsweise in Betracht kommen. Im übrigen kann auch ein im Überschuss eingesetztes Carbonsäureanhydrid gleichzeitig als Verdünnungsmittel fungieren.

Als gegebenenfalls zugesetzte Säurebindemittel kommen beim Verfahren (I-β) vorzugsweise diejenigen Säurebindemittel in Frage, die auch bei der Verwendung von Säurehalogeniden vorzugsweise in Betracht kommen.

Die Reaktionstemperaturen können bei dem erfindungsgemäßen Verfahren (I-β) innerhalb eines größeren Bereiches variiert werden. Im Allgemeinen arbeitet man bei Temperaturen zwischen -20°C und +150°C, vorzugsweise zwischen 0°C und 100°C.

Bei der Durchführung des erfindungsgemäßen Verfahrens (I-β) werden die Ausgangsstoffe der Formeln (I-1-a) bis (I-2-a) und das Carbonsäureanhydrid der Formel (XIV) im Allgemeinen in jeweils angenähert äquivalenten Mengen verwendet. Es ist jedoch auch möglich, das Carbonsäureanhydrid in einem größeren Überschuss (bis zu 5 Mol) einzusetzen. Die Aufarbeitung erfolgt nach üblichen Methoden.

Im Allgemeinen geht man so vor, dass man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (J) ist dadurch gekennzeichnet, dass man Verbindungen der Formeln (I-1-a) bis (I-2-a) jeweils mit Chlorameisensäureestern der Formel (XV) gegebenenfalls in Gegenwart eines Verdünnungsmittels und gegebenenfalls in Gegenwart eines Säurebindemittels umsetzt.

Als Säurebindemittel kommen bei der Umsetzung nach dem erfindungsgemäßen Verfahren (J) alle üblichen Säureakzeptoren in Betracht. Vorzugsweise verwendbar sind tertiäre Amine, wie Triethylamin, Pyridin, DABCO, DBU, DBA, Hünig-Base und N,N-Dimethyl-anilin, ferner Erdalkalimetalloxide, wie Magnesium- und Calciumoxid, außerdem Alkali- und Erdalkalimetallcarbonate, wie Natriumcarbonat, Kaliumcarbonat und Calciumcarbonat sowie Alkalihydroxide wie Natriumhydroxid und Kaliumhydroxid.

Als Verdünnungsmittel können bei dem erfindungsgemäßen Verfahren (J) alle gegenüber den Chlorameisensäureestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (J) innerhalb eines größeren Bereiches variiert werden. Arbeitet man in Gegenwart eines Verdünnungsmittels und eines Säurebindemittels, so liegen die Reaktionstemperaturen im Allgemeinen zwischen -20°C und +100°C, vorzugsweise zwischen 0°C und 50°C.

Das erfindungsgemäße Verfahren (J) wird im Allgemeinen unter Normaldruck durchgeführt.

Bei der Durchführung des erfindungsgemäßen Verfahrens (J) werden die Ausgangsstoffe der Formeln (I-1-a) bis (I-2-a) und der entsprechende Chlorameisensäureester der Formel (XIII) im Allgemeinen jeweils in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt nach üblichen Methoden. Im Allgemeinen geht man so vor, dass man ausgefallene Salze entfernt und das verbleibende Reaktionsgemisch durch Abziehen des Verdünnungsmittels einengt.

Das erfindungsgemäße Verfahren (L) ist dadurch gekennzeichnet, dass man Verbindungen der Formeln (I-1-a) bis (I-2-a) jeweils mit Sulfonsäurechloriden der Formel (XVII) gegebenenfalls in Gegenwart eines Verdünnungsmittels und gegebenenfalls in Gegenwart eines Säurebindemittels umsetzt.

Beim Herstellungsverfahren (L) setzt man pro Mol Ausgangsverbindung der Formel (I-1-a bis I-2-a) ca. 1 Mol Sulfonsäurechlorid der Formel (XVII) bei -20 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Sulfone, Sulfoxide oder halogenierte Kohlenwasserstoffe wie Methylenchlorid.

Vorzugsweise werden Dimethylsulfoxid, Tetrahydrofuran, Dimethylformamid, Methylenchlorid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindungen (I-1-a) bis (I-2-a) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Das Verfahren (N) ist dadurch gekennzeichnet, dass man Verbindungen der Formeln (I-1-a) bis (I-2-a) mit Metallhydroxiden bzw. Metallalkoxiden der Formel (XIX) oder Aminen der Formel (XX), gegebenenfalls in Gegenwart eines Verdünnungsmittels, umsetzt.

Als Verdünnungsmittel können bei dem erfindungsgemäßen Verfahren (N) vorzugsweise Ether wie Tetrahydrofuran, Dioxan, Diethylether oder aber Alkohole wie Methanol, Ethanol, Iso-propanol, aber auch Wasser eingesetzt werden.

Das erfindungsgemäße Verfahren (N) wird im Allgemeinen unter Normaldruck durchgeführt.

Die Reaktionstemperaturen liegen im Allgemeinen zwischen -20°C und 100°C, vorzugsweise zwischen 0°C und 50°C.

Das Verfahren (P) ist dadurch gekennzeichnet, dass man Verbindungen der Formeln (I-1-a') bis (I-2-a'), in welchen A, B, D, X und Y die oben angegebenen Bedeutungen haben und W' bevorzugt für Brom steht, mit Alkoholen der Formel W-OH in welcher W die oben angegebene Bedeutung hat in Gegenwart einer Base und eines Cu-I-Salzes (z.B. CuBr oder CuJ) umsetzt.

Als Verdünnungsmittel können bei dem erfindungsgemäßen Verfahren (P) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid, Dimethylacetamid und N-Methyl-pyrrolidon, Ester wie Methylacetat, Ethylacetat, Propylacetat sowie Alkohole der Formel W-OH wie z.B. Methanol, Ethanol, Propanol, Iso-Propanol, Butanol, Iso-Butanol, tert.-Butanol, Glykolmonomethylether oder Diethylenglykolmonoethylether.

Als Base (Deprotonierungsmittel) können bei der Durchführung des erfindungsgemäßen Verfahrens (P) alle üblichen Protonenakzeptoren eingesetzt werden. Vorzugsweise verwendbar sind Alkalimetalle wie Natrium oder Kalium. Ferner sind Alkalimetall- und Erdalkalimetallamide und - hydride, wie Natriumamid , Natriumhydrid und Calciumhydrid und bevorzugt auch Alkalimetallalkoholate, wie Natrium-methylat, Natrium-ethylat, Natriumisopropylat, Natrium-tert-butylat und Kalium-tert.-butylat einsetzbar.

Die Reaktionstemperaturen können bei der Durchführung des erfindungsgemäßen Verfahrens (P) innerhalb eines größeren Bereiches variiert werden. Im Allgemeinen arbeitet man bei Temperaturen zwischen 0°C und 250°C, vorzugsweise zwischen 50°C und 150°C. Das erfindungsgemäße Verfahren (P) wird im Allgemeinen unter Normaldruck durchgeführt.

Bei der Durchführung des erfindungsgemäßen Verfahrens (P) setzt man die Reaktionskomponenten der Formel (I-1-a') bis (I-2-a') im Allgemeinen mit Überschüssen der Alkohole W-OH und der Basen bis zu 20 Mol, bevorzugt 3 bis 5 Mol um. Die Kupfer-I-Salze werden in der Regel katalytisch eingesetzt: 0,001 bis 0,5 Mol, bevorzugt 0,01 bis 0,2 Mol. Es ist jedoch auch möglich diese äquimolar einzusetzen.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Acheta domesticus, Gryllotalpa spp., Locusta migratoria migratorioides, Melanoplus spp., Schistocerca gregaria.
Aus der Ordnung der Blattaria z.B. Blatta orientalis, Periplaneta americana, Leucophaea maderae, Blattella germanica. Aus der Ordnung der Dermaptera z.B. Forficula auricularia.
Aus der Ordnung der Isoptera z.B. Reticulitermes spp.
Aus der Ordnung der Phthiraptera z.B. Pediculus humanus corporis, Haematopinus spp., Linognathus spp., Trichodectes spp., Damalinia spp.
Aus der Ordnung der Thysanoptera z.B. Hercinothrips femoralis, Thrips tabaci, Thrips palmi, Frankliniella accidentalis.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium comi, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Oulema oryzae.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp.
Aus der Ordnung der Siphonaptera z.B. Xenopsylla cheopis, Ceratophyllus spp.
Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp.

Die erfindungsgemäßen Verbindungen bzw. Wirkstoffkombinationen können gegebenenfalls in bestimmten Konzentrationen bzw. Aufwandmengen auch als Herbizide verwendet werden. Die Verbindungen lassen sich gegebenenfalls auch als Zwischen- oder Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen bzw. Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Spritzen, Verdampfen, Vernebeln, Streuen, Aufstreichen und bei Vermehrungsmaterial, insbesondere bei Samen, weiterhin durch ein- oder mehrschichtiges Umhüllen.

Die Wirkstoffe bzw. Wirkstoffkombinationen können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkynaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstängeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gew.-% Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

2-Phenylphenol; 8-Hydroxyquinoline sulfate; Acibenzolar-S-methyl; Aldimorph; Amidoflumet; Ampropylfos; Ampropylfos-potassium; Andoprim; Anilazine; Azaconazole; Azoxystrobin; Benalaxyl; Benodanil; Benomyl; Benthiavalicarb-isopropyl; Benzamacril; Benzamacril-isobutyl; Bilanafos; Binapacryl; Biphenyl; Bitertanol; Blasticidin-S; Bromuconazole; Bupirimate; Buthiobate; Butylamine; Calcium polysulfide; Capsimycin; Captafol; Captan; Carbendazim; Carboxin; Carpropamid; Carvone; Chinomethionat; Chlobenthiazone; Chlorfenazole; Chloroneb; Chlorothalonil; Chlozolinate; Clozylacon; Cyazofamid; Cyflufenamid; Cymoxanil; Cyproconazole; Cyprodinil; Cyprofuram; Dagger G; Debacarb; Dichlofluanid; Dichlone; Dichlorophen; Diclocymet; Diclomezine; Dicloran; Diethofencarb; Difenoconazole; Diflumetorim; Dimethirimol; Dimethomorph; Dimoxystrobin; Diniconazole; Diniconazole-M; Dinocap; Diphenylamine; Dipyrithione; Ditalimfos; Dithianon; Dodine; Drazoxolon; Edifenphos; Epoxiconazole; Ethaboxam; Ethirimol; Etridiazole; Famoxadone; Fenamidone; Fenapanil; Fenarimol; Fenbuconazole; Fenfuram; Fenhexamid; Fenitropan; Fenoxanil; Fenpiclonil; Fenpropidin; Fenpropimorph; Ferbam; Fluazinam; Flubenzimine; Fludioxonil; Flumetover; Flumorph; Fluoromide; Fluoxastrobin; Fluquinconazole; Flurprimidol; Flusilazole; Flusulfamide; Flutolanil; Flutriafol; Folpet; Fosetyl-Al; Fosetyl-sodium; Fuberidazole; Furalaxyl; Furametpyr; Furcarbanil; Furmecyclox; Guazatine; Hexachlorobenzene; Hexaconazole; Hymexazol; Imazalil; Imibenconazole; Iminoctadine triacetate; Iminoctadine tris(albesilate); Iodocarb; Ipconazole; Iprobenfos; Iprodione; Iprovalicarb; Irumamycin; Isoprothiolane; Isovaledione; Kasugamycin; Kresoxim-methyl; Mancozeb; Maneb; Meferimzone; Mepanipyrim; Mepronil; Metalaxyl; Metalaxyl-M; Metconazole; Methasulfocarb; Methfuroxam; Metiram; Metominostrobin; Metsulfovax; Mildiomycin; Myclobutanil; Myclozolin; Natamycin; Nicobifen; Nitrothal-isopropyl; Noviflumuron; Nuarimol; Ofurace; Orysastrobin; Oxadixyl; Oxolinic acid; Oxpoconazole; Oxycarboxin; Oxyfenthiin; Paclobutrazol; Pefurazoate; Penconazole; Pencycuron; Phosdiphen; Phthalide; Picoxystrobin; Piperalin; Polyoxins; Polyoxorim; Probenazole; Prochloraz; Procymidone; Propamocarb; Propanosine-sodium; Propiconazole; Propineb; Proquinazid; Prothioconazole; Pyraclostrobin; Pyrazophos; Pyrifenox; Pyrimethanil; Pyroquilon; Pyroxyfur; Pyrrolnitrine; Quinconazole; Quinoxyfen; Quintozene; Simeconazole; Spiroxamine; Sulfur; Tebuconazole; Tecloftalam; Tecnazene; Tetcyclacis; Tetraconazole; Thiabendazole; Thicyofen; Thifluzamide; Thiophanate-methyl; Thiram; Tioxymid; Tolclofos-methyl; Tolylfluanid; Triadimefon; Triadimenol; Triazbutil; Triazoxide; Tricyclamide; Tricyclazole; Tridemorph; Trifloxystrobin; Triflumizole; Triforine; Triticonazole; Uniconazole; Validamycin A; Vinclozolin; Zineb; Ziram; Zoxamide; (2S)-N-[2-[4-[[3-(4-chlorophenyl)-2-propynyl]oxy]-3-methoxyphenyl]-ethyl]-3-methyl- 2-[(methylsulfonyl)amino]-butanamide; 1-(1-naphthalenyl)-1H-pyrrole-2,5-dione; 2,3,5,6-tetrachloro-4-(methylsulfonyl)-pyridine; 2-amino-4-methyl-N-phenyl-5-thiazolecarboxamide; 2-chloro-N-(2,3-dihydro-1,1,3-trimethyl-1H-inden-4-yl)-3-pyridincarboxamide; 3,4,5-trichloro-2,6-pyridinedicarbonitrile; Actinovate; cis-1-(4-chlorophenyl)-2-(1H-1,2,4-triazole-1-yl)-cycloheptanol; methyl 1-(2,3-dihydro-2,2-dimethyl-1H-inden-1-yl)-1H-imidazole-5-carboxylate; monopotassium carbonate; N-(6-methoxy-3-pyridinyl)-cyclopropanecarboxamide; N-butyl-8-(1,1-dimethylethyl)-1-oxaspiro[4.5]decan-3-amine; Sodium tetrathiocarbonate;
sowie Kupfersalze und -zubereitungen, wie Bordeaux mixture; Copper hydroxide; Copper naphthenate; Copper oxychloride; Copper sulfate; Cufraneb; Cuprous oxide; Mancopper; Oxinecopper.

### Bakterizide:

Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, ABG-9008, Acephate, Acequinocyl, Acetamiprid, Acetoprole, Acrinathrin, AKD-1022, AKD-3059, AKD-3088, Alanycarb, Aldicarb, Aldoxycarb, Allethrin, Alpha-Cypermethrin (Alphamethrin), Amidoflumet, Aminocarb, Amitraz, Avermectin, AZ-60541, Azadirachtin, Azamethiphos, Azinphos-methyl, Azinphos-ethyl, Azocyclotin,
Bacillus popilliae, Bacillus sphaericus, Bacillus subtilis, Bacillus thuringiensis, Bacillus thuringiensis strain EG-2348, Bacillus thuringiensis strain GC-91, Bacillus thuringiensis strain NCTC-11821, Baculoviren, Beauveria bassiana, Beauveria tenella, Benclothiaz, Bendiocarb, Benfuracarb, Bensultap, Benzoximate, Beta-Cyfluthrin, Beta-Cypermethrin, Bifenazate, Bifenthrin, Binapacryl, Bioallethrin, Bioallethrin-S-cyclopentyl-isomer, Bioethanomethrin, Biopermethrin, Bioresmethrin, Bistrifluron, BPMC, Brofenprox, Bromophos-ethyl, Bromopropylate, Bromfenvinfos (-methyl), BTG-504, BTG-505, Bufencarb, Buprofezin, Butathiofos, Butocarboxim, Butoxycarboxim, Butylpyridaben,
Cadusafos, Camphechlor, Carbaryl, Carbofuran, Carbophenothion, Carbosulfan, Cartap, CGA-50439, Chinomethionat, Chlordane, Chlordimeform, Chloethocarb, Chlorethoxyfos, Chlorfenapyr, Chlorfenvinphos, Chlorfluazuron, Chlormephos, Chlorobenzilate, Chloropicrin, Chlorproxyfen, Chlorpyrifosmethyl, Chlorpyrifos (-ethyl), Chlovaporthrin, Chromafenozide, Cis-Cypermethrin, Cis-Resmethrin, Cis-Permethrin, Clocythrin, Cloethocarb, Clofentezine, Clothianidin, Clothiazoben, Codlemone, Coumaphos, Cyanofenphos, Cyanophos, Cycloprene, Cycloprothrin, Cydia pomonella, Cyfluthrin, Cyhalothrin, Cyhexatin, Cypermethrin, Cyphenothrin (1R-trans-isomer), Cyromazine,
DDT, Deltamethrin, Demeton-S-methyl, Demeton-S-methylsulphon, Diafenthiuron, Dialifos, Diazinon, Dichlofenthion, Dichlorvos, Dicofol, Dicrotophos, Dicyclanil, Diflubenzuron, Dimefluthrin, Dimethoate, Dimethylvinphos, Dinobuton, Dinocap, Dinotefuran, Diofenolan, Disulfoton, Docusatsodium, Dofenapyn, DOWCO-439,
Eflusilanate, Emamectin, Emamectin-benzoate, Empenthrin (1R-isomer), Endosulfan, Entomopthora spp., EPN, Esfenvalerate, Ethiofencarb, Ethiprole, Ethion, Ethoprophos, Etofenprox, Etoxazole, Etrimfos,
Famphur, Fenamiphos, Fenazaquin, Fenbutatin oxide, Fenfluthrin, Fenitrothion, Fenobucarb, Fenothiocarb, Fenoxacrim, Fenoxycarb, Fenpropathrin, Fenpyrad, Fenpyrithrin, Fenpyroximate, Fensulfothion, Fenthion, Fentrifanil, Fenvalerate, Fipronil, Flonicamid, Fluacrypyrim, Fluazuron, Flubenzimine, Flubrocythrinate, Flucycloxuron, Flucythrinate, Flufenerim, Flufenoxuron, Flufenprox, Flumethrin, Flupyrazofos, Flutenzin (Flufenzine), Fluvalinate, Fonofos, Formetanate, Formothion, Fosmethilan, Fosthiazate, Fubfenprox (Fluproxyfen), Furathiocarb,
Gamma-Cyhalothrin, Gamma-HCH, Gossyplure, Grandlure, Granuloseviren,
Halfenprox, Halofenozide, HCH, HCN-801, Heptenophos, Hexaflumuron, Hexythiazox, Hydramethylnone, Hydroprene,
IKA-2002, Imidacloprid, Imiprothrin, Indoxacarb, Iodofenphos, Iprobenfos, Isazofos, Isofenphos, Isoprocarb, Isoxathion, Ivermectin,
Japonilure,
Kadethrin, Kernpolyederviren, Kinoprene,
Lambda-Cyhalothrin, Lindane, Lufenuron,
Malathion, Mecarbam, Mesulfenfos, Metaldehyd, Metam-sodium, Methacrifos, Methamidophos, Metharhizium anisopliae, Metharhizium flavoviride, Methidathion, Methiocarb, Methomyl, Methoprene, Methoxychlor, Methoxyfenozide, Metofluthrin, Metolcarb, Metoxadiazone, Mevinphos, Milbemectin, Milbemycin, MKI-245, MON-45700, Monocrotophos, Moxidectin, MTI-800,
Naled, NC-104, NC-170, NC-184, NC-194, NC-196, Niclosamide, Nicotine, Nitenpyram, Nithiazine, NNI-0001, NNI-0101, NNI-0250, NNI-9768, Novaluron, Noviflumuron,
OK-5101, OK-5201, OK-9601, OK-9602, OK-9701, OK-9802, Omethoate, Oxamyl, Oxydemetonmethyl,
Paecilomyces fumosoroseus, Parathion-methyl, Parathion (-ethyl), Permethrin (cis-, trans-), Petroleum, PH-6045, Phenothrin (1R-trans isomer), Phenthoate, Phorate, Phosalone, Phosmet, Phosphamidon, Phosphocarb, Phoxim, Piperonyl butoxide, Pirimicarb, Pirimiphos-methyl, Pirimiphos-ethyl, Potassium oleate, Prallethrin, Profenofos, Profluthrin, Promecarb, Propaphos, Propargite, Propetamphos, Propoxur, Prothiofos, Prothoate, Protrifenbute, Pymetrozine, Pyraclofos, Pyresmethrin, Pyrethrum, Pyridaben, Pyridalyl, Pyridaphenthion, Pyridathion, Pyrimidifen, Pyriproxyfen,
Quinalphos,
Resmethrin, RH-5849, Ribavirin, RU-12457, RU-15525,
S-421, S-1833, Salithion, Sebufos, SI-0009, Silafluofen, Spinosad, Spirodiclofen, Spiromesifen, Spirotetramat, Sulfluramid, Sulfotep, Sulprofos, SZI-121,
Tau-Fluvalinate, Tebufenozide, Tebufenpyrad, Tebupirimfos, Teflubenzuron, Tefluthrin, Temephos, Temivinphos, Terbam, Terbufos, Tetrachlorvinphos, Tetradifon, Tetramethrin, Tetramethrin (IRisomer), Tetrasul, Theta-Cypermethrin, Thiacloprid, Thiamethoxam, Thiapronil, Thiatriphos, Thiocyclam hydrogen oxalate, Thiodicarb, Thiofanox, Thiometon, Thiosultap-sodium, Thuringiensin, Tolfenpyrad, Tralocythrin, Tralomethrin, Transfluthrin, Triarathene, Triazamate, Triazophos, Triazuron, Trichlophenidine, Trichlorfon, Trichoderma atroviride, Triflumuron, Trimethacarb,
Vamidothion, Vaniliprole, Verbutin, Verticillium lecanii,
WL-108477, WL-40027,
YI-5201, YI-5301, YI-5302,
XMC, Xylylcarb,
ZA-3274, Zeta-Cypermethrin, Zolaprofos, ZXI-8901,
die Verbindung 3-Methyl-phenyl-propylcarbarnat (Tsumacide Z),
die Verbindung 3-(5-Chlor-3-pyridinyl)-8-(2,2,2-trifluorethyl)-8-azabicyclo[3.2.1]octan-3-carbonitril (CAS-Reg.-Nr. 185982-80-3) und das entsprechende 3-endo-Isomere (CAS-Reg.-Nr. 185984-60-5) (vgl. WO-96/37494, WO-98/25923),
sowie Präparate, welche insektizid wirksame Pflanzenextrakte, Nematoden, Pilze oder Viren enthalten.

Auch eine Mischung mit anderen bekannten Wirkstoffen, wie Herbiziden oder mit Düngemitteln und Wachstumsregulatoren ist möglich.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne dass der zugesetzte Synergist selbst aktiv wirksam sein muss.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.% liegen.

Die Anwendung geschieht in einer den Anwendungsformen angepaßten üblichen Weise.

Bei der Anwendung gegen Hygiene- und Vorratsschädlinge zeichnet sich der Wirkstoff bzw. Wirkstoffkombinationen durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im Folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit den erfindungsgemäßen Verbindungen bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die erfindungsgemäßen Wirkstoffe bzw. Wirkstoffkombinationen wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..
Aus der Ordnung der Siphonapterida z.B. Pulex spp., Ctenocephalides spp., Xenopsylla spp., Ceratophyllus spp..
Aus der Ordnung der Heteropterida z.B. Cimex spp., Triatoma spp., Rhodnius spp., Panstrongylus spp.. Aus der Ordnung der Blattarida z.B. Blatta orientalis, Periplaneta americana, Blattela germanica, Supella spp..
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe bzw. Wirkstoffkombinationen eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so dass durch den Einsatz der erfindungsgemäßen Wirkstoffe eine wirtschaftlichere und einfachere Tierhaltung möglich ist.

Die Anwendung der erfindungsgemäßen Wirkstoffe bzw. Wirkstoffkombinationen geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe bzw. Wirkstoffkombinationen als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, dass die erfindungsgemäßen Verbindungen bzw. Wirkstoffkombinationen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie
   Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus.
   Hautflügler wie Sirexjuvencus, Urocerus gigas, Urocerus gigas taignus, Urocerus augur.
   Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus.
Borstenschwänze wie Lepisma saccharina.
   Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel. Ganz besonders bevorzugt handelt es sich bei dem vor Insektenbefall zu schützenden Material um Holz und Holzverarbeitungsprodukte.
   Unter Holz und Holzverarbeitungsprodukten, welche durch das erfindungsgemäße Mittel bzw. dieses enthaltende Mischungen geschützt werden kann, ist beispielhaft zu verstehen:
   Bauholz, Holzbalken, Eisenbahnschwellen, Brückenteile, Bootsstege, Holzfahrzeuge, Kisten, Paletten, Container, Telefonmasten, Holzverkleidungen, Holzfenster und -türen, Sperrholz, Spanplatten, Tischlerarbeiten oder Holzprodukte, die ganz allgemein beim Hausbau oder in der Bautischlerei Verwendung finden.

Die Wirkstoffe bzw. Wirkstoffkombinationen können als solche, in Form von Konzentraten oder allgemein üblichen Formulierungen wie Pulver, Granulate, Lösungen, Suspensionen, Emulsionen oder Pasten angewendet werden.

Die genannten Formulierungen können in an sich bekannter Weise hergestellt werden, z.B. durch Vermischen der Wirkstoffe mit mindestens einem Lösungs- bzw. Verdünnungsmittel, Emulgator, Dispergier- und/oder Binde- oder Fixiermittels, Wasser-Repellent, gegebenenfalls Sikkative und UV-Stabilisatoren und gegebenenfalls Farbstoffen und Pigmenten sowie weiteren Verarbeitungshilfsmitteln. Die zum Schutz von Holz und Holzwerkstoffen verwendeten insektiziden Mittel oder Konzentrate enthalten den erfindungsgemäßen Wirkstoff in einer Konzentration von 0,0001 bis 95 Gew.-%, insbesondere 0,001 bis 60 Gew.-%.

Die Menge der eingesetzten Mittel bzw. Konzentrate ist von der Art und dem Vorkommen der Insekten und von dem Medium abhängig. Die optimale Einsatzmenge kann bei der Anwendung jeweils durch Testreihen ermittelt werden. Im Allgemeinen ist es jedoch ausreichend 0,0001 bis 20 Gew.-%, vorzugsweise 0,001 bis 10 Gew.-%, des Wirkstoffs, bezogen auf das zu schützende Material, einzusetzen.

Als Lösungs- und/oder Verdünnungsmittel dient ein organisch-chemisches Lösungsmittel oder Lösungsmittelgemisch und/oder ein öliges oder ölartiges schwer flüchtiges organisch-chemisches Lösungsmittel oder Lösungsmittelgemisch und/oder ein polares organisch-chemisches Lösungsmittel oder Lösungsmittelgemisch und/oder Wasser und gegebenenfalls einen Emulgator und/oder Netzmittel.

Als organisch-chemische Lösungsmittel werden vorzugsweise ölige oder ölartige Lösungsmittel mit einer Verdunstungszahl über 35 und einem Flammpunkt oberhalb 30°C, vorzugsweise oberhalb 45°C, eingesetzt. Als derartige schwerflüchtige, wasserunlösliche, ölige und ölartige Lösungsmittel werden entsprechende Mineralöle oder deren Aromatenfraktionen oder mineralölhaltige Lösungsmittelgemische, vorzugsweise Testbenzin, Petroleum und/oder Alkylbenzol verwendet.

Vorteilhaft gelangen Mineralöle mit einem Siedebereich von 170 bis 220°C, Testbenzin mit einem Siedebereich von 170 bis 220°C, Spindelöl mit einem Siedebereich von 250 bis 350°C, Petroleum bzw. Aromaten vom Siedebereich von 160 bis 280°C, Terpentinöl und dgl. zum Einsatz.

In einer bevorzugten Ausführungsform werden flüssige aliphatische Kohlenwasserstoffe mit einem Siedebereich von 180 bis 210°C oder hochsiedende Gemische von aromatischen und aliphatischen Kohlenwasserstoffen mit einem Siedebereich von 180 bis 220°C und/oder Spindeöl und/oder Monochlornaphthalin, vorzugsweise α-Monochlornaphthalin, verwendet.

Die organischen schwerflüchtigen öligen oder ölartigen Lösungsmittel mit einer Verdunstungszahl über 35 und einem Flammpunkt oberhalb 30°C, vorzugsweise oberhalb 45°C, können teilweise durch leicht oder mittelflüchtige organisch-chemische Lösungsmittel ersetzt werden, mit der Maßgabe, dass das Lösungsmittelgemisch ebenfalls eine Verdunstungszahl über 35 und einen Flammpunkt oberhalb 30°C, vorzugsweise oberhalb 45°C, aufweist und dass das Insektizid-Fungizid-Gemisch in diesem Lösungsmittelgemisch löslich oder emulgierbar ist.

Nach einer bevorzugten Ausführungsform wird ein Teil des organisch-chemischen Lösungsmittel oder Lösungsmittelgemisches durch ein aliphatisches polares organisch-chemisches Lösungsmittel oder Lösungsmittelgemisch ersetzt. Vorzugsweise gelangen Hydroxyl- und/oder Ester- und/oder Ethergruppen enthaltende aliphatische organisch-chemische Lösungsmittel wie beispielsweise Glycolether, Ester oder dgl. zur Anwendung.

Als organisch-chemische Bindemittel werden im Rahmen der vorliegenden Erfindung die an sich bekannten wasserverdünnbaren und/oder in den eingesetzten organisch-chemischen Lösungsmitteln löslichen oder dispergier- bzw. emulgierbaren Kunstharze und/oder bindende trocknende Öle, insbesondere Bindemittel bestehend aus oder enthaltend ein Acrylatharz, ein Vinylharz, z.B. Polyvinylacetat, Polyesterharz, Polykondensations- oder Polyadditionsharz, Polyurethanharz, Alkydharz bzw. modifiziertes Alkydharz, Phenolharz, Kohlenwasserstoffharz wie Inden-Cumaronharz, Siliconharz, trocknende pflanzliche und/oder trocknende Öle und/oder physikalisch trocknende Bindemittel auf der Basis eines Natur- und/oder Kunstharzes verwendet.

Das als Bindemittel verwendete Kunstharz kann in Form einer Emulsion, Dispersion oder Lösung, eingesetzt werden. Als Bindemittel können auch Bitumen oder bituminöse Substanzen bis zu 10 Gew.-%, verwendet werden. Zusätzlich können an sich bekannte Farbstoffe, Pigmente, wasserabweisende Mittel, Geruchskorrigentien und Inhibitoren bzw. Korrosionsschutzmittel und dgl. eingesetzt werden.

Bevorzugt ist gemäß der Erfindung als organisch-chemische Bindemittel mindestens ein Alkydharz bzw. modifiziertes Alkydharz und/oder ein trocknendes pflanzliches Öl im Mittel oder im Konzentrat enthalten. Bevorzugt werden gemäß der Erfindung Alkydharze mit einem Ölgehalt von mehr als 45 Gew.-%, vorzugsweise 50 bis 68 Gew.-%, verwendet.

Das erwähnte Bindemittel kann ganz oder teilweise durch ein Fixierungsmittel(gemisch) oder ein Weichmacher(gemisch) ersetzt werden. Diese Zusätze sollen einer Verflüchtigung der Wirkstoffe sowie einer Kristallisation bzw. Ausfällem vorbeugen. Vorzugsweise ersetzen sie 0,01 bis 30 % des Bindemittels (bezogen auf 100 % des eingesetzten Bindemittels).

Die Weichmacher stammen aus den chemischen Klassen der Phthalsäureester wie Dibutyl-, Dioctyl- oder Benzylbutylphthalat, Phosphorsäureester wie Tributylphosphat, Adipinsäureester wie Di-(2-ethylhexyl)-adipat, Stearate wie Butylstearat oder Amylstearat, Oleate wie Butyloleat, Glycerinether oder höhermolekulare Glykolether, Glycerinester sowie p-Toluolsulfonsäureester.

Fixierungsmittel basieren chemisch auf Polyvinylalkylethern wie z.B. Polyvinylmethylether oder Ketonen wie Benzophenon, Ethylenbenzophenon.

Als Lösungs- bzw. Verdünnungsmittel kommt insbesondere auch Wasser in Frage, gegebenenfalls in Mischung mit einem oder mehreren der oben genannten organisch-chemischen Lösungs- bzw. Verdünnungsmittel, Emulgatoren und Dispergatoren.

Ein besonders effektiver Holzschutz wird durch großtechnische Imprägnierverfahren, z.B. Vakuum, Doppelvakuum oder Druckverfahren, erzielt.

Die anwendungsfertigen Mittel können gegebenenfalls noch weitere Insektizide und gegebenenfalls noch ein oder mehrere Fungizide enthalten.

Als zusätzliche Zumischpartner kommen vorzugsweise die in der WO 94/29 268 genannten Insektizide und Fungizide in Frage. Die in diesem Dokument genannten Verbindungen sind ausdrücklicher Bestandteil der vorliegenden Anmeldung.

Als ganz besonders bevorzugte Zumischpartner können Insektizide, wie Chlorpyriphos, Phoxim, Silafluofin, Alphamethrin, Cyfluthrin, Cypermethrin, Deltamethrin, Permethrin, Imidacloprid, NI-25, Flufenoxuron, Hexaflumuron, Transfluthrin, Thiacloprid, Methoxyphenoxid und Triflumuron,
sowie Fungizide wie Epoxyconazole, Hexaconazole, Azaconazole, Propiconazole, Tebuconazole, Cyproconazole, Metconazole, Imazalil, Dichlorfluanid, Tolylfluanid, 3-Iod-2-propinyl-butyl-carbamat, N-Octyl-isothiazolin-3-on und 4,5-Dichlor-N-octylisothiazolin-3-on, sein.

Zugleich können die erfindungsgemäßen Verbindungen bzw. Wirkstoffkombinationen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Bewuchs durch sessile Oligochaeten, wie Kalkröhrenwürmer sowie durch Muscheln und Arten der Gruppe Ledamorpha (Entenmuscheln), wie verschiedene Lepas- und Scalpellum-Arten, oder durch Arten der Gruppe Balanomorpha (Seepocken), wie Balanus- oder Pollicipes-Species, erhöht den Reibungswiderstand von Schiffen und führt in der Folge durch erhöhten Energieverbrauch und darüber hinaus durch häufige Trockendockaufenthalte zu einer deutlichen Steigerung der Betriebskosten.

Neben dem Bewuchs durch Algen, beispielsweise Ectocarpus sp. und Ceramium sp., kommt insbesondere dem Bewuchs durch sessile Entomostraken-Gruppen, welche unter dem Namen Cirripedia (Rankenflusskrebse) zusammengefasst werden, besondere Bedeutung zu.

Es wurde nun überraschenderweise gefunden, dass die erfindungsgemäßen Verbindungen allein oder in Kombination mit anderen Wirkstoffen, eine hervorragende Antifouling (Antibewuchs)-Wirkung aufweisen.

Durch Einsatz von erfindungsgemäßen Verbindungen allein oder in Kombination mit anderen Wirkstoffen, kann auf den Einsatz von Schwermetallen wie z.B. in Bis(trialkylzinn)-sulfiden, Tri-n-butylzinnlaurat, Tri-n-butylzinnchlorid, Kupfer(I)-oxid, Triethylzinnchlorid, Tri-n-butyl(2-phenyl-4-chlorphenoxy)-zinn, Tributylzinnoxid, Molybdändisulfid, Antimonoxid, polymerem Butyltitanat, Phenyl-(bispyridin)-wismutchlorid, Tri-n-butylzinnfluorid, Manganethylenbisthiocarbamat, Zinkdimethyldithiocarbamat, Zinkethylenbisthiocarbamat, Zink- und Kupfersalze von 2-Pyridin-thiol-1-oxid, Bisdimethyldithiocarbamoylzinkethylenbisthiocarbamat, Zinkoxid, Kupfer(I)-ethylen-bisdithiocarbamat, Kupferthiocyanat, Kupfemaphthenat und Tributylzinnhalogeniden verzichtet werden oder die Konzentration dieser Verbindungen entscheidend reduziert werden.

Die anwendungsfertigen Antifoulingfarben können gegebenenfalls noch andere Wirkstoffe, vorzugsweise Algizide, Fungizide, Herbizide, Molluskizide bzw. andere Antifouling-Wirkstoffe enthalten.

Als Kombinationspartner für die erfindungsgemäßen Antifouling-Mittel eignen sich vorzugsweise:
Algizide wie
   2-*tert*.-Butylamino-4-cyclopropylamino-6-methylthio-1,3,5-triazin, Dichlorophen, Diuron, Endothal, Fentinacetat, Isoproturon, Methabenzthiazuron, Oxyfluorfen, Quinoclamine und Terbutryn;
Fungizide wie
   Benzo[b]thiophencarbonsäurecyclohexylamid-S,S-dioxid, Dichlofluanid, Fluorfolpet, 3-Iod-2-propinyl-butylcarbamat Tolylfluanid und Azole wie
   Azaconazole, Cyproconazole, Epoxyconazole, Hexaconazole, Metconazole, Propiconazole und Tebuconazole;
Molluskizide wie
   Fentinacetat, Metaldehyd, Methiocarb, Niclosamid, Thiodicarb und Trimethacarb;
   oder herkömmliche Antifouling-Wirkstoffe wie
   4,5-Dichlor-2-octyl-4-isothiazolin-3-on, Diiodmethylparatrylsulfon, 2-(N,N-Dimethylthiocarbamo-ylthio)-5-nitrothiazyl, Kalium-, Kupfer-, Natrium- und Zinksalze von 2-Pyridinthiol-1-oxid, Pyridin-triphenylboran, Tetrabutyldistannoxan, 2,3,5,6-Tetrachlor-4-(methylsulfonyl)-pyridin, 2,4,5,6-Tetrachloroisophthalonitril, Tetramethylthiuramdisulfid und 2,4,6-Trichlorphenylmaleinimid.

Die verwendeten Antifouling-Mittel enthalten die erfindungsgemäßen Wirkstoff der erfindungsgemäßen Verbindungen in einer Konzentration von 0,001 bis 50 Gew.-%, insbesondere von 0,01 bis 20 Gew.-%.

Die erfindungsgemäßen Antifouling-Mittel enthalten desweiteren die üblichen Bestandteile wie z.B. in Ungerer, Chem. Ind. 1985, 37, 730-732 und Williams, Antifouling Marine Coatings, Noyes, Park Ridge, 1973 beschrieben.

Antifouling-Anstrichmittel enthalten neben den algiziden, fungiziden, molluskiziden und erfindungsgemäßen insektiziden Wirkstoffen insbesondere Bindemittel.

Beispiele für anerkannte Bindemittel sind Polyvinylchlorid in einem Lösungsmittelsystem, chlorierter Kautschuk in einem Lösungsmittelsystem, Acrylharze in einem Lösungsmittelsystem insbesondere in einem wässrigen System, Vinylchlorid/Vinylacetat-Copolymersysteme in Form wässriger Dispersionen oder in Form von organischen Lösungsmittelsystemen, Butadien/Styrol/Acrylnitril-Kautschuke, trocknende Öle, wie Leinsamenöl, Harzester oder modifizierte Hartharze in Kombination mit Teer oder Bitumina, Asphalt sowie Epoxyverbindungen, geringe Mengen Chlorkautschuk, chloriertes Polypropylen und Vinylharze.

Gegebenenfalls enthalten Anstrichmittel auch anorganische Pigmente, organische Pigmente oder Farbstoffe, welche vorzugsweise in Seewasser unlöslich sind. Ferner können Anstrichmittel Materialien, wie Kolophonium enthalten, um eine gesteuerte Freisetzung der Wirkstoffe zu ermöglichen. Die Anstriche können ferner Weichmacher, die rheologischen Eigenschaften beeinflussende Modifizierungsmittel sowie andere herkömmliche Bestandteile enthalten. Auch in Self-Polishing-Antifouling-Systemen können die erfindungsgemäßen Verbindungen oder die oben genannten Mischungen eingearbeitet werden.

Die Wirkstoffe bzw. Wirkstoffkombinationen eignen sich auch zur Bekämpfung von tierischen Schädlingen, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.
Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.
Aus der Ordnung der Araneae z.B. Aviculariidae, Araneidae.
Aus der Ordnung der Opiliones z.B. Pseudoscorpiones chelifer, Pseudoscorpiones cheiridium, Opiliones phalangium.
Aus der Ordnung der Isopoda z.B. Oniscus asellus, Porcellio scaber.
Aus der Ordnung der Diplopoda z.B. Blaniulus guttulatus, Polydesmus spp..
Aus der Ordnung der Chilopoda z.B. Geophilus spp..
Aus der Ordnung der Zygentoma z.B. Ctenolepisma spp., Lepisma saccharina, Lepismodes inquilinus.
Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.
Aus der Ordnung der Saltatoria z.B. Acheta domesticus. Aus der Ordnung der Dermaptera z.B. Forficula auricularia. Aus der Ordnung der Isoptera z.B. Kalotermes spp., Reticulitermes spp.
Aus der Ordnung der Psocoptera z.B. Lepinatus spp., Liposcelis spp.
Aus der Ordnung der Coleptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.
Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.
Aus der Ordnung der Lepidoptera z.B. Achroia grisella, Galleria mellonella, Plodia interpunctella, Tinea cloacella, Tinea pellionella, Tineola bisselliella.
Aus der Ordnung der Siphonaptera z.B. Ctenocephalides canis, Ctenocephalides felis, Pulex irritans, Tunga penetrans, Xenopsylla cheopis.
Aus der Ordnung der Hymenoptera z.B. Camponotus herculeanus, Lasius fuliginosus, Lasius niger, Lasius umbratus, Monomorium pharaonis, Paravespula spp., Tetramorium caespitum.
Aus der Ordnung der Anoplura z.B. Pediculus humanus capitis, Pediculus humanus corporis, Phthirus pubis.
Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködem oder Köderstationen.

Die erfindungsgemäßen Wirkstoffe bzw. Wirkstoffkombinationen können auch als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe bzw. Wirkstoffkombinationen können z.B. bei den folgenden Pflanzen verwendet werden:
Dikotyle Unkräuter der Gattungen: Abutilon, Amaranthus, Ambrosia, Anoda, Anthemis, Aphanes, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.
Dikotyle Kulturen der Gattungen: Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia.
Monokotyle Unkräuter der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.
Monokotyle Kulturen der Gattungen: Allium, Ananas, Asparagus, Avena, Hordeum, Oryza, Panicum, Saccharum, Secale, Sorghum, Triticale, Triticum, Zea.

Die Verwendung der erfindungsgemäßen Wirkstoffe bzw. Wirkstoffkombinationen ist jedoch keineswegs auf diese Gattungen beschränkt, sondern erstreckt sich in gleicher Weise auch auf andere Pflanzen.

Die erfindungsgemäßen Wirkstoffe bzw. Wirkstoffkombinationen eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen bzw. Wirkstoffkombinationen zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die erfindungsgemäßen Wirkstoffe bzw. Wirkstoffkombinationen können in bestimmten Konzentrationen bzw. Aufwandmengen auch zur Bekämpfung von tierischen Schädlingen und pilzlichen oder bakteriellen Pflanzenkrankheiten verwendet werden. Sie lassen sich gegebenenfalls auch als Zwischen- oder Vorprodukte für die Synthese weiterer Wirkstoffe einsetzen.

Die Wirkstoffe bzw. Wirkstoffkombinationen können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden und/oder mit Stoffen, welche die Kulturpflanzen-Verträglichkeit verbessern ("Safenern") zur Unkrautbekämpfung verwendet werden, wobei Fertigformulierungen oder Tankmischungen möglich sind. Es sind also auch Mischungen mit Unkrautbekämpfungsmitteln möglich, welche ein oder mehrere bekannte Herbizide und einen Safener enthalten.

Für die Mischungen kommen bekannte Herbizide infrage, beispielsweise
Acetochlor, Acifluorfen (-sodium), Aclonifen, Alachlor, Alloxydim (-sodium), Ametryne, Amicarbazone, Amidochlor, Amidosulfuron, Anilofos, Asulam, Atrazine, Azafenidin, Azimsulfuron, Beflubutamid, Benazolin (-ethyl), Benfuresate, Bensulfuron (-methyl), Bentazon, Benzfendizone, Benzobicyclon, Benzofenap, Benzoylprop (-ethyl), Bialaphos, Bifenox, Bispyribac (-sodium), Bromobutide, Bromofenoxim, Bromoxynil, Butachlor, Butafenacil (-allyl), Butroxydim, Butylate, Cafenstrole, Caloxydim, Carbetamide, Carfentrazone (-ethyl), Chlomethoxyfen, Chloramben, Chloridazon, Chlorimuron (-ethyl), Chlornitrofen, Chlorsulfuron, Chlortoluron, Cinidon (-ethyl), Cinmethylin, Cinosulfuron, Clefoxydim, Clethodim, Clodinafop (-propargyl), Clomazone, Clomeprop, Clopyralid, Clopyrasulfuron (-methyl), Cloransulam (-methyl), Cumyluron, Cyanazine, Cybutryne, Cycloate, Cyclosulfamuron, Cycloxydim, Cyhalofop (-butyl), 2,4-D, 2,4-DB, Desmedipham, Diallate, Dicamba, Dichlorprop (-P), Diclofop (-methyl), Diclosulam, Diethatyl (-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron (-methyl), Ethofumesate, Ethoxyfen, Ethoxysulfuron, Etobenzanid, Fenoxaprop (-P-ethyl), Fentrazamide, Flamprop (-isopropyl, -isopropyl-L, -methyl), Flazasulfuron, Florasulam, Fluazifop (-P-butyl), Fluazolate, Flucarbazone (-sodium), Flufenacet, Flumetsulam, Flumiclorac (-pentyl), Flumioxazin, Flumipropyn, Flumetsulam, Fluometuron, Fluorochloridone, Fluoroglycofen (-ethyl), Flupoxam, Flupropacil, Flurpyrsulfuron (-methyl, -sodium), Flurenol (-butyl), Fluridone, Fluroxypyr (-butoxypropyl, -meptyl), Flurprimidol, Flurtamone, Fluthiacet (-methyl), Fluthiamide, Fomesafen, Foramsulfuron, Glufosinate (-ammonium), Glyphosate (-isopropylammonium), Halosafen, Haloxyfop (-ethoxyethyl, - P-methyl), Hexazinone, Imazamethabenz (-methyl), Imazamethapyr, Imazamox, Imazapic, Imazapyr, Imazaquin, Imazethapyr, Imazosulfuron, Iodosulfuron (-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, Mecoprop, Mefenacet, Mesosulfurone, Mesotrione, Metamitron, Metazachlor, Methabenzthiazuron, Metobenzuron, Metobromuron, (alpha-) Metolachlor, Metosulam, Metoxuron, Metribuzin, Metsulfuron (-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Picolinafen, Pinoxaden, Piperophos, Pretilachlor, Primisulfuron (-methyl), Profluazol, Prometryn, Propachlor, Propanil, Propaquizafop, Propisochlor, Propoxycarbazone (-sodium), Propyzamide, Prosulfocarb, Prosulfuron, Pyraflufen (-ethyl), Pyrazogyl, Pyrazolate, Pyrazosulfuron (-ethyl), Pyrazoxyfen, Pyribenzoxim, Pyributicarb, Pyridate, Pyridatol, Pyriftalid, Pyriminobac (-methyl), Pyrithiobac (-sodium), Quinchlorac, Quinmerac, Quinoclamine, Quizalofop (-P-ethyl, -P-tefuryl), Rimsulfuron, Sethoxydim, Simazine, Simetryn, Sulcotrione, Sulfentrazone, Sulfometuron (-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron (-methyl), Thiobencarb, Tiocarbazil, Tralkoxydim, Triallate, Triasulfuron, Tribenuron (-methyl), Triclopyr, Tridiphane, Trifluralin, Trifloxysulfuron, Triflusulfuron (-methyl), Tritosulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe bzw. Wirkstoffkombinationen können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe bzw. Wirkstoffkombinationen können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im Allgemeinen liegen die Aufwandmengen zwischen 1 g und 10 kg Wirkstoff pro Hektar Bodenfläche, vorzugsweise zwischen 5 g und 5 kg pro ha.

Der vorteilhafte Effekt der Kulturpflanzen-Verträglichkeit der erfindungsgemäßen Wirkstoffkombinationen ist bei bestimmten Konzentrationsverhältnissen besonders stark ausgeprägt. Jedoch können die Gewichtsverhältnisse der Wirkstoffe in den Wirkstoffkombinationen in relativ großen Bereichen variiert werden. Im allgemeinen entfallen auf 1 Gewichtsteil Wirkstoff der Formel (I) Salzen 0,001 bis 1000 Gewichtsteile, vorzugsweise 0,01 bis 100 Gewichtsteile, besonders bevorzugt 0,05 bis 20 Gewichtsteile einer der oben unter (b') genannten, die Kulturpflanzen Verträglichkeit verbessernden Verbindungen (Antidots/Safener).

Die erfindungsgemäßen Wirkstoffkombinationen werden im allgemeinen in Form von Fertigformulierungen zur Anwendung gebracht. Die in den Wirkstoffkombinationen enthaltenen Wirkstoffe können aber auch in Einzelformulierungen bei der Anwendung gemischt, d.h. in Form von Tankmischungen zur Anwendung gebracht werden.

Für bestimmte Anwendungszwecke, insbesondere im Nachauflauf-Verfahren, kann es ferner vorteilhaft sein, in die Formulierungen als weitere Zusatzstoffe pflanzenverträgliche mineralische oder vegetabilische Öle (z.B. das Handelspräparat "Rako Binol") oder Ammoniumsalze wie z.B. Ammoniumsulfat oder Ammoniumrhodanid aufzunehmen.

Die neuen Wirkstoffkombinationen können als solche, in Form ihrer Formulierungen oder der daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Stäuben oder Streuen.

Die Aufwandmengen der erfindungsgemäßen Wirkstoffkombinationen können in einem gewissen Bereich variiert werden; sie hängen u.a. vom Wetter und von den Bodenfaktoren ab. Im allgemeinen liegen die Aufwandmengen zwischen 0,001 und 5 kg pro ha, vorzugsweise zwischen 0,005 und 2 kg pro ha, besonders bevorzugt zwischen 0,01 und 0,5 kg pro ha.

Die erfindungsgemäßen Wirkstoffkombinationen können vor und nach dem Auflaufen der Pflanzen appliziert werden, also im Vorauflauf und Nachauflauf-Verfahren.

Die erfindungsgemäß einzusetzenden Safener können je nach ihren Eigenschaften zur Vorbehandlung des Saatgutes der Kulturpflanze (Beizung der Samen) verwendet werden oder vor der Saat in die Saatfurchen eingebracht oder vor dem Herbizid separat angewendet werden oder zusammen mit dem Herbizid vor oder nach dem Ablaufen der Pflanzen angewendet werden.

Als Beispiele der Pflanzen werden die wichtigen Kulturpflanzen, wie Getreide (Weizen, Reis), Mais, Soja, Kartoffel, Baumwolle, Raps, Rüben, Zuckerrohr sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden.

Die Herstellung und die Verwendung der erfindungsgemäßen Wirkstoffe geht aus den nachfolgenden Beispielen hervor.

### Herstellungsbeispiele

### Beispiel-Nr. I-1-a-1

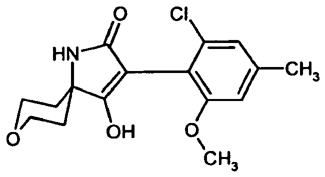

In einem 100 ml Dreihalskolben mit Thermometer und Rückflusskühler werden unter Argon 2,6 g Kalium-tert.-butylat-95 %ig (21,6 mMol) in 8 ml Dimethylacetamid vorgelegt. Bei 50-60°C tropft man 3,5 g (9,8 mMol) der Verbindung gemäß Beispiel II-1 in 10 ml Dimethylacetamid zu. Man rührt unter dünnschichtchromatographischer Kontrolle 1 h. Das Reaktionsgemisch wird in 100 ml Eiswasser eingerührt; mit konz. HCl auf pH 2 gestellt und der Niederschlag abgesaugt. Es erfolgt säulenchromatographische Reinigung an Kieselgel, (Dichlormethan: Essigsäureethylester = 5 : 3).
Ausbeute: 3,15 g (98 % der Theorie), Fp.: 193°C

In Analogie zu Beispiel (I-1-a-1) und gemäß den allgemeinen Angaben zur Herstellung erhält man folgende Verbindungen der Formel (I-1-a)

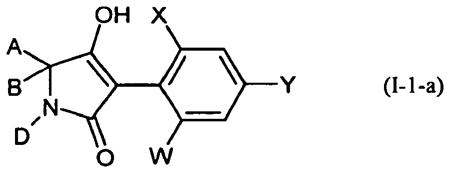

| **Bsp.-Nr.** | **W** | **X** | **Y** | **D** | **A** | **B** | **Fp.°C** | **Isomer** |
|---|---|---|---|---|---|---|---|---|
| I-1-a-2 | OCH₃ | Cl | CH₃ | H | CH₃ | CH₃ | 191 | - |
| I-1-a-3 | OCH₃ | Cl | CH₃ | H | -(CH₂)₂-CHOCH₃-(CH₂)₂- | | 219 | ß |
| I-1-a-4 | OC₃H₇ | Cl | CH₃ | H | -(CH₂)₂-CHOCH₃-(CH₂)₂- | | ** | ß |
| I-1-a-5 | OC₂H₅ | Cl | CH₃ | H | -(CH₂)₂-CHOCH₃-(CH₂)₂- | | 132 | ß |
| I-1-a-6 | O-(CH₂)₂O-CH₃ | Cl | CH₃ | H | -(CH₂)₂-CHOCH₃-(CH₂)₂- | | 130 | ß |
| I-1-a-7 | O-CH₂◁ | Cl | CH₃ | H | -(CH₂)₂-CHOCH₃-(CH₂)₂- | | 148 | ß |
| I-1-a-8 | OCH₃ | Cl | CH₃ | H | C₂H₅ | CH₃ | *0.77 (m, 3H, CH₂CH₃) 2.30 (s, 3H, Ar-CH₃) | |
| I-1-a-9 | OC₂H₅ | Cl | CH₃ | H | 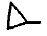 | CH₃ | 195 | |
| I-1-a-10 | OC₂H₅ | Cl | CH₃ | H | C₂H₅ | CH₃ | *0.78 (dt, 3H, CH₂-CH₃) 2.29 (s, 3H, Ar-CH₃) | |
| I-1-a-11 | OC₂H₅ | Cl | CH₃ | C₂H₅ | CH₃ | H | 107 | |
| I-1-a-12 | OCH₃ | Cl | CH₃ | H | i-C₃H₇ | CH₃ | 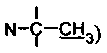 * 1.30 (s, 3H, 1.85-1.95 (m, 1H, -CH-(CH₃)₂) | |
| I-1-a-13 | OCH₃ | Cl | CH₃ | H | i-C₄H₉ | CH₃ | *0.85-0.95 (m, 6H, CH₂-(CH₃)₂) 1.60-1.70 (m, 2H, CH₂-(CH₃)₂) | |
| I-1-a-14 | OCH₃ | Cl | CH₃ | C₂H₅ | CH₃ | H | *1.30 (d, 3H, CH₂CH₃) 2.30 (s, 3H, Ar-CH₃) | |
| I-1-a-15 | OCH3 | Cl | CH₃ | H | 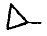 | CH₃ | *1.11 (m, 1H, cyclo-propyl-CH) 2.30 (s, 3H, Ar-CH₃) | |

| | | | | | | | | |
|---|---|---|---|---|---|---|---|---|
| ** ¹H-NMR (400 MHz, d₆-DMSO): δ = 2,29 (s, 3H, ArCH₃), 3,83 (m, 2H, O-CH₂) ppm * ¹H-NMR (400 MHz, d₆-DMSO): Verschiebungen δ in ppm | | | | | | | | |

### Beispiel Nr. I-1-b-1

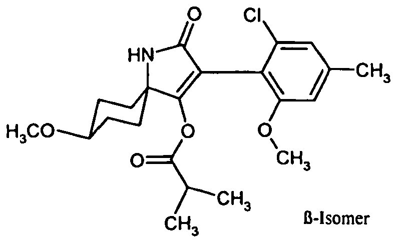

In einem 100 ml Dreihalskolben mit Thermometer und Rückflusskühler werden unter Argon 0,7 g der Verbindung gemäß Beispiel I-1-a-3 in 30 ml wasserfreiem Essigsäureethylester und 0,2 g Triethylamin (2 mMol) = 0,28 ml vorgelegt, mit 10 mg 4-N,N-Dimethylamino-pyridin katalysiert und unter Rückfluss mit 0,22 g (0,002 Mol) Isobuttersäurechlorid in 2 ml wasserfreiem Essigsäureethylester versetzt. Man rührt 1 Stunde; Reaktionsverfolgung durch Dünnschichtchromatographie. Es erfolgt säulenchromatographische Reinigung an Kieselgel, (Hexan: Essigsäureethylester = 8:2)
Ausbeute: 0,8 g (81 % der Theorie), Fp.: 180°C

In Analogie zu Beispiel (I-1-b-1) und gemäß den allgemeinen Angaben zur Herstellung erhält man folgende Verbindungen der Formel (I-1-b)

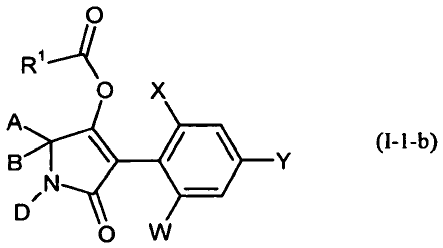

| **Bsp.-Nr.** | **W** | **X** | **Y** | **D** | **A** | **B** | **R¹** | **Fp.°C** | **Isomer** |
|---|---|---|---|---|---|---|---|---|---|
| I-1-b-2 | OCH₃ | Cl | CH₃ | H | CH₃ | CH₃ | H₃CO-CH₂- | 191 | - |
| I-1-b-3 | OCH₃ | Cl | CH₃ | H | i-C₄H₉ | CH₃ | t-C₄H₉ | 154 | - |
| I-1-b-4 | OCH₃ | Cl | CH₃ | H | i-C₄H₉ | CH₃ | H₃CO-CH₂- | ** | - |
| I-1-b-5 | OCH₃ | Cl | CH₃ | H | -(CH₂)₂-O-(CH₂)₂- | | 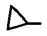 | 216-218 | - |
| I-1-b-6 | OCH₃ | Cl | CH₃ | H | -(CH₂)₂-O-(CH₂)₂- | | H₃CO-CH₂- | *2.30 (3H, s, Ar-CH₃) 4.11 (s, 2H, CH₂-O-CH₃) | - |
| I-1-b-7 | OCH₃ | Cl | CH₃ | H | -(CH₂)₂-CHOCH₃-(CH₂)₂- | | 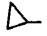 | 156-159 | ß |
| I-1-b-8 | OCH₃ | Cl | CH₃ | H | -(CH₂)₂-O-(CH₂)₂- | | ClCH₂- | 188-207 | - |
| I-1-b-9 | OCH₃ | Cl | CH₃ | H | -(CH₂)₂-CHOCH₃-(CH₂)₂- | | H₃CO-CH₂- | 193-195 | - |
| I-1-b-10 | OCH₃ | Cl | CH₃ | H | -(CH₂)₂-O-(CH₂)₂- | | 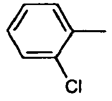 | 245-247 | - |

| | | | | | | | | | |
|---|---|---|---|---|---|---|---|---|---|
| * ¹H-NMR (300 MHz, CDCl₃): Verschiebungen δ in ppm ** * 3.30 (s, 3H, CH₂-O-CH₃) 4.10 (s, 2H, CH₂-O-CH₃) | | | | | | | | | |

### Beispiel Nr. I-1-c-1

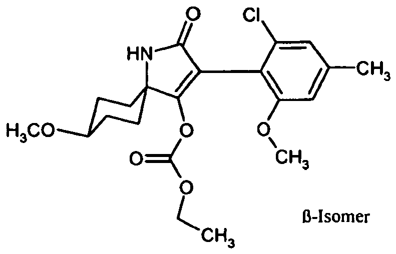

In einem 100 ml Dreihalskolben mit Thermometer und Rückflusskühler werden unter Argon 0,7 g der Verbindung gemäß Beispiel I-1-a-3 in 30 ml wasserfreiem Dichlormethan und 0,2 g Triethylamin (2 mMol) = 0,28 ml vorgelegt und bei 20°C mit 0,22 g (0,002 Mol) Chlorameisensäureethylester in 2 ml wasserfreiem Dichlormethan versetzt. Man rührt 1 Stunde; Reaktionsverfolgung durch Dünnschichtchromatographie. Es erfolgt säulenchromatographische Reinigung an Kieselgel, (Hexan: Essigsäureethylester = 8 : 2).
Ausbeute: 0,8 g (94 % der Theorie), Fp.: 201°C

In Analogie zu Beispiel (I-1-c-1) und gemäß den allgemeinen Angaben zur Herstellung erhält man folgende Verbindungen der Formel (I-1-c)

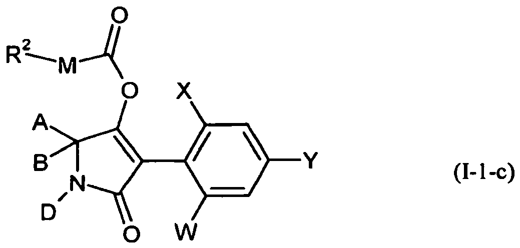

| **Bsp.-Nr.** | **W** | **X** | **Y** | **D** | **A** | **B** | **M** | **R²** | **Fp.°C** | **Isomer** |
|---|---|---|---|---|---|---|---|---|---|---|
| I-1-c-2 | OCH₃ | Cl | CH₃ | H | CH₃ | CH₃ | O | C₂H₅ | * | - |
| I-1-c-3 | OCH3 | Cl | CH₃ | H | i-C₃H₇ | CH₃ | O | C₂H₅ | +¹ | - |
| I-1-c-4 | OCH₃ | Cl | CH₃ | H | i-C₄H₉ | CH₃ | O | C₂H₅ | +² | - |
| I-1-c-5 | OC₃H₇ | Cl | CH₃ | H | -(CH₂)₂-CHOCH₃-(CH₂)₂- | | O | C₂H₅ | 195 | ß |
| I-1-c-6 | OCH₃ | Cl | CH₃ | H | -(CH₂)₂CHOCH₃-(CH₂)₂- | | O | C₆H₅-CH₂- | 178 | ß |
| I-1-c-7 | OCH₃ | Cl | CH₃ | H | -(CH₂)₂-O-(CH₂)₂- | | O | CH₂=CH-CH₂- | 213 | - |
| I-1-c-8 | OCH₃ | Cl | CH₃ | H | -(CH₂)₂-O-(CH₂)₂- | | O | C₆H₅-CH₂- | 188-190 | - |
| I-1-c-9 | OCH₃ | Cl | CH₃ | C₂H₅ | CH₃ | H | O | C₂H₅ | +³ | - |
| I-1-c-10 | OCH₃ | Cl | CH₃ | H | C₂H₅ | CH₃ | O | C₂H₅ | +⁴ | - |

| | | | | | | | | | | |
|---|---|---|---|---|---|---|---|---|---|---|
| *¹H-NMR (400 MHz, CDCl₃): δ = 2,31 (s, 3H, Aryl-CH₃), 4,05 (q, 2H, CH₂O) in ppm **¹H-NMR (300 MHz, CDCl₃): Verschiebungen δ in ppm +^{1**} 1.15 (tr, 3H, O-CH₂-CH₃) +^{3**} 4.21 (ddq, 2H, CH₂-O) 4.05 (m, 2H, O-CH₂-CH₃) 2.29 (s, 3H, Ar-CH₃) +^{2**} 0.90 (d, 6H, CH(CH₃)₂) +^{4**} 2.31 (s, 3H, Ar-CH₃) 3.75 (s, 3H, Ar-O-CH₃) 4.05 (dq, 2H, CH₂-O) | | | | | | | | | | |

### Beispiel Nr. I-1-d-1

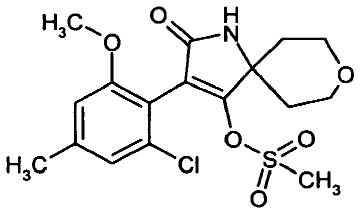

0,194 g (0,6 mmol) der Verbindung gemäß Bsp. I-1-a-1 wird in 10 ml Dichlormethan aufgenommen und mit 0,1 ml Triethylamin versetzt. Nach 5 Min. wird 0,05 ml Methansulfonsäurechlorid zugegeben und 24 Stunden bei Raumtemperatur gerührt. Man versetzt mit 5 %iger Natriumhydrogencarbonatlösung und rührt 30 Min. nach. Die organische Phase wird abgetrennt und mit Natriumsulfat getrocknet. Nach Einengen zur Trockne wird der Rückstand in 2 ml Essigsäureethylester aufgenommen und abgesaugt. Man wäscht noch zweimal mit je 0,5 ml Essigsäureethylester, um das gewünschte Produkt zu erhalten.
Ausbeute: 0,105 g (44 % d. Theorie), Fp. 221-224°C

### Beispiel Nr. I-1-f-1

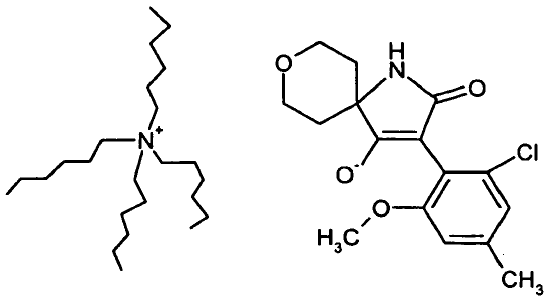

0,162 g (0,5 mmol) der Verbindung gemäß Bsp. (I-1-a-1) wird in 8 ml Methanol aufgenommen und zu dieser Lösung bei Raumtemperatur 0,48 ml Tetrahexylammoniumhydroxyd gegeben. Man rührt 4 Stunden bei Raumtemperatur und engt anschließend ein. Der erhaltene glasartige Rückstand wird noch viermal mit Methanol ausgekreist, anschließend mit Dichlormethan aufgenommen und mit Natriumsulfat getrocknet. Nach Entfernen des Dichlormethans im Vakuum erhält man das gewünschte Produkt als glasartige Substanz.
Ausbeute: 0.31 g (91 % d. Theorie)
- ¹H-NMR (300 MHz, CDCl₃): δ =: 3.01 (t breit, 8H, N-CH₂), 3.72 (s, 3H, OCH₃)

### Beispiel Nr. II-1

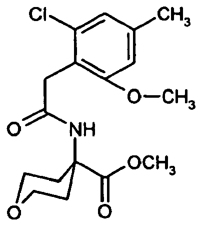

In einem 100 ml Dreihalskolben mit Thermometer und Rückflusskühler werden unter Argon 3,24 g (16,5 mmol) 4-Amino-tetrahydropyran-4-carbonsäuremethylester-hydrochlorid in 40 ml wasserfreiem Tetrahydrofuran und 4,7 ml (33 mMol) Triethylamin vorgelegt. Man rührt 5 min gibt 3,2 g 2-Chlor-6-methoxy-4-methyl-phenylessigsäure (15 mMol) hinzu und rührt weitere 15 min. Dann gibt man 3,3 ml Triethylamin zu und tropft sofort 0,9 ml Phosphoroxychlorid so zu, dass die Lösung mäßig siedet. Man rührt 30 min unter Rückfluss. Das Lösungsmittel wird abdestilliert und es erfolgt säulenchromatographische Reinigung an Kieselgel, (Dichlormethan / Essigsäureethylester = 3 : 1).
Ausbeute: 3,6 g (59 % der Theorie), Fp.: 160°C

### Beispiel II-8

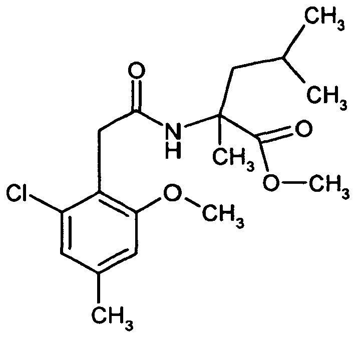

Zu 4.6 ml konzentrierter Schwefelsäure tropft man bei 30-40 °C 4.8 g der Verbindung gemäß Beispiel XXIX-1 und rührt 2 Stunden nach. Nach Zutropfen von 10.84 ml Methanol wird 5 Stunden bei 40 - 70°C Außentemperatur nachgerührt und über Nacht stehen gelassen. Reaktionslösung auf Eis/H₂O geben, mit Dichlormethan extrahieren, mit gesättigter Natriumhydrogencarbonatlösung waschen, trocknen und einrotieren.
Ausbeute: 3,93 g (67 % d. Theorie)
- ¹H-NMR (400 MHz, CDCl₃): δ =: 0.68, 0.80 (2d, 6H, CH₂CH₃)₂), 1.60 (dd, 2H, CH₂-(CH₃)₂), 3.70 (s, 2H, CH₂-CO), 3.85 (s, 3H, CO₂CH₃) ppm.

In Analogie zu Beispiel (II-1) und gemäß den allgemeinen Angaben zur Herstellung erhält man folgende Verbindungen der Formel (II)

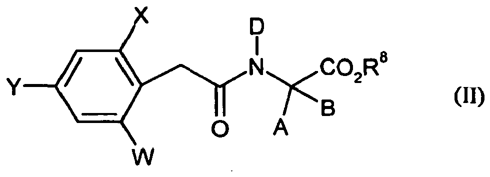

| **Bsp.-Nr.** | **W** | **X** | **Y** | **D** | **A** | **B** | **R⁸** | **Fp.°C** | **Isomer** |
|---|---|---|---|---|---|---|---|---|---|
| 11-2 | OCH₃ | Cl | CH₃ | H | CH₃ | CH₃ | CH₃ | 126 | - |
| II-3 | OCH₃ | Cl | CH₃ | H | -(CH₂)₂-CHOCH₃-(CH₂)₂- | | CH₃ | 149 | ß |
| II-4 | OC₃H₇ | Cl | CH₃ | H | -(CH₂)₂-CHOCH₃-(CH₂)₂- | | CH₃ | 128 | ß |
| II-5 | OC₂H₅ | Cl | CH₃ | H | -(CH₂)₂-CHOCH₃-(CH₂)₂- | | CH₃ | 113 | ß |
| II-6 | 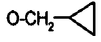 | Cl | CH₃ | H | -(CH₂)₂-CHOCH₃-(CH₂)₂- | | CH₃ | 147 | ß |
| II-7 | O-(CH₂)-OCH₃ | Cl | CH₃ | H | -(CH₂)₂-CHOCH₃-(CH₂)₂- | | CH₃ | 112 | ß |
| II-8 | OCH₃ | Cl | CH₃ | H | -CH₂-i-C₃H₇ | CH₃ | CH₃ | Öl | - |
| II-9 | OCH₃ | Cl | CH₃ | H | i-C₃H₇ | CH₃ | CH₃ | *0,78, 0.85 (2d, 6H, CH(CH₃)₂), 3.85 (s, 3H, CO₂ CH₃) | |
| II-10 | OCH₃ | Cl | CH₃ | H | 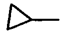 | CH₃ | CH₃ | 95 | - |
| II-11 | OCH₃ | Cl | CH₃ | C₂H₅ | CH₃ | H | C₂H₅ | *2.29, (s, 3H, Ar-CH₃), 4.11 (q, 2H, O-CH₂) | |
| II-12 | OCH₃ | Cl | CH₃ | H | C₂H₅ | CH₃ | CH₃ | 106-108 - | |
| II-13 | OC₂H₅ | Cl | CH₃ | C₂H₅ | CH₃ | H | C₂H₅ | *1.46, (d, 3H, CHCH₃), 2.29 (s, 3H, Ar-CH₃) | |
| II-14 | OC₂H₅ | Cl | CH₃ | H | 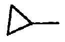 | CH₃ | CH₃ | 123-125 | - |
| II-15 | OC₂H₅ | Cl | CH₃ | H | C₂H₅ | CH₃ | CH₃ | 85-87 | - |

| | | | | | | | | | |
|---|---|---|---|---|---|---|---|---|---|
| *¹H-NMR (300 MHz, CDCl₃): Verschiebungen δ in ppm | | | | | | | | | |

### Beispiel Nr. XXIX-1

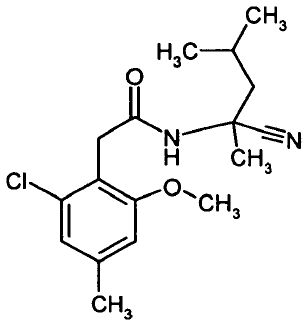

3,2 g der Verbindung gemäß Beispiel XXVII-1 und 5,44 ml Thionylchlorid werden auf 80°C erwärmt und gerührt bis die Gasentwicklung beendet ist. Der Ansatz wird mit Toluol einrotiert, um überschüssiges Thionylchlorid zu entfernen, nochmals mit Tetrahydrofuran einrotiert und der Rückstand in 10 mL Tetrahydrofuran gelöst = Lsg. 1. Zu 1,88 g 1-Amino-1,4-dimethylpentancarbonsäurenitril in 20 ml Tetrahydrofuran und 2,48 ml Triethylamin werden bei 0 - 10°C Lsg. 1 zugetropft, bei Raumtemperatur für ~ 3 Stunden nachgerührt, die Reaktionslösung einrotiert, der Rückstand in Dichlormethan gelöst, mit 0,5 M HCl gewaschen, getrocknet und einrotiert. Ausbeute: 4,86 g (96 % der Theorie), Fp. 131°C

In Analogie zu Beispiel (XXIX-1) und gemäß den allgemeinen Angaben zur Herstellung erhält man folgende Verbindungen der Formel (XXIX)

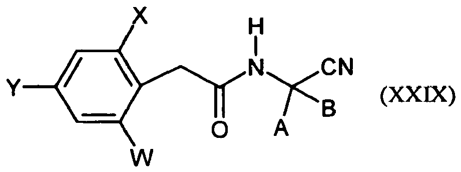

| **Bsp.-Nr** | **W** | **X** | **Y** | **A** | **B** | **Fp.°C** |
|---|---|---|---|---|---|---|
| XXIX-2 | OCH₃ | Cl | CH₃ | i-C₃H₇ | CH₃ | * 0.93, 0.98 (2d, 6H, CH(CH₃)₂) 3.85 (s, 3H, Ar-OCH₃) |
| XXIX-3 | OCH₃ | Cl | CH₃ | 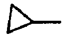 | CH₃ | 132 |
| XXIX-4 | OCH₃ | Cl | CH₃ | C₂H₅ | CH₃ | 134-136 |
| XXIX-5 | OC₂H₅ | Cl | CH₃ | 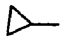 | CH₃ | * 1.61, (s, 3H, CH₃) 2.33 (s, 3H, Ar-CH₃) |
| XXIX-6 | OC₂H₅ | Cl | CH₃ | C₂H₅ | CH₃ | * 1.73, (s, 3H, CH₃) 2.32 (s, 3H, Ar-CH₃) |

| | | | | | | |
|---|---|---|---|---|---|---|
| *¹-NMR (300 MHz, CDCl₃): Verschiebungen δ in ppm | | | | | | |

### Beispiel Nr. I-2-a-1

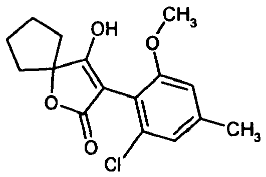

Zu 1,85 g (16 mMol) Kalium tert.-butylat in 10 ml Dimethylformamid tropft man bei 0-10°C 3,9 g (11 mMol) der Verbindung gemäß Beispiel III-1 in 10 ml Dimethylformamid gelöst. Man rührt 15 Stunden bei Raumtemperatur. Das Lösungsmittel wird abdestilliert, der Rückstand in Wasser verrührt und mit HCl-Lösung angesäuert, der Niederschlag abgesaugt und getrocknet.
Ausbeute: 2,9 g (77 % der Theorie), Fp.: 198°C

In Analogie zu Beispiel (I-2-a-1) und gemäß den allgemeinen Angaben zur Herstellung erhält man folgende Verbindungen der Formel (I-2-a)

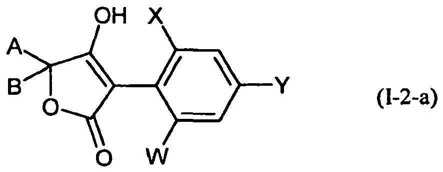

| **Bsp.-Nr.** | **W** | **X** | **Y** | **A** | **B** | **Fp.°C** |
|---|---|---|---|---|---|---|
| I-2-a-2 | OCH₃ | Cl | CH₃ | -(CH₂)₂-CHOCH₃-(CH₂)₂- | | 173-175 |
| I-2-a-3 | OCH₃ | Cl | CH₃ | -(CH₂)₂-O-(CH₂)₂- | | 216 |

### Beispiel Nr. I-2-b-1

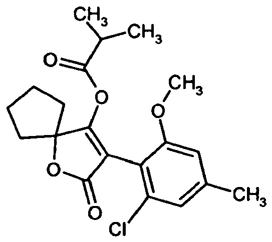

Zu 0,61 g (2 mMol) der Verbindung gemäß Beispiel I-2-a-1 in 10 ml Dichlormethan und 0,31 ml Triethylamin gibt man unter Eiskühlung 0,23 g (2,2 mMol) Isobuttersäurechlorid und rührt über Nacht. Die Lösung wird mit 10 % Citronensäure und 10 % NaOH gewaschen, getrennt, getrocknet und eingeengt.
Ausbeute: 0,85 g (Öl)
¹H-NMR (400 MHz, CD₃CN): δ = 2.35 (s, 3H, Ar-CH₃), 2.70 (m, 1H, CH-(CH₃)₂),
3.75 (s, 3H, OCH₃) ppm.

In Analogie zu Beispiel (I-2-b-1) und gemäß den allgemeinen Angaben zur Herstellung erhält man folgende Verbindungen der Formel (I-2-b)

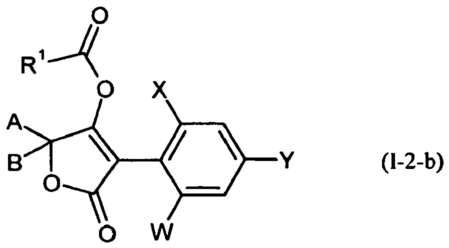

| **Bsp.-Nr.** | **W** | **X** | **Y** | **A** | **B** | **R¹** | **Fp.°C** |
|---|---|---|---|---|---|---|---|
| I-2-b-2 | OCH₃ | Cl | CH₃ | -(CH₂)₂-CHOCH₃-(CH₂)₂- | | i-C₃H₇ | *3.30, 3.34 (2s, 3H, CH-OCH₃) 3.74, 3.75 (2s, 3H, Ar-OCH₃) |
| I-2-b-3 | OCH₃ | Cl | CH₃ | -(CH₂)₂-O-(CH₂)2- | | i-C₃H₇ | *1.07-1.19 (8s, 6H, CH-(CH₃)₂) 3.74, 3.76 (2s, 3H, Ar-OCH₃) |

| | | | | | | | |
|---|---|---|---|---|---|---|---|
| * ¹H-NMR (400 MHz, CD₃CN): Angaben δ in ppm. | | | | | | | |

### Beispiel Nr.I-2-c-1

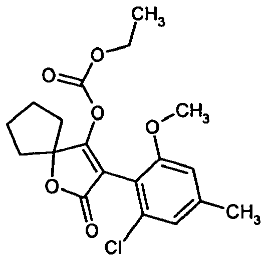

Zu 0,6 g (2 mMol) der Verbindung gemäß Beispiel I-2-a-1 in 10 ml Dichlormethan und 0,31 ml Triethylamin gibt man unter Eiskühlung 0,24 g (2,2 mMol) Chlorameisensäureethylester und rührt bei Raumtemperatur 8 h. Die Lösung wird mit 10 % Citronensäure und 10 % NaOH gewaschen, getrennt, getrocknet und eingeengt.
Ausbeute: 0,7 g (85 % der Theorie)
¹H-NMR (400 MHz, CD₃CN): δ = 1.13 (t, 3H, -O-CH₂CH₃), 4.05 (q, 2H, -O-CH₂CH₃) ppm.

In Analogie zu Beispiel (I-2-c-1) und gemäß den allgemeinen Angaben zur Herstellung erhält man folgende Verbindungen der Formel (I-2-c)

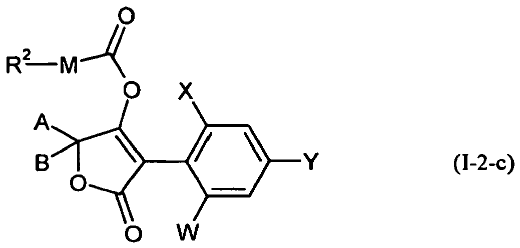

| **Bsp.-Nr.** | **W** | **X** | **Y** | **A** | **M** | **R²** | **Fp.°C** |
|---|---|---|---|---|---|---|---|
| I-2-c-2 | OCH₃ | Cl | CH₃ | -(CH₂)₂-CHOCH₃-(CH₂)₂- | O | C₂H₅ | 1.10-1.15 (6s, 3H, -OCH₂CH₃) 3.31, 3,32 (2s, 3H, CHOCH₃) |
| I-2-c-3 | OCH₃ | Cl | CH₃ | -(CH₂)₂-O-(CH₂)₂- | O | C₂H₅ | 3.91-3.96 (m, 2H, -O-CH₂-CH₂) 4.05-4.07 (m, 2H,-O-CH₂CH₃) |

| | | | | | | | |
|---|---|---|---|---|---|---|---|
| * ¹H-NMR (400 MHz, CD₃CN): Angaben 8 in ppm. | | | | | | | |

### Beispiel Nr. III-1

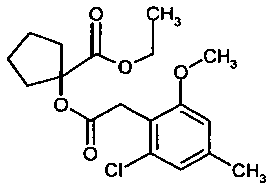

1,7 g (11 mol) 1-Hydroxy-cyclopentancarbonsäure-ethylester und 2,6 g (11 mmol) 2-Chlor-4-methyl-6-methoxy-phenylessigsäurechlorid werden auf 120-140°C erhitzt bis die Gasentwicklung beendet ist und dann an der Ölpumpe kurz entgast.
Das Produkt wird ohne weitere Reinigung zur Herstellung des Beispieles I-2-a-1 verwendet.
Ausbeute: 3,9 g (87 % der Theorie)

In Analogie zu Beispiel (III-I) und gemäß den allgemeinen Angaben zur Herstellung erhält man folgende Verbindungen der Formel (III)

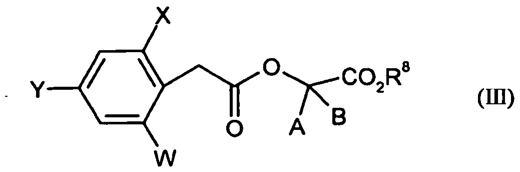

| **Bsp.-Nr.** | **W** | **X** | **Y** | **A** | **B** | **R⁸** |
|---|---|---|---|---|---|---|
| III-2 | OCH₃ | Cl | CH₃ | -(CH₂)₂-CHOCH₃-(CH₂)₂- | | C₂H₅ |
| III-3 | OCH₃ | Cl | CH₃ | -(CH₂)₂-O-(CH₂)₂- | | C₂H₅ |

Die Verbindungen der Formel (III) wurden ohne weitere Aufreinigung zur Herstellung der Beispiele (I-2-a) verwendet.

### Verfahren R

### Beispiel (XXXI-1): 2-Chlor-4-methyl-6-methoxyphenylessigsäuremethylester

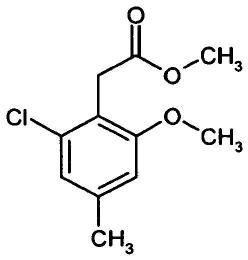

100 g (360 mmol) 2-Brom-6-chlor-4-methylessigsäuremethylester bekannt aus WO 96/35664, 10.3 g (72 mmol) Kupfer(I)bromid und 105 ml (1.08 ml) Essigsäuremethylester werden unter Argonatmosphäre in 345 ml (1.80 mol) einer 30 %igen Natriummethylatlösung vorgelegt und für 12 h refluxiert. Nach dem Abkühlen der Reaktionsmischung wird das Lösungsmittel im Vakuum entfernt, der Rückstand in 200 ml Wasser aufgenommen und 200 ml Dichlormethan versetzt. Die Phasen werden getrennt und die organische noch zweimal mit Wasser und einmal mit gesättigter Kochsalzlösung nachgewaschen. Die vereinigten organischen Extrakte werden über Natriumsulfat getrocknet, anschließend wird das Lösungmittel im Valuum entfernt und der Rückstand chromatographisch aufgereinigt.
Ausbeute: 75 g (91 %)
¹H-NMR {400 MHz, DMSO-d₆}: 2.30 (s, 3H, CH₃); 3.60 (s, 3H OCH₃); 3.70 (s, 2H, CH₂);
3.77 (s, 3H, OCH₃); 6,84 (s 1H, Ph-H); 6.89 (s, 1H, Ph-H).
MS/CI: 229 (M+1).

### Beispiel (XXVII-1): 2-Chlor-4-methyl-6-methoxyphenylessigsäure

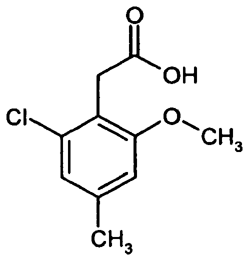

75 g (328 mmol) 2-Chlor-4-methyl-6-methoxyphenylessigsäuremethylester gemäß Bsp. XXXI-1 werden in 750 ml Methanol gelöst und anschließend mit 55,20 g (984 mmol) Kaliumhydroxid in 250 ml Wasser für 12 h auf 80°C erhitzt. Das Methanol wird am Rotationsverdampfer abgezogen, der Rückstand auf pH 3 gestellt und das ausgefallene Produkt abfiltriert und getrocknet.
Ausbeute: 63,2 g (90 %)
¹H-NMR {400 MHz, DMSO-d₆}: 2.30 (s, 3H, CH₃); 3.61 (s, 2H CH₂); 3.77 (s, 3H, OCH₃);
6.80 (s, 1H, Ph-H); 6.88 (s, 1H, Ph-H); 12.2 (s, 1H, CO₂H).
MS/CI: 215 (M+1).

### Beispiel (XXXI-2): 2-Chlor-4-methyl-6-ethoxyphenylessigsäureethylester

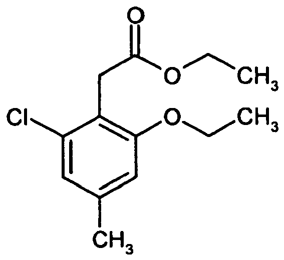

¹H-NMR {400 MHz, DMSO-d₆}: 1.18 (t, ³J_{HH} = 7 Hz, 3H, CH₃); 1.26 (t, ³J_{HH} = 7 Hz , 3H, CH₃); 2.28 (s, 3H, Ph-CH₃); 3.67 (s, 2H, CH₂); 4.02 (d, ³J_{HH} = 7 Hz, 2H, OCH₂); 4.07 (d, ³J_{HH} = 7 Hz, 2H, OCH₂), 6.81 (s, 1H Ph-H); 6.87 (s, 1H, Ph-H).
MS/CI: 257 (M+1).

### Beispiel (XXVII-2): 2-Chlor-4-methyl-6-ethoxyphenylessigsäure

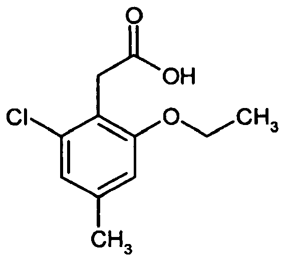

¹H-NMR {400 MHz, DMSO-d₆}: 1.27 (t, ³J_{HH} = 7 Hz, 3H, CH₃); 2.27 (s, 3H, Ph-CH₃); 3.10 (s, 2H, CH₂); 4.02 (d, ³J_{HH} = 7 Hz, 2H, OCH₂); 6.76 (s, 1H, Ph-H), 6.83 (s, 1H Ph-H); 12.3 (s, 1H, CO₂H).
MS/CI: 229 (M+1).

### Beispiel (XXXI-3): 2-Chlor-4-methyl-6-propoxyphenylessigsäurepropylester

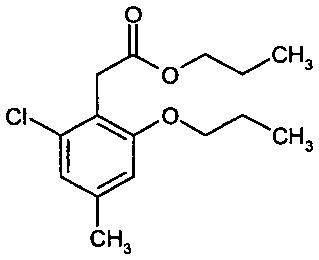

¹H-NMR {400 MHz, DMSO-d₆}: 0.88 (t, ³J_{HH} = 7 Hz, 3H, CH₃); 0.95 (t, ³J_{HH} = 7 Hz , 3H, CH₃); 1.54 (sext, ³J_{HH} = 7 Hz, 2H, CH₂); 1.68 (sext, ³J_{HH} = 7 Hz, 2H, CH₂); 2.28 (s, 3H, Ph-CH₃); 3.69 (s, 2H, CH₂); 3.92 (t, ³J_{HH} = 7 Hz, 2H, OCH₂), 3.97 (t, ³J_{HH} = 7 Hz, 2H, OCH₂); 6.80 (s, 1H, Ph-H); 6.86 (s, 1H, Ph-H).
MS/CI: 285 (M+1).

### Beispiel (XXVII-3): 2-Chlor-4-methyl-6-propoxyphenylessigsäure

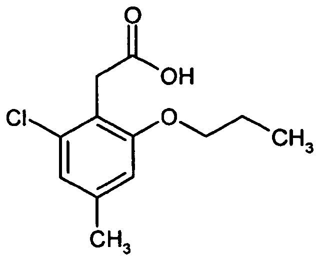

¹H-NMR {400 MHz, DMSO-d₆}: 0.95 (t, ³J_{HH} = 7 Hz, 3H, CH₃); 1.71 (sext, ³J_{HH} = 7 Hz , 2H, CH₂); 2.23 (s, 3H, Ph-CH₃); 3.61 (s, 2H, CH₂); 3.92 (t, ³J_{HH} = 7 Hz, 2H, OCH₂), 6.79 (s, 1H, Ph-H); 6.85 (s, 1H, Ph-H); 12.2 (s, 1H, CO₂H).
MS/CI: 243 (M+1), Fp.: 116°C

### Beispiel (XXXI-4): 2-Chlor-4-methyl-6-methoxyethoxy-phenylessigsäure-methoxyethylester

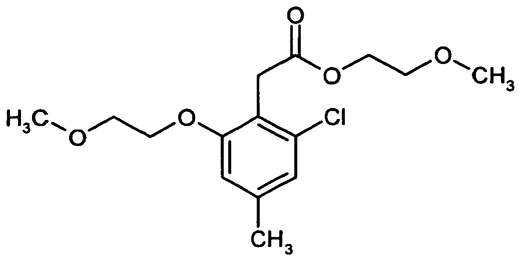

LC-MS: (ESI pos) M = 317 (100)
1H-NMR {400 MHz, DMSO-d₆} : 6.89 (s, 1H); 6.84 (s, 1H); 4.14 (m, 2H); 4.09 (m, 2H); 3.71 (s, 2H); 3.62 (m, 2H); 3.51 (m, 2H); 3.30 (s, 3H); 3.25 (s, 3H); 2.28 (s, 3H)

### Beispiel (XXVII-4): 2-Chlor-4-methyl-6-methoxyethoxy-phenylessigsäure

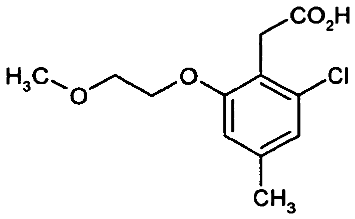

LC-MS: (ESI pos) M = 258 (100)
¹H-NMR {400 MHz, DMSO-d₆}: 12.16 (s, 1H); 6.87 (s, 1H); 6.83 (s, 1H); 4.10 (m, 2H); 3.63 (m, 3H); 3.62 (s, 2H); 3.31 (s, 3H); 2.28 (s, 3H).

### Beispiel (XXXI-5):

2-Chlor-4-methyl-6-cyclopropylmethoxy-phenylessigsäure-cyclopropylmethylester 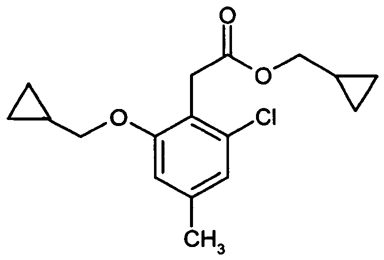 LC-MS: (ESI pos) M = 309 (100)

### Beispiel (XXVII-5): 2-Chlor-4-methyl-6-cyclopropylmethoxy-phenylessigsäure

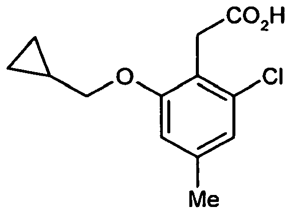

LC-MS: (ESI pos) M = 255 (100)
¹H-NMR {400 MHz, DMSO-d₆}: 12.2 (s, 1H); 6.85 (s, 1H); 6.78 (s, 1H); 3.85 (d.J = 6.7 Hz, 2H); 3.63 (s, 2H); 2.27 (s, 3H); 1.19 (m, 1H); 0.53 (m, 2H); 0.31 (m, 2H).

### Beispiel A

### Aphis gossypii-Test

| | |
|---|---|
| Lösungsmittel: | 7 Gewichtsteile Dimethylformamid |
| Emulgator: | 2 Gewichtsteil Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Baumwollblätter *(Gossypium hirsutum)*, die stark von der Baumwollblattlaus *(Aphis gossypii)* befallen sind, werden durch Tauchen indie Wirkstoffzubereitung der gewünschten Konzentration behandelt.

Nach der gewünschten Zeit wird die Abtötung in % bestimmt. Dabei bedeutet 100 %, dass alle Blattläuse angetötet wurden; 0 % bedeutet, dass keine Blattläuse abgetötet wurden.

Die Wirkstoffe Bsp. I-2-a-1, Bsp. I-2-c-3 und Bsp. I-2-b-3 zeigen mit Wirkstoffkonzentrationen von 100 ppm eine abtötende Wirkung gegen Aphis gossypii von ≥ 80 % nach 6 d.

### Beispiel B

### Meloidogyne-Test

| | |
|---|---|
| Lösungsmittel: | 80 Gewichtsteile Aceton |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Gefäße werden mit Sand, Wirkstofflösung, Meloidogyne incognita-Ei-Larven-Suspension und Salatsamen gefüllt. Die Salatsamen keimen und die Pflänzchen entwickeln sich. An den Wurzeln entwickeln sich die Gallen.

Nach der gewünschten Zeit wird die nematizide Wirkung an Hand der Gallenbildung in % bestimmt. Dabei bedeutet 100 %, dass keine Gallen gefunden wurden; 0 % bedeutet, dass die Zahl der Gallen an den behandelten Pflanzen der der unbehandelten Kontrolle entspricht.

Die Wirkstoffe Bsp. I-1-a-3 und Bsp. I-1-a-1 zeigen mit Wirkstoffkonzentrationen von 20 ppm eine abtötende Wirkung gegen Meloidogyne von 100 % nach 14 d.

### Beispiel C

### Myzus-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Kohlblätter *(Brassica oleracea),* die stark von der Grünen Pfirsichblattlaus *(Myzus persicae)* befallen sind, werden durch Tauchen in die Wirkstoffzubereitung der gewünschten Konzentration behandelt.

Nach der gewünschten Zeit wird die Abtötung in % bestimmt. Dabei bedeutet 100 %, dass alle Blattläuse abgetötet wurden; 0 % bedeutet, dass keine Blattläuse abgetötet wurden.

Die Wirkstoffe Bsp. I-2-a-1, Bsp. I-2-a-2, Bsp. I-2-c-1, Bsp. I-2-b-1, Bsp. I-2-c-2, Bsp. I-2-c-3, Bsp. I-2-b-3 und Bsp. I-1-a-3 zeigen mit Wirkstoffkonzentrationen von 500 g/ha eine abtötende Wirkung gegen Myzus persicae von 100 % nach 5 d.

### Beispiel D

### Phaedon-Test (Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Die Wirkstoffe Bsp. I-2-c-2, Bsp. I-2-b-2, Bsp. I-1-a-3 und Bsp. I-1-a-1 zeigen mit Wirkstoffkonzentrationen von 500 g/ha eine abtötende Wirkung gegen Phaedon cochleariae von ≥ 80 % nach 7 d.

### Beispiel E

### Tetranychus-Test (OP-resistent/Spritzbehandlung)

| | |
|---|---|
| Lösungsmittel: | 78 Gewichtsteile Aceton |
| | 1,5 Gewichtsteile Dimethylformamid |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben (*Phaseolus vulgaris*)*,* die von allen Stadien der Gemeinen Spinnmilbe (*Tetranychus urticae*) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Die Wirkstoffe Bsp. I-2-a-2, Bsp. I-2-b-1, Bsp. I-2-c-2, Bsp. I-2-b-2, Bsp. I-2-c-3, Bsp. I-1-a-2 und Bsp. I-1-a-3 zeigen mit Wirkstoffkonzentrationen von 100 g/ha eine abtötende Wirkung gegen Tetranychus urticae von ≥ 80 % nach 5 d.

### Beispiel F

### 1. Herbizide Wirkung im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten Testverbindungen werden dann als wässrige Suspension mit einer Wasseraufwandmenge von umgerechnet 600 l/ha unter Zusatz von 0,2 % Netzmittel in unterschiedlichen Dosierungen auf die Oberfläche der Abdeckerde appliziert.

Nach der Behandlung werden die Töpfe im Gewächshaus aufgestellt und unter guten Wachstumsbedingungen für die Testpflanzen gehalten. Die visuelle Bonitur der Auflaufschäden an den Versuchspflanzen erfolgt nach einer Versuchszeit von 3 Wochen im Vergleich zu unbehandelten Kontrollen (herbizide Wirkung in Prozent (%): 100 % Wirkung = Pflanzen sind abgestorben, 0 % Wirkung = wie Kontrollpflanzen).

### 2. Herbizide Wirkung im Nachauflauf

Samen von mono- bzw. dikotylen Unkraut- bzw. Kulturpflanzen werden in Holzfaserföpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die als Spritzpulver (WP) formulierten Testverbindungen werden in verschiedenen Dosierungen mit einer Wasseraufwandmenge von umgerechnet 600 1/ha unter Zusatz von 0,2 % Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100 % Wirkung = Pflanzen sind abgestorben, 0 % Wirkung = wie Kontrollpflanzen).

Der Wirkstoff I-1-a-2 zeigt mit Wirkstoffkonzentrationen von 320 g a.i/ha im Vorauflauf gegen Lolium und Setaria eine abtötende Wirkung von ≥ 80 %.

Die Wirkstoffe Bsp. I-2-a-2, Bsp. I-2-c-2, Bsp. I-2-b-2, Bsp. I-2-b-1, Bsp. I-1-a-2, Bsp. I-1-a-3, Bsp. I-1-b-2, Bsp. I-2-a-3, Bsp. I-1-a-1, Bsp. I-1-c-2 zeigen mit Wirkstoffkonzentrationen von 320 g a.i/ha im Nachauflauf gegen Avena sativa, Lolium, Setaria eine abtötende Wirkung von ≥ 70 %.

### Beispiel G

### Herbizidwirkung und Safenerwirkung im Vor- oder Nachauflauf

Samen bzw. Rhizomstücke von mono- und dikotylen Schadpflanzen und von Kulturpflanzen werden in Torftöpfen in sandiger Lehmerde ausgelegt, mit Erde abgedeckt und im Gewächshaus unter guten Wachstumsbedingungen kultiviert. Bei Vorauflaufanwendung werden Safener und Herbizid nach der Aussaat appliziert, die Nachauflaufbehandlung erfolgt etwa zwei bis drei Wochen nach der Aussaat im Dreiblattstadium der Versuchspflanzen . Die als Emulsionskonzentrate formulierten erfindungsgemäßen Herbizid-Safener-Wirkstoffkombinationen sowie in parallelen Versuchen die entsprechend formulierten Einzelwirkstoffe werden in verschiedenen Dosierungen mit einer Wasseraufwandmenge von umgerechnet 300 1/ha im Vorauflauf auf die Bodenoberfläche, im Nachauflauf auf die grünen Pflanzenteile gesprüht. Die Auswertung im Vorauflauf erfolgt nach 2 - 4 Wochen, im Nachauflauf nach 1-3 Wochen. Während der Standzeit werden die Versuchspflanzen im Gewächshaus unter optimalen Wachstumsbedingungen gehalten. Die Wirkung der Präparate wird visuell im Vergleich zu unbehandelten Kontrollen bonitiert.

Gefäßversuche mit Getreide im Gewächshaus.

Safener und Herbizid wurden im Nachauflauf nacheinander auf die Pflanzen aufgebracht (Aufwandmengen siehe Tabellen)

**Tabelle 1'**

| | Aufwandmenge g a.i./ha | Sommergerste beobachtet (%) |
|---|---|---|
| Beispiel I-1-c-1 | 100 | 60 |
| | 50 | 30 |
| | 25 | 10 |
| | | |
| Beispiel I-1-c-1 | 100 + 100 | 50 |
| + Mefenpyr | 50 + 100 | 10 |
| | 25 + 100 | 0 |
| | | |
| Beispiel I-1-c-1 | 100 + 100 | 40 |
| + Isoxadifen | 50 + 100 | 15 |
| | 25 + 100 | 0 |

**Tabelle 2'**

| | Aufwandmenge g a.i./ha | Winterweizen beobachtet (%) |
|---|---|---|
| Beispiel I-1-c-1 | 50 | 30 |
| | 25 | 10 |
| | | |
| Beispiel I-1-c-1 | 50 + 100 | 20 |
| + Cloquintocet | 25 + 100 | 0 |
| | | |
| Beispiel I-1-c-1 | 50 + 100 | 20 |
| + Isoxadifen | 25 + 100 | 0 |

**Tabelle 3'**

| | Aufwandmenge g a.i./ha | Sommerweizen (%) | Winterweizen (%) | Sommergerste (%) |
|---|---|---|---|---|
| Beispiel I-1-a-5 | 100 | 60 | 60 | 20 |
| | 50 | 50 | 30 | 20 |
| | 25 | 20 | 10 | 10 |
| | | | | |
| Beispiel I-1-a-5 | 100 + 100 | 50 | 30 | 10 |
| + Mefenpyr | 50 + 100 | 20 | 10 | 0 |
| | 25 + 100 | 10 | 0 | 0 |

**Tabelle 4'**

| | Aufwandmenge g a.i./ha | Winterweizen (%) | Sommergerste (%) |
|---|---|---|---|
| Beispiel I-1-a-5 | 100 | 60 | 20 |
| | 50 | 30 | 20 |
| | 25 | 10 | 10 |
| | | | |
| Beispiel I-1-a-5 | 100 + 100 | 30 | 20 |
| + Cloquintocet | 50 + 100 | 10 | 0 |
| | 25 + 100 | 0 | 0 |
| | | | |
| Beispiel I-1-a-5 | 50 + 100 | 20 | 10 |
| + Isoxadifen | 25 + 100 | 10 | 0 |

**Tabelle 5'**

| | Aufwandmenge g a.i./ha | Sommergerste (%) |
|---|---|---|
| Beispiel I-1-b-1 | 100 | 55 |
| | 50 | 40 |
| | 25 | 30 |
| | | |
| Beispiel I-1-b-1 | 100 + 100 | 30 |
| + Mefenpyr | 50 + 100 | 10 |
| | 25 + 100 | 0 |
| | | |
| Beispiel I-1-b-1 | 100 + 100 | 40 |
| + Cloquintocet | 50 + 100 | 20 |
| | 25 + 100 | 10 |
| | | |
| Beispiel I-1-b-1 | 100 + 100 | 50 |
| + Isoxadifen | 50 + 100 | 25 |
| | 25 + 100 | 10 |

**Tabelle 6'**

| | Aufwandmenge g a.i./ha | Sommerweizen (%) | Winterweizen (%) | Sommergerste (%) |
|---|---|---|---|---|
| Beispiel I-1-a-4 | 100 | 60 | 50 | 20 |
| | 50 | 45 | 30 | 10 |
| | 25 | 20 | 10 | 0 |
| | | | | |
| Beispiel I-1-a-4 | 100 + 100 | 10 | 10 | 0 |
| + Mefenpyr | 50 + 100 | 0 | 0 | 0 |
| | 25 + 100 | 0 | 0 | 0 |
| | | | | |
| Beispiel I-1-a-4 | 100 + 100 | 10 | 10 | 10 |
| + Cloquintocet | 50 + 100 | 5 | 0 | 0 |
| | 25 + 100 | 0 | 0 | 0 |

Safener und Herbizid im Vorauflauf nacheinander ausgebracht.

**Tabelle 7'**

| | Aufwandmenge g a.i./ha | Mais (%) |
|---|---|---|
| Beispiel I-1-a-4 | 50 | 20 |
| | 25 | 10 |
| | | |
| Beispiel I-1-a-4 | 50 + 100 | 5 |
| + IIe-5 | 25 + 100 | 5 |

### Beispiel H

### Safenerwirkung nach Saatgutbeize

Die Anzahl an Kulturpflanzensamen, die für jede Safeneraufwandmenge benötigt wird, wurde berechnet. Ausgehend vom Gewicht von 100 Samen wurden ausreichend Samen in Glasflaschen mit Schraubverschluß und einem Volumen, das in etwa dem doppelten Volumen der Samen entsprach, eingewogen.

Die potentiellen Safener wurden als Spritzpulver oder wasserdispergierbare Granulate formuliert. Diese Formulierungen wurden so eingewogen, daß die erforderlichen Aufwandmengen (g Wirkstoff/kg Samen) erzielt wurden. Die Proben wurden zu dem Saatgut in den Flaschen gegeben, und anschließend wurde so viel Wasser zugesetzt, daß man eine Beizflüssigkeit erhielt. Die Flaschen wurden verschlossen und dann in einen Überkopfschüttler (eingestellt auf mittlere Geschwindigkeit über eine Zeitdauer von bis zu einer Stunde) gespannt, so daß die Saatkörner gleichmäßig mit der Beizflüssigkeit überzogen wurden. Die Flaschen wurden geöffnet, und das Saatgut wurde in den Vorauflaufversuchen verwendet.

Vorauflaufapplikation von Herbiziden
Die mit Safener behandelten Samen und unbehandelte Samen als Kontrollen wurden in runden Töpfen mit einem Durchmesser von 7 bis 13 cm in einen sandigen Lehmboden gesät und mit einer ca. 0,5 bis 1 cm dicken Schicht einer 1:1 Mischung von sandigem Lehmboden und Sand bedeckt. Die Herbizide in flüssigen (z.B. Emulsionskonzentrate) oder trockenen (z.B. Spritzpulver) Formulierungen wurden mit deionisiertem Wasser auf die erforderlichen Konzentrationen verdünnt und mit einem auf die Ausbringung von 300 Litern Sprühlösung pro Hektar eingestellten Spritzschlitten auf die Bodenoberfläche aufgebracht.

Die Töpfe wurden in einem Gewächshaus unter günstigen Wachstumsbedingungen aufgestellt, und 3 bis 4 Wochen nach der Herbizidapplikation erfolgte eine optische Auswertung der herbiziden Wirkung. Die Auswertung erfolgte auf einer prozentualen Basis im Vergleich zu unbehandelten Kontrollpflanzen (0% = keine Schädigung, 100% = vollständig abgetötet).

Anwendung des Herbizids im Vorauflauf, Safener gebeizt:

**Tabelle 8'**

| | Aufwandmenge g a.i./ha | Mais (%) |
|---|---|---|
| Beispiel I-1-c-1 | 25 | 35 |
| Beispiel I-1-c-1 | 25 | 15 |
| + IIe-5 | 0,5 g/kg Saatgut | |

**Tabelle 9'**

| | Aufwandmenge g a.i./ha | Mais (%) |
|---|---|---|
| Beispiel I-1-a-5 | 50 | 20 |
| | 25 | 20 |
| | | |
| Beispiel I-1-a-5 | 50 + 0,5 g/kg Saatgut | 10 |
| + IIe-5 | 25 + 0,5 g/kg Saatgut | 5 |

**Tabelle 10'**

| | Aufwandmenge g a.i./ha | Mais (%) |
|---|---|---|
| Beispiel I-1-a-4 | 50 | 20 |
| | 25 | 10 |
| | | |
| Beispiel I-1-a-4 | 50 + 0,5 g/kg Saatgut | 15 |
| + IIe-5 | 25 + 0,5 g/kg Saatgut | 0 |

### Beispiel I

### Grenzkonzentrations-Test / Bodeninsekten-Behandlung transgener Pflanzen

| | |
|---|---|
| Testinsekt: | Diabrotica balteata - Larven im Boden |
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird auf den Boden gegossen. Dabei spielt die Konzentration des Wirkstoffs in der Zubereitung praktisch keine Rolle, entscheidend ist allein die Wirkstoffgewichtsmenge pro Volumeneinheit Boden, welche in ppm (mg/l) angegeben wird. Man füllt den Boden in 0,25 l Töpfe und lässt diese bei 20°C stehen.

Sofort nach dem Ansatz werden je Topf 5 vorgekeimte Maiskörner der Sorte YIELD GUARD (Warenzeichen von Monsanto Domp., USA) gelegt. Nach 2 Tagen werden in den behandelten Boden die entsprechenden Testinsekten gesetzt. Nach weiteren 7 Tagen wird der Wirkungsgrad des Wirkstoffs durch Auszählen der aufgelaufenen Maispflanzen bestimmt (1 Pflanze = 20 % Wirkung).

### Beispiel J

### Heliothis virescens - Test - Behandlung transgener Pflanzen

| | |
|---|---|
| Lösungsmittel: | 7 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) der Sorte Roundup Ready (Warenzeichen der Monsanto Comp. USA) werden durch Tauchen in die Wirkstoffzubereitung der gewünschten Konzentration behandelt und mit der Tabakknospenraupe Heliothis virescens besetzt, solange die Blätter noch feucht sind.

Nach der gewünschten Zeit wird die Abtötung der Insekten bestimmt.

## Patentansprüche

1. Verbindungen der Formel (I) 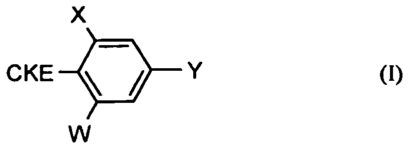 in welcher
W für Methoxy, Ethoxy, n-Propoxy, Methoxy-ethoxy oder Cyclopropyl-methoxy steht,
X für Chlor steht,
Y für Methyl steht,
CKE für eine der Gruppen 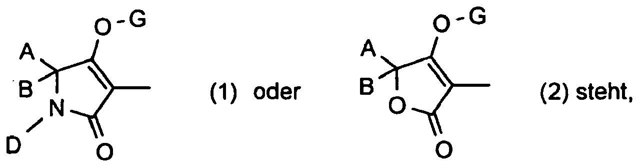
A für Methyl, i-Propyl, i-Butyl oder Cyclopropyl steht,
B für Wasserstoff, Methyl oder Ethyl steht,
A, B und das Kohlenstoffatom an das sie gebunden sind für gesättigtes C₅-C₆-Cycloalkyl stehen, in welchem gegebenenfalls ein Ringatom durch Sauerstoff ersetzt ist und welches gegebenenfalls einfach durch Methyl oder Methoxy substituiert ist,
D für Wasserstoff, Methyl oder Ethyl steht,
G für Wasserstoff (a) oder für eine der Gruppen 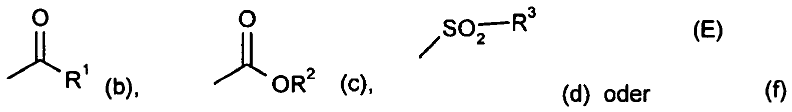
E für ein Ammoniumion steht,
R¹ für ₁-C₆-Alkyl, C₁-C₂-Alkoxy-C₁-alkyl, C₃-C₆-Cycloalkyl, einfach durch Chlor substituiertes C₁-C₄-Alkyl oder für gegebenenfalls einfach durch Chlor substituiertes Phenyl steht,
R² für C₁-C₈-Alkyl, C₃-C₆-Alkenyl oder Benzyl steht,
R³ für C₁-C₆-Alkyl steht.

2. Verfahren zur Herstellung von Verbindungen der Formel (I) gemäß Anspruch 1, **dadurch gekennzeichnet, dass** man zum Erhalt von
(A) Verbindungen der Formel (I-1-a) 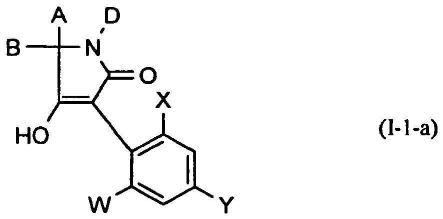 in welcher
A, B, D, W, X und Y die oben angegebenen Bedeutungen haben, Verbindungen der Formel (II) 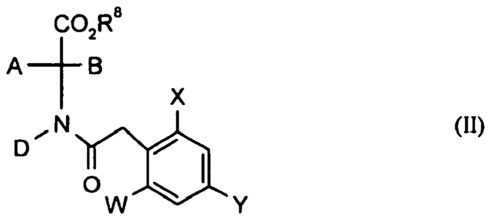 in welcher
A, B, D, W, X und Y die oben angegebenen Bedeutungen haben, und
R⁸ für Alkyl steht,
in Gegenwart eines Verdünnungsmittels und in Gegenwart einer Base intramolekular kondensiert,
(B) Verbindungen der Formel (I-2-a) 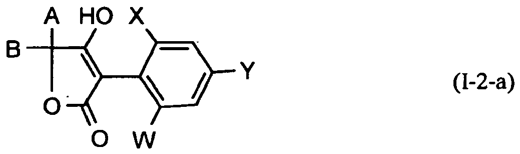 in welcher
A, B, W, X und Y die oben angegebenen Bedeutungen haben, Verbindungen der Formel (III) 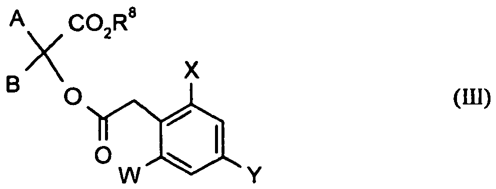 in welcher
A, B, W, X , Y und R⁸ die oben angegebenen Bedeutungen haben,
in Gegenwart eines Verdünnungsmittels und in Gegenwart einer Base intramolekular kondensiert,
(I) Verbindungen der oben gezeigten Formeln (I-1-b) bis (1-2-b), in welchen A, B, D, R¹, W, X, und Y die oben angegebenen Bedeutungen haben, Verbindungen der oben gezeigten Formeln (I-1-a) bis I-2-a), in welchen A, B, D, W, X und Y die oben angegebenen Bedeutungen haben, jeweils
(α) mit Säurehalogeniden der Formel (XIII) 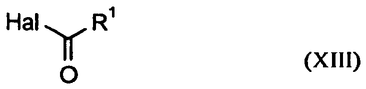
in welcher
R¹ die oben angegebene Bedeutung hat und
Hal für Halogen steht
oder
(ß) mit Carbonsäureanhydriden der Formel (XIV)
R¹-CO-O-CO-R¹ (XIV) in welcher
R¹ die oben angegebene Bedeutung hat,
gegebenenfalls in Gegenwart eines Verdünnungsmittels und gegebenenfalls in Gegenwart eines Säurebindemittels umsetzt;
(J) Verbindungen der oben gezeigten Formeln (I-1-c) bis (I-2-c), in welchen A, B, D, R², M, W, X und Y die oben angegebenen Bedeutungen haben und L für Sauerstoff steht, Verbindungen der oben gezeigten Formeln (I-1-a) bis (I-2-a), in welchen A, B, D, W, X und Y die oben angegebenen Bedeutungen haben, jeweils
mit Chlorameisensäureestern oder Chlorameisensäurethioestern der Formel (XV)
R²-M-CO-Cl (XV)
in welcher
R² und M die oben angegebenen Bedeutungen haben,
gegebenenfalls in Gegenwart eines Verdünnungsmittels und gegebenenfalls in Gegenwart eines Säurebindemittels umsetzt;
(L) Verbindungen der oben gezeigten Formeln (I-1-d) bis (1-2-d), in welchen A, B, D, R³, W, X und Y die oben angegebenen Bedeutungen haben, Verbindungen der oben gezeigten Formeln (I-1-a) bis (I-2-a), in welchen A, B, D, W, X und Y die oben angegebenen Bedeutungen haben, jeweils
mit Sulfonsäurechloriden der Formel (XVII)
R³-SO₂-Cl (XVII)
in welcher
R³ die oben angegebene Bedeutung hat,
gegebenenfalls in Gegenwart eines Verdünnungsmittels und gegebenenfalls in Gegenwart eines Säurebindemittels umsetzt,
(N) Verbindungen der oben gezeigten Formeln (I-1-f) bis (I-2-f), in welchen A, B, D, E, W, X und Y die oben angegebenen Bedeutungen haben, Verbindungen der Formeln (I-1-a) bis (I-2-a), in welchen A, B, D, W, X und Y die oben angegebenen Bedeutungen haben, jeweils mit Metallverbindungen oder Aminen der Formeln (XIX) oder (XX)
Me(OR¹⁰)ₜ (XIX) 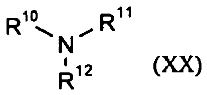
in welchen
Me für ein ein- oder zweiwertiges Metall,
t für die Zahl 1 oder 2 und
R¹⁰, R¹¹, R¹² unabhängig voneinander für Wasserstoff oder Alkyl stehen,
gegebenenfalls in Gegenwart eines Verdünnungsmittels umsetzt,
(P) Verbindungen der oben gezeigten Formeln (I-1-a) bis (I-2-a), in welchen A, B, D, W, X und Y die oben angegebene Bedeutung haben, Verbindungen der Formeln (I-1-a') bis (I-2-a'), in welchen A, B, D, X und Y die oben genannte Bedeutung haben und W' für Brom steht 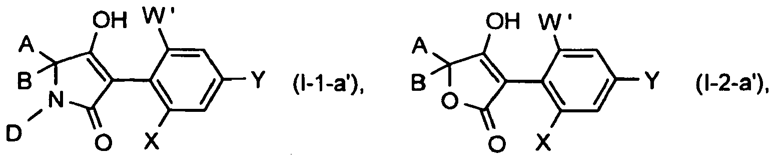 mit Alkoholen der Formel
W-OH
in welcher
W die oben angegebene Bedeutung hat, gegebenenfalls in Gegenwart eines Verdünnungsmittel, eines Cu-I-Salzes und einer starken Base umsetzt.

3. Verbindungen der Formel (II) 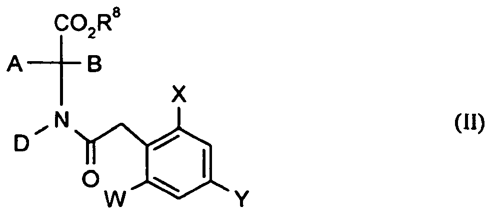 in welcher
R⁸, A, B, D, W, X und Y die oben angegebenen Bedeutungen haben.

4. Verbindungen der Formel (III) 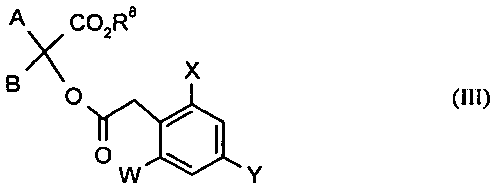 in welcher
R⁸, A, B, W, X und Y die oben angegebenen Bedeutungen haben.

5. Verbindungen der Formel (XXV) 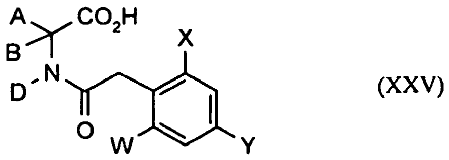 in welcher
A, B, D, W, X und Y die oben angegebenen Bedeutungen haben.

6. Verbindungen der Formel (XXIV) 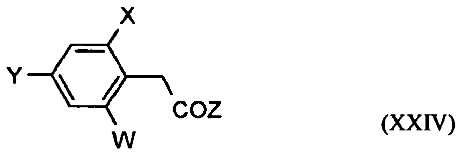 in welcher
W, X, Y und Z die oben angegebenen Bedeutungen haben.

7. Verbindungen der Formel (XXVII) 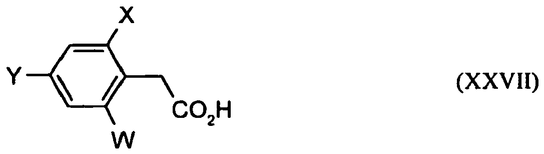 in welcher
W, X und Y die oben angegebene Bedeutung haben.

8. Verbindungen der Formel (XXIX) 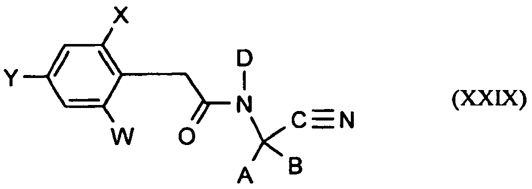 in welcher
A, B, D, W, X und Y die oben angegebenen Bedeutungen haben.

9. Verbindungen der Formel (XXXI) 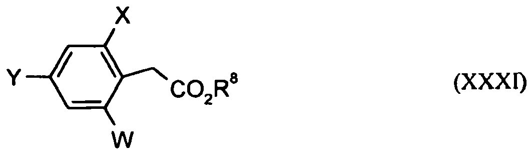 in welcher
W, X, Y und R⁸ die oben angegebene Bedeutung haben.

10. Verbindungen der Formel (XLII) 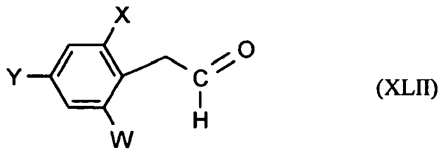 in welcher
W, X und Y die oben angegebene Bedeutung haben.

11. Verwendung von Verbindungen der Formel (I) gemäß Anspruch 1 zur Herstellung von Schädlingsbekämpfungsmitteln und/oder Herbiziden.

12. Schädlingsbekämpfungsmittel und/oder Herbizide, **gekennzeichnet durch** einen Gehalt an mindestens einer Verbindung der Formel (I) gemäß Anspruch 1.

13. Verfahren zur Bekämpfung von tierischen Schädlingen und/oder unerwünschtem Pflanzenbewuchs, **dadurch gekennzeichnet, dass** man Verbindungen der Formel (I) gemäß Anspruch 1 auf Schädlinge und/oder ihren Lebensraum einwirken lässt
wobei Verfahren zur chirurgischen oder therapeutischen Behandlung des menschlichen und tierischen Körpers ausgenommen sind.

14. Verwendung von Verbindungen der Formel (I) gemäß Anspruch 1 zur Bekämpfung von tierischen Schädlingen und/oder unerwünschtem Pflanzenbewuchs,
wobei die Verwendung in Verfahren zur chirurgischen oder therapeutischen Behandlung des menschlichen und tierischen Körpers ausgenommen ist.

15. Verfahren zur Herstellung von Schädlingsbekämpfungsmitteln und/oder Herbiziden, **dadurch gekennzeichnet, dass** man Verbindungen der Formel (I) gemäß Anspruch 1 mit Streckmitteln und/oder oberflächenaktiven Stoffen vermischt.

16. Mittel enthaltend einen wirksamen Gehalt an einer Wirkstoffkombination umfassend
a') mindestens ein substituiertes, cyclisches Ketoenol der Formel (I) gemäβ Anspruch 1, in welcher CKE, W, X und Y die oben angegebene Bedeutung haben
und
(b') zumindest eine die Kulturpflanzen-Verträglichkeit verbessernde Verbindung aus der folgenden Gruppe von Verbindungen:
4-Dichloracetyl-1-oxa-4-aza-spiro[4.5]-decan (AD-67, MON-4660), 1-Dichloracetyl-hexa-hydro-3,3,8a-trimethylpyrrolo[1,2-a]-pyrimidin-6(2H)-on (Dicyclonon, BAS-145138), 4-Dichloracetyl-3,4-dihydro-3-methyl-2H-1,4-benzoxazin (Benoxacor), 5-Chlor-chinolin-8-oxy-essigsäure-(1-methyl-hexylester) (Cloquintocet-mexyl - vgl. auch verwandte Verbindungen in EP-A-86750, EP-A-94349, EP-A-191736, EP-A-492366), 3-(2-Chlorbenzyl)-1-(1-methyl-1-phenyl-ethyl)-harnstoff (Cumyluron), α-(Cyanomethoximino)-phenylacetonitril (Cyometrinil), 2,4-Dichlor-phenoxyessigsäure (2,4-D), 4-(2,4-Dichlorphenoxy)-buttersäure (2,4-DB), 1-(1-Methyl-1-phenyl-ethyl)-3-(4-methyl-phenyl)-harnstoff (Daimuron, Dymron), 3,6-Dichlor-2-methoxy-benzoesäure (Dicamba), Piperidin-1-thiocarbonsäure-S-1-methyl-1-phenyl-ethylester (Dimepiperate), 2,2-Dichlor-N-(2-oxo-2-(2-propenylamino)-ethyl)-N-(2-propenyl)-acetamid (DKA-24), 2,2-Dichlor-N,N-di-2-propenyl-acetamid (Dichlormid), 4,6-Dichlor-2-phenyl-pyrimidin (Fenclorim), 1-(2,4-Dichlorphenyl)-5-trichlormethyl-1H-1,2,4-triazol-3-carbonsäure-ethylester (Fenchlorazole-ethyl - vgl. auch verwandte Verbindungen in EP-A-174562 und EP-A-346620), 2-Chlor-4-trifluormethyl-thiazol-5-carbonsäure-phenylmethylester (Flurazole), 4-Chlor-N-(1,3-dioxolan-2-yl-methoxy)-α-trifluor-acetophenonoxim (Fluxofenim), 3-Dichloracetyl-5-(2-furanyl)-2,2-dimethyl-oxazolidin (Furilazole, MON-13900), Ethyl-4,5-dihydro-5,5-diphenyl-3-isoxazol-carboxylat (Isoxadifen-ethyl - vgl. auch verwandte Verbindungen in WO-A-95/07897), 1-(Ethoxycarbonyl)-ethyl-3,6-dichlor-2-methoxybenzoat (Lactidichlor), (4-Chlor-o-tolyl-oxy)-essigsäure (MCPA), 2-(4-Chlor-o-tolyloxy)-propionsäure (Mecoprop), Diethyl-1-(2,4-dichlor-phenyl)-4,5-dihydro-5-methyl-1H-pyrazol-3,5-dicarboxylat (Mefenpyr-diethyl - vgl. auch verwandte Verbindungen in WO-A-91/07874) 2-Dichlormethyl-2-methyl-1,3-dioxolan (MG-191), 2-Propenyl-1-oxa-4-azaspiro[4.5]decane-4-carbodithioate (MG-838), 1,8-Naphthalsäureanhydrid, α-(1,3-Dioxolan-2-yl-methoximino)-phenylacetonitril (Oxabetrinil), 2,2-Dichlor-N-(1,3-dioxolan-2-yl-methyl)-N-(2-propenyl)-acetamid (PPG-1292), 3-Dichloracetyl-2,2-dimethyl-oxazolidin (R-28725), 3-Dichloracetyl-2,2,5-trimethyl-oxa-zolidin (R-29148), 4-(4-Chlor-o-tolyl)-buttersäure, 4-(4-Chlor-phenoxy)-buttersäure, Diphenylmethoxyessigsäure, Diphenylmethoxyessigsäure-methylester, Diphenylmethoxyessigsäure-ethylester, 1-(2-Chlor-phenyl)-5-phenyl-1H-pyrazol-3-carbonsäure-methylester, 1-(2,4-Dichlor-phenyl)-5-methyl-1H-pyrazol-3-carbonsäure-ethylester, 1-(2,4-dichlorphenyl)-5-isopropyl-1H-pyrazol-3-carbonsäure-ethylester, 1-(2,4-Dichlor-phenyl)-5-(1,1-dimethyl-ethyl)-1H-pyrazol-3-carbonsäure-ethylester, 1-(2,4-Dichlor-phenyl)-5-phenyl-1H-pyrazol-3-carbonsäure-ethylester (vgl. auch verwandte Verbindungen in EP-A-269806 und EP-A-333131), 5-(2,4-Dichlor-benzyl)-2-isoxazolin-3-carbonsäure-ethylester, 5-Phenyl-2-isoxazolin-3-carbonsäure-ethylester, 5-(4-Fluor-phenyl)-5-phenyl-2-isoxazolin-3-carbonsäure-ethylester (vgl. auch verwandte Verbindungen in WO-A-91/08202), 5-Chlor-chinolin-8-oxy-essigsäure-(1,3-dimethyl-but-1-yl)-ester, 5-Chlor-chinolin-8-oxy-essigsäure-4-allyloxy-butylester, 5-Chlor-chinolin-8-oxy-essigsäure-1-allyloxy-prop-2-yl-ester, 5-Chlor-chinoxalin-8-oxy-essigsäure-methylester, 5-Chlor-chinolin-8-oxy-essigsäure-ethylester, 5-Chlor-chinoxalin-8-oxy-essigsäure-allylester, 5-Chtor-chinolin-8-oxy-essigsäure-2-oxo-prop-1-yl-ester, 5-Chlor-chinolin-8-oxy-malonsäure-diethylester, 5-Chlor-chinoxalin-8-oxy-malonsäure-diallylester, 5-Chlor-chinolin-8-oxy-malonsäure-diethylester (vgl. auch verwandte Verbindungen in EP-A-582198), 4-Carboxy-chroman-4-yl-essigsäure (AC-304415, vgl. EP-A-613618), 4-Chlor-phenoxy-essigsäure, 3,3'-Dimethyl-4-methoxy-benzophenon, 1-Brom-4-chlormethylsulfonyl-benzol, 1-[4-(N-2-Methoxybenzoyl-sulfamoyl)-phenyl]-3-methyl-harnstoff (alias N-(2-Methoxy-benzoyl)-4-[(methylamino-carbonyl)-amino]-benzolsulfonamid), 1-[4-(N-2-Methoxybenzoylsulfamoyl)-phenyl]-3,3-dimethyl-harnstoff, 1-[4-(N-4,5-Dimethylbenzoylsulfamoyl)-phenyl]-3-methyl-harnstoff, 1-[4-(N-Naphthylsulfamoyl)-phenyl]-3,3-dimethyl-harnstoff, N-(2-Methoxy-5-methyl-benzoyl)-4-(cyclopropylaminocarbonyl)-benzolsulfonamid,
und/oder eine der folgenden durch allgemeine Formeln definierten Verbindungen der allgemeinen Formel (IIa) 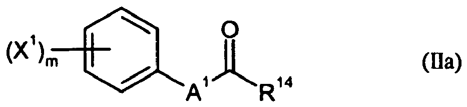 oder der allgemeinen Formel (IIb) 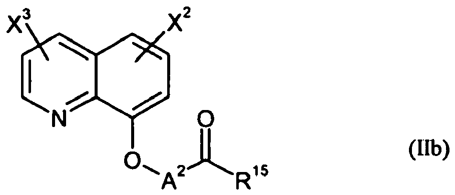 oder der Formel (IIc) 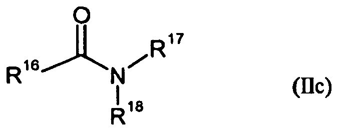 wobei
m für eine Zahl 0, 1, 2, 3, 4, oder 5 steht,
A¹ für eine der nachstehend skizzierten divalenten heterocyclischen Gruppierungen steht, 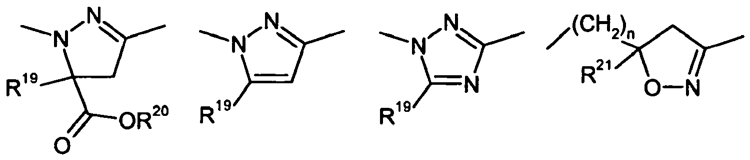
n für eine Zahl 0, 1 ,2, 3, 4 oder 5 steht,
A² für gegebenenfalls durch C₁-C₄-Alkyl und/oder C₁-C₄-Alkoxy-carbonyl und/oder C₁-C₄-Alkenyloxy-carbonyl substituiertes Alkandiyl mit 1 oder 2 Kohlenstoffatomen steht,
R¹⁴ für Hydroxy, Mercapto, Amino, C₁-C₆-Alkoxy, C₁-C₆-Alkylthio, C₁-C₆-Alkylamino oder Di-(C₁-C₄-alkyl)-amino steht,
R¹⁵ für Hydroxy, Mercapto, Amino, C₁-C₇-Alkoxy, C₁-C₆-Alkenyloxy, C₁-C₆-Alkenyloxy-C₁-C₆-alkoxy, C₁-C₆-Alkylthio, C₁-C₆-Alkylamino oder Di-(C₁-C₄-alkyl)-amino steht,
R¹⁶ für gegebenenfalls durch Fluor, Chlor und/oder Brom substituiertes C₁-C₄-Alkyl steht,
R¹⁷ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl steht,
R¹⁸ für Wasserstoff, jeweils gegebenenfalls durch Fluor, Chlor und/oder Brom substituiertes C₁-C₆-Alkyl, C₂-C₆-Alkenyl oder C₂-C₆-Alkinyl, C₁-C₄-Alkoxy-C₁-C₄-alkyl, Dioxolanyl-C₁-C₄-alkyl, Furyl, Furyl-C₁-C₄-alkyl, Thienyl, Thiazolyl, Piperidinyl, oder gegebenenfalls durch Fluor, Chlor und/oder Brom oder C₁-C₄-Alkyl substituiertes Phenyl, R¹⁷ und R¹⁸ auch gemeinsam für jeweils gegebenenfalls durch C₁-C₄-Alkyl, Phenyl, Furyl, einen annellierten Benzolring oder durch zwei Substituenten, die gemeinsam mit dem C-Atom, an das sie gebunden sind, einen 5- oder 6-gliedrigen Carboxyclus bilden, substituiertes C₃-C₆-Alkandiyl oder C₂-C₅-Oxaalkandiyl stehen,
R¹⁹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
R²⁰ für Wasserstoff, jeweils gegebenenfalls durch Hydroxy, Cyano, Halogen oder C₁-C₄-Alkoxy substituiertes C₁-C₆-Alkyl, C₃-C₆-Cycloalkyl oder Tri-(C₁-C₄-alkyl)-silyl steht,
R²¹ für Wasserstoff, Cyano, Halogen, oder für jeweils gegebenenfalls durch Fluor, Chlor und/oder Brom substituiertes C₁-C₄-Alkyl, C₃-C₆-Cycloalkyl oder Phenyl steht,
X¹ für Nitro, Cyano, Halogen, C₁-C₄-Alkyl, C₁-C₄-Halogenalkyl, C₁-C₄-Alkoxy oder C₁-C₄-Halogenalkoxy steht,
X² für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
X³ für Wasserstoff, Cyano, Nitro, Halogen, C₁-C₄-Alkyl, C₁-C₄-Halogenalkyl, C₁-C₄-Alkoxy oder C₁-C₄-Halogenalkoxy steht,
und/oder die folgenden durch allgemeine Formeln definierten Verbindungen der allgemeinen Formel (IId) 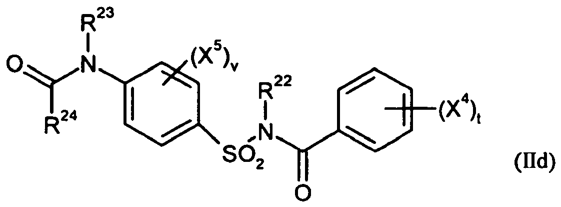 oder der allgemeinen Formel (IIe) 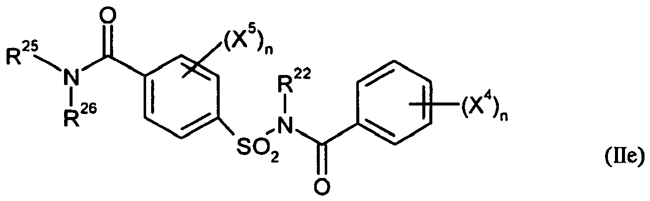 wobei
t für eine Zahl 0, 1, 2, 3, 4 oder 5 steht,
v für eine Zahl 0, 1, 2, 3, 4 oder 5 steht,
R²² für Wasserstoff oder C₁-C₄-Alkyl steht,
R²³ für Wasserstoff oder C₁-C₄-Alkyl steht,
R²⁴ für Wasserstoff, jeweils gegebenenfalls durch Cyano, Halogen oder C₁-C₄-Alkoxy substituiertes C₁-C₆-Alkyl, C₁-C₆-Alkoxy, C₁-C₆-Alkylthio, C₁-C₆-Alkylamino oder Di-(C₁-C₄-alkyl)-amino, oder jeweils gegebenenfalls durch Cyano, Halogen oder C₁ C₄-Alkyl substituiertes C₃-C₆-Cycloalkyl, C₃-C₆-Cycloalkyloxy, C₃-C₆-Cycloalkyl-thio oder C₃-C₆-Cycloalkylamino steht,
R²⁵ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, oder gegebenenfalls durch Cyano, Halogen oder C₁-C₄-Alkyl substituiertes C₃-C₆-Cycloalkyl steht,
R²⁶ für Wasserstoff, gegebenenfalls durch Cyano, Hydroxy, Halogen oder C₁-C₄-Alkoxy substituiertes C₁-C₆-Alkyl, jeweils gegebenenfalls durch Cyano oder Halogen substituiertes C₃-C₆-Alkenyl oder C₃-C₆-Alkinyl, gegebenenfalls durch Cyano, Halogen oder C₁-C₄-Alkyl substituiertes C₃-C₆-Cycloalkyl, oder gegebenenfalls durch Nitro, Cyano, Halogen, C₁-C₄-Alkyl, C₁-C₄-Halogenalkyl, C₁-C₄-Alkoxy oder C₁-C₄-Halogenalkoxy substituiertes Phenyl steht, oder zusammen mit R²⁵ für jeweils gegebenenfalls durch C₁-C₄-Alkyl substituiertes C₂-C₆-Alkandiyl oder C₂-C₅-Oxa-alkandiyl steht,
X⁴ für Nitro, Cyano, Carboxy, Carbamoyl, Formyl, Sulfamoyl, Hydroxy, Amino, Halogen, C₁-C₄-Alkyl, C₁-C₄-Halogenalky), C₁-C₄-Alkoxy oder C₁-C₄-Halogenalkoxy steht, und
X⁵ für Nitro, Cyano, Carboxy, Carbamoyl, Formyl, Sulfamoyl, Hydroxy, Amino, Halogen, C₁-C₄-Alkyl, C₁-C₄-Halogenalkyl, C₁-C₄-Alkoxy oder C₁-C₄-Halogenalkoxy steht.

17. Mittel nach Anspruch 16, bei dem die die Kulturpflanzen-Verträglichkeit verbessernde Verbindung aus der folgenden Gruppe von Verbindungen ausgewählt ist:
Cloquintocet-mexyl, Fenchlorazole-ethyl, Isoxadifen-ethyl, Mefenpyr-diethyl, Furilazole, Fenclorim, Cumyluron, Dymron oder die Verbindungen IIe-5 oder IIe-11.

18. Verfahren zum Bekämpfen von unerwünschtem Pflanzenwuchs, **dadurch gekennzeichnet, dass** man ein Mittel gemäß Anspruch 16 auf die Pflanzen oder ihre Umgebung einwirken lässt.

19. Verwendung eines Mittels gemäß Anspruch 16 zur Bekämpfung von unerwünschtem Pflanzenwuchs.

20. Mittel gemäß Anspruch 16 bei dem die Kulturpflanzenverträglichkeit verbessernde Verbindung Cloquintocet-metyl oder Mefenpyr-diethyl ist.

## Claims

1. Compounds of the formula (I) 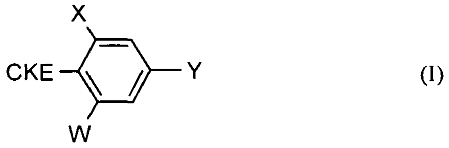 in which
W represents methoxy, ethoxy, n-propoxy, methoxyethoxy or cyclopropylmethoxy,
X represents chlorine,
Y represents methyl,
CKE represents one of the groups 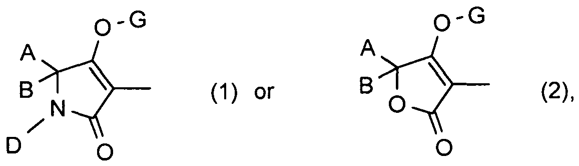
A represents methyl, isopropyl, isobutyl or cyclopropyl,
B represents hydrogen, methyl or ethyl,
A, B and the carbon atom to which they are attached represent saturated C₃-C₆-cycloalkyl in which optionally one ring atom is replaced by oxygen and which is optionally monosubstituted by methyl or methoxy,
D represents hydrogen, methyl or ethyl,
G represents hydrogen (a) or represents one of the groups 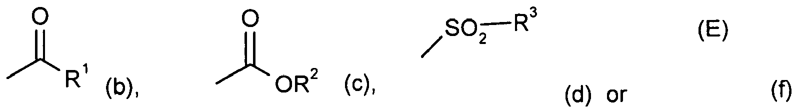
E represents an ammonium ion,
R¹ represents C₁-C₆-alkyl, C₁-C₂-alkoxy-C₁-alkyl, C₃-C₆-cycloalkyl, C₁-C₄-alkyl which is monosubstituted by chlorine or represents phenyl which is optionally monosubstituted by chlorine,
R² represents C₁-C₈-alkyl, C₃-C₆-alkenyl or benzyl,
R³ represents C₁-C₆-alkyl.

2. Process for preparing compounds of the formula (I) according to Claim 1, **characterized in that**, to obtain
(A) compounds of the formula (I-1-a) 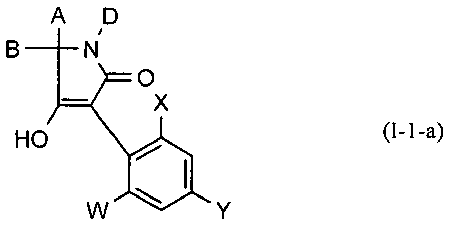 in which
A, B, D, W, X and Y are as defined above,
compounds of the formula (II) 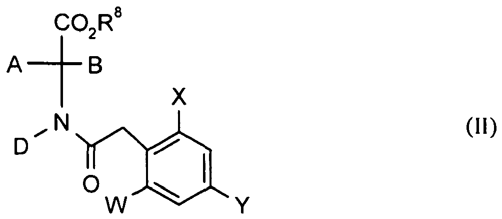 in which
A, B, D, W, X and Y are as defined above,
and
R⁸ represents alkyl,
are condensed intramolecularly in the presence of a diluent and in the presence of a base,
(B) compounds of the formula (I-2-a) 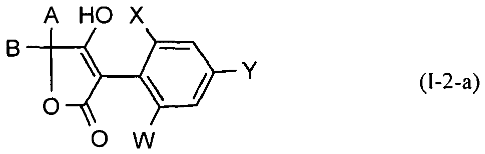 in which
A, B, W, X and Y are as defined above,
compounds of the formula (III) 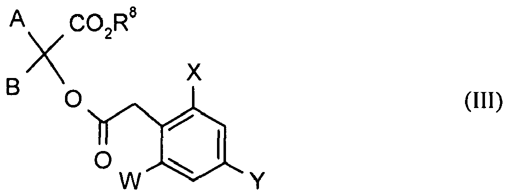 in which
A, B, W, X, Y and R⁸ are as defined above,
are condensed intramolecularly in the presence of a diluent and in the presence of a base,
(I) compounds of the formulae (I-1-b) to (I-2-b) shown above in which A, B, D, R¹, W, X and Y are as defined above, compounds of the formulae (I-1-a) to (I-2-a) shown above in which A, B, D, W, X and Y are as defined above are in each case
(α) reacted with acid halides of the formula (XIII) 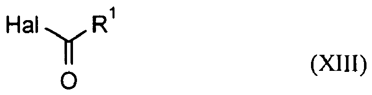
in which
R¹ is as defined above and
Hal represents halogen,
or
(β) reacted with carboxylic anhydrides of the formula (XIV)
R¹-CO-O-CO-R¹ (XIV)
in which
R¹ is as defined above,
if appropriate in the presence of a diluent and if appropriate in the presence of an acid binder,
(J) compounds of the formulae (I-1-c) to (I-2-c) shown above in which A, B, D, R², M, W, X and Y are as defined above and L represents oxygen, compounds of the formulae (I-1-a) to (I-2-a) shown above in which A, B, D, W, X and Y are as defined above are in each case
reacted with chloroformic esters or chloroformic thioesters of the formula (XV)
R²-M-CO-Cl (XV)
in which
R² and M are as defined above,
if appropriate in the presence of a diluent and if appropriate in the presence of an acid binder,
(L) compounds of the formulae (I-1-d) to (I-2-d) shown above in which A, B, D, R³, W, X and Y are as defined above, compounds of the formulae (I-1-a) to (I-2-a) shown above in which A, B, D, W, X and Y are as defined above are in each case
reacted with sulphonyl chlorides of the formula (XVII)
R³-SO₂-Cl (XVII)
in which
R³ is as defined above,
if appropriate in the presence of a diluent and if appropriate in the presence of an acid binder,
(N) compounds of the formulae (I-1-f) to (I-2-f) shown above in which A, B, D, E, W, X and Y are as defined above, compounds of the formulae (I-1-a) to (I-2-a) shown above in which A, B, D, W, X and Y are as defined above are in each case
reacted with metal compounds or amines of the formulae (XIX) and (XX), respectively,
Me(OR¹⁰)ₜ (XIX) 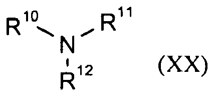
in which
Me represents a mono- or divalent metal,
t represents the number 1 or 2 and
R¹⁰, R¹¹, R¹² independently of one another represent hydrogen or alkyl,
if appropriate in the presence of a diluent,
(P) compounds of the formulae (I-1-a) to (I-2-a) shown above in which A, B, D, W, X and Y are as defined above, compounds of the formulae (I-1-a') to (I-2-a') in which A, B, D, X and Y are as defined above and W' represents bromine 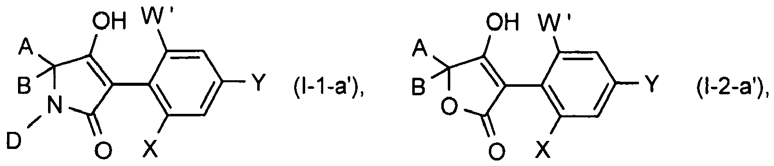 are reacted with alcohols of the formula
W-OH
in which
W is as defined above, if appropriate in the presence of a solvent, a Cu(I) salt and a strong base.

3. Compounds of the formula (II) 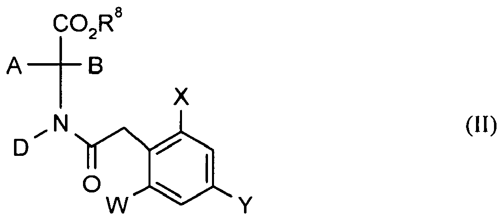 in which
R⁸, A, B, D, W, X and Y are as defined above.

4. Compounds of the formula (III) 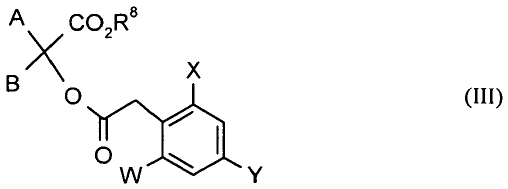 in which
R⁸, A, B, W, X and Y are as defined above.

5. Compounds of the formula (XXV) 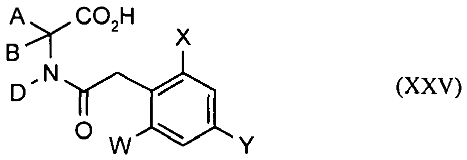 in which
A, B, D, W, X and Y are as defined above.

6. Compounds of the formula (XXIV) 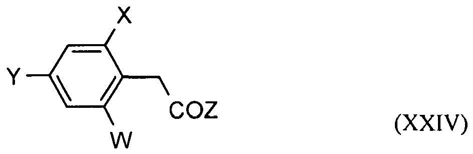 in which
W, X, Y and Z are as defined above.

7. Compounds of the formula (XXVII) 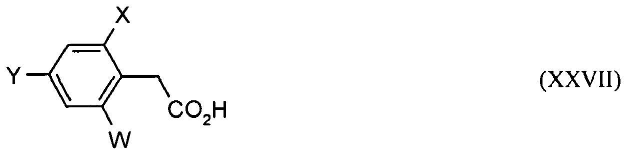 in which
W, X and Y are as defined above.

8. Compounds of the formula (XXIX) 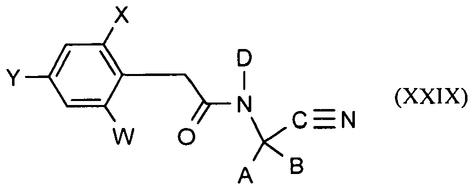 in which
A, B, D, W, X and Y are as defined above.

9. Compounds of the formula (XXXI) 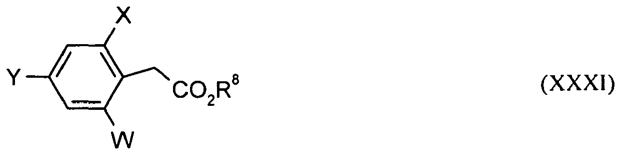 in which W, X, Y and R⁸ are as defined above.

10. Compounds of the formula (XLII) 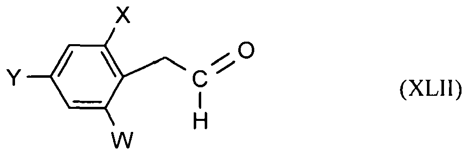 in which
W, X and Y are as defined above.

11. Use of compounds of the formula (I) according to Claim 1 for preparing pesticides and/or herbicides.

12. Pesticides and/or herbicides, **characterized in that** they comprise at least one compound of the formula (I) according to Claim 1.

13. Method for controlling animal pests and/or unwanted vegetation, **characterized in that** compounds of the formula (I) according to Claim 1 are allowed to act on pests and/or their habitat, wherein methods for the surgical or therapeutic treatment of the human and animal body are excluded.

14. Use of compounds of the formula (I) according to Claim 1 for controlling animal pests and/or unwanted vegetation, wherein the use in methods for the surgical or therapeutic treatment of the human and animal body is excluded.

15. Process for preparing pesticides and/or herbicides, **characterized in that** compounds of the formula (I) according to Claim 1 are mixed with extenders and/or surfactants.

16. Compositions, comprising an effective amount of an active compound combination comprising
a') at least one substituted cyclic ketoenol of the formula (I) according to Claim 1 in which CKE, W, X and Y are as defined above
and
(b') at least one compound which improves crop plant tolerance and which is selected from the following group of compounds:
4-dichloroacetyl-1-oxa-4-aza-spiro[4.5]-decane (AD-67, MON-4660), 1-dichloroacetylhexahydro-3,3,8a-trimethylpyrrolo[1,2-a]-pyrimidin-6(2H)-one (dicyclonon, BAS-145138), 4-dichloroacetyl-3,4-dihydro-3-methyl-2H-1,4-benzoxazine (benoxacor), 1-methyl-hexyl 5-chloroquinolin-8-oxy-acetate (cloquintocet-mexyl - cf. also related compounds in EP-A-86750, EP-A-94349, EP-A-191736, EP-A-492366), 3-(2-chlorobenzyl)-1-(1-methyl-1-phenyl-ethyl)-urea (cumyluron), α-(cyanomethoximino)-phenylacetonitrile (cyometrinil), 2,4-dichlorophenoxyacetic acid (2,4-D), 4-(2,4-dichloro-phenoxy)-butyric acid (2,4-DB), 1-(1-methyl-1-phenyl-ethyl)-3-(4-methyl-phenyl)-urea (daimuron, dymron), 3,6-dichloro-2-methoxy-benzoic acid (dicamba), S-1-methyl-1-phenyl-ethyl piperidine-1-thiocarboxylate (dimepiperate), 2,2-dichloro-N-(2-oxo-2-(2-propenylamino)-ethyl)-N-(2-propenyl)-acetamide (DKA-24), 2,2-dichloro-N,N-di-2-propenyl-acetamide (dichlormid), 4,6-dichloro-2-phenyl-pyrimidine (fenclorim), ethyl 1-(2,4-dichloro-phenyl)-5-trichloromethyl-1H-1,2,4-triazole-3-carboxylate (fenchlorazole-ethyl - cf. also related compounds in EP-A-174562 and EP-A-346620), phenylmethyl 2-chloro-4-trifluoromethyl-thiazole-5-carboxylate (flurazole), 4-chloro-N-(1,3-dioxolan-2-yl-methoxy)-α-trifluoroacetophenone oxime (fluxofenim), 3-dichloroacetyl-5-(2-furanyl)-2,2-dimethyl-oxazolidine (furilazole, MON-13900), ethyl 4,5-dihydro-5,5-diphenyl-3-isoxazolecarboxylate (isoxadifen-ethyl - cf. also related compounds in WO-A-95/07897), 1-(ethoxycarbonyl)-ethyl-3,6-dichloro-2-methoxybenzoate (lactidichlor), (4-chloro-o-tolyloxy)-acetic acid (MCPA), 2-(4-chloro-o-tolyloxy)-propionic acid (mecoprop), diethyl 1-(2,4-dichloro-phenyl)-4,5-dihydro-5-methyl-1H-pyrazole-3,5-dicarboxylate (mefenpyr-diethyl - cf. also related compounds in WO-A-91/07874), 2-dichloromethyl-2-methyl-1,3-dioxolane (MG-191), 2-propenyl-1-oxa-4-azaspiro[4.5]decane 4-carbodithioate (MG-838), 1,8-naphthalic anhydride, α-(1,3-dioxolan-2-yl-methoximino)-phenylacetonitrile (oxabetrinil), 2,2-dichloro-N-(1,3-dioxolan-2-yl-methyl)-N-(2-propenyl)-acetamide (PPG-1292), 3-dichloroacetyl-2,2-dimethyl-oxazolidine (R-28725), 3-dichloroacetyl-2,2,5-trimethyl-oxazolidine (R-29148), 4-(4-chloro-o-tolyl)-butyric acid, 4-(4-chloro-phenoxy)-butyric acid, diphenylmethoxyacetic acid, methyl diphenylmethoxyacetate, ethyl diphenylmethoxyacetate, methyl 1-(2-chloro-phenyl)-5-phenyl-1H-pyrazole-3-carboxylate, ethyl 1-(2,4-dichloro-phenyl)-5-methyl-1H-pyrazole-3-carboxylate, ethyl 1-(2,4-dichloro-phenyl)-5-isopropyl-1H-pyrazole-3-carboxylate, ethyl 1-(2,4-dichloro-phenyl)-5-(1,1-dimethyl-ethyl)-1H-pyrazole-3-carboxylate, ethyl 1-(2,4-dichloro-phenyl)-5-phenyl-1H-pyrazole-3-carboxylate (cf. also related compounds in EP-A-269806 and EP-A-333131), ethyl 5-(2,4-dichloro-benzyl)-2-isoxazoline-3-carboxylate, ethyl 5-phenyl-2-isoxazoline-3-carboxylate, ethyl 5-(4-fluoro-phenyl)-5-phenyl-2-isoxazoline-3-carboxylate (cf. also related compounds in WO-A-91/08202), 1,3-dimethyl-but-1-yl 5-chloro-quinolin-8-oxy-acetate, 4-allyloxy-butyl 5-chloro-quinolin-8-oxy-acetate, 1-allyloxy-prop-2-yl 5-chloro-quinolin-8-oxy-acetate, methyl 5-chloro-quinoxalin-8-oxy-acetate, ethyl 5-chloro-quinolin-8-oxy-acetate, allyl 5-chloro-quinoxalin-8-oxyacetate, 2-oxo-prop-1-yl 5-chloro-quinolin-8-oxy-acetate, diethyl 5-chloro-quinolin-8-oxy-malonate, diallyl 5-chloro-quinoxalin-8-oxy-malonate, diethyl 5-chloro-quinolin-8-oxy-malonate (cf. also related compounds in EP-A-582198), 4-carboxy-chroman-4-yl-acetic acid (AC-304415, cf. EP-A-613618), 4-chloro-phenoxy-acetic acid, 3,3'-dimethyl-4-methoxy-benzophenone, 1-bromo-4-chloromethylsulphonyl-benzene, 1-[4-(N-2-methoxybenzoylsulphamoyl)-phenyl]-3-methyl-urea (alias N-(2-methoxy-benzoyl)-4-[(methylamino-carbonyl)-amino]-benzenesulphonamide), 1-[4-(N-2-methoxybenzoy)sulphamoyl)-phenyl]-3,3-dimethyl-urea, 1-[4-(N-4,5-dimethylbenzoylsulphamoyl)-phenyl]-3-methyl-urea, 1-[4-(N-naphthylsulphamoyl)-phenyl]-3,3-dimethyl-urea, N-(2-methoxy-5-methyl-benzoyl)-4-(cyclopropylaminocarbonyl)-benzenesulphonamide,
and/or one of the following compounds (defined by general formulae)
of the general formula (IIa) 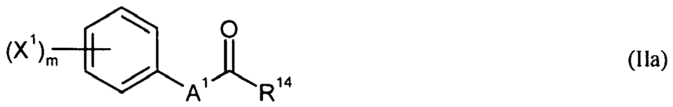 or of the general formula (IIb) 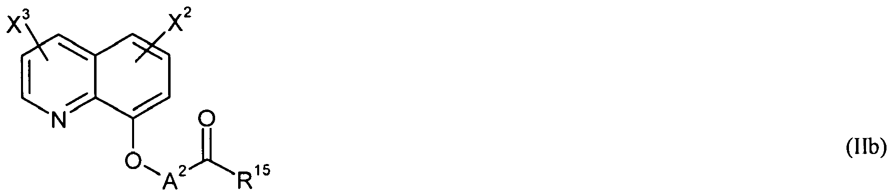 or of the formula (IIc) 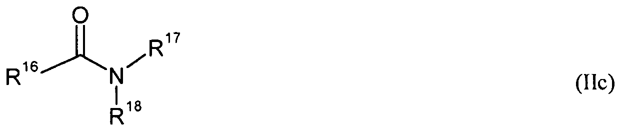 where
m is 0, 1, 2, 3, 4 or 5,
A¹ represents one of the divalent heterocyclic groups outlined hereinbelow, 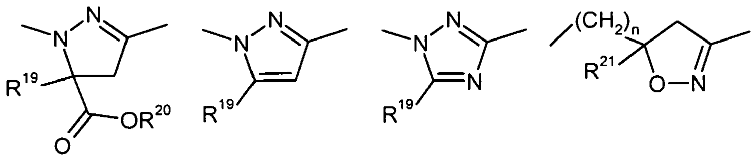
n is 0, 1, 2, 3, 4 or 5,
A² represents alkanediyl having 1 or 2 carbon atoms which is optionally substituted by C₁-C₄-alkyl and/or C₁-C₄-alkoxy-carbonyl and/or C₁-C₄-alkenyloxy-carbonyl,
R¹⁴ represents hydroxyl, mercapto, amino, C₁-C₆-alkoxy, C₁-C₆-alkylthio, C₁-C₆-alkylamino or di-(C₁-C₄-alkyl)amino,
R¹⁵ represents hydroxyl, mercapto, amino, C₁-C₇-alkoxy, C₁-C₆-alkenyloxy, C₁-C₆-alkenyloxy-C₁-C₆-alkoxy, C₁-C₆-alkylthio, C₁-C₆-alkylamino or di-(C₁-C₄-alkyl)amino,
R¹⁶ represents C₁-C₄-alkyl which is optionally substituted by fluorine, chlorine and/or bromine,
R¹⁷ represents hydrogen, or represents C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, each of which is optionally substituted by fluorine, chlorine and/or bromine, or represents phenyl which is optionally substituted by fluorine, chlorine and/or bromine or C₁-C₄-alkyl,
R¹⁸ represents hydrogen, or represents C₁-C₆-alkyl, C₂-C₆-alkenyl or C₂-C₆-alkynyl, C₁-C₄-alkoxy-C₁-C₄-alkyl, dioxolanyl-C₁-C₄-alkyl, furyl, furyl-C₁-C₄-alkyl, thienyl, thiazolyl, piperidinyl, each of which is optionally substituted by fluorine, chlorine and/or bromine, or represents phenyl which is optionally substituted by fluorine, chlorine and/or bromine or C₁-C₄-alkyl, or R¹⁷ and R¹⁸ together also represent C₃-C₆-alkanediyl or C₂-C₅-oxaalkanediyl, each of which is optionally substituted by C₁-C₄-alkyl, phenyl, furyl, a fused benzene ring or by two substituents which, together with the C atom to which they are bonded, form a 5- or 6-membered carbocycle,
R¹⁹ represents hydrogen, cyano, halogen, or represents C₁-C₄-alkyl, C₃-C₆-cycloalkyl or phenyl, each of which is optionally substituted by fluorine, chlorine and/or bromine,
R²⁰ represents hydrogen, or represents C₁-C₆-alkyl, C₃-C₆-cycloalkyl or tri(C₁-C₄-alkyl)silyl, each of which is optionally substituted by hydroxyl, cyano, halogen or C₁-C₄-alkoxy,
R²¹ represents hydrogen, cyano, halogen, or represents C₁-C₄-alkyl, C₃-C₆-cycloalkyl or phenyl, each of which is optionally substituted by fluorine, chlorine and/or bromine,
X¹ represents nitro, cyano, halogen, C₁-C₄-alkyl, C₁-C₄-haloalkyl, C₁-C₄-alkoxy or C₁-C₄-haloalkoxy,
X² represents hydrogen, cyano, nitro, halogen, C₁-C₄-alkyl, C₁-C₄-haloalkyl, C₁-C₄-alkoxy or C₁-C₄-haloalkoxy,
X³ represents hydrogen, cyano, nitro, halogen, C₁-C₄-alkyl, C₁-C₄-haloalkyl, C₁-C₄-alkoxy or C₁-C₄-haloalkoxy,
and/or the following compounds (defined by general formulae)
of the general formula (IId) 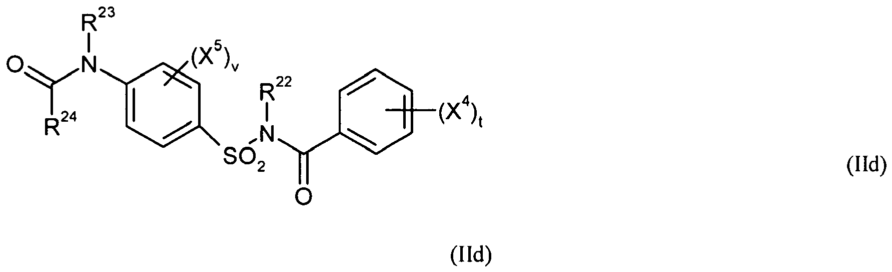 or of the general formula (IIe) 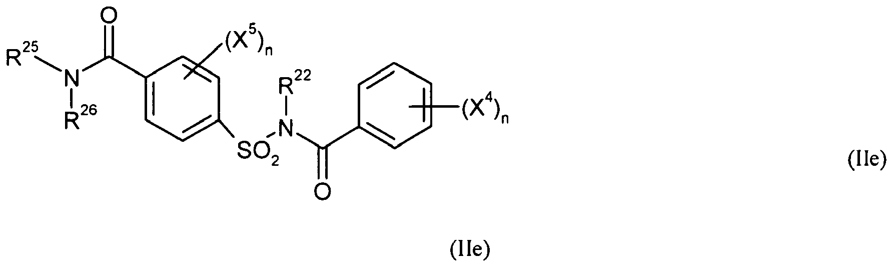 where
t is 0, 1, 2, 3, 4 or 5,
v is 0, 1, 2, 3, 4 or 5,
R²² represents hydrogen or C₁-C₄-alkyl,
R²³ represents hydrogen or C₁-C₄-alkyl,
R²⁴ represents hydrogen, or represents C₁-C₆-alkyl, C₁-C₆-alkoxy, C₁-C₆-alkylthio, C₁-C₆-alkylamino or di-(C₁-C₄-alkyl)amino, each of which is optionally substituted by cyano, halogen or C₁-C₄-alkoxy, or represents C₃-C₆-cycloalkyl, C₃-C₆-cycloalkyloxy, C₃-C₆-cycloalkylthio or C₃-C₆-cycloalkylamino, each of which is optionally substituted by cyano, halogen or C₁-C₄-alkyl,
R²⁵ represents hydrogen, or represents C₁-C₆-alkyl which is optionally substituted by cyano, hydroxyl, halogen or C₁-C₄-alkoxy, or represents C₃-C₆-alkenyl or C₃-C₆-alkynyl, each of which is optionally substituted by cyano or halogen, or represents C₃-C₆-cycloalkyl which is optionally substituted by cyano, halogen or C₁-C₄-alkyl,
R²⁶ represents hydrogen, or represents C₁-C₆-alkyl which is optionally substituted by cyano, hydroxyl, halogen or C₁-C₄-alkoxy, or represents C₃-C₆-alkenyl or C₃-C₆-alkynyl, each of which is optionally substituted by cyano or halogen, or represents C₃-C₆-cycloalkyl which is optionally substituted by cyano, halogen or C₁-C₄-alkyl, or represents phenyl which is optionally substituted by nitro, cyano, halogen, C₁-C₄-alkyl, C₁-C₄-haloalkyl, C₁-C₄-alkoxy or C₁-C₄-haloalkoxy, or together with R²⁵ represents C₂-C₆-alkanediyl or C₂-C₅-oxaalkanediyl, each of which is optionally substituted by C₁-C₄-alkyl,
X⁴ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy, and
X⁵ represents nitro, cyano, carboxyl, carbamoyl, formyl, sulphamoyl, hydroxyl, amino, halogen, C₁-C₄-alkyl, C₁-C₄-haloalkyl, C₁-C₄-alkoxy or C₁-C₄-haloalkoxy.

17. Composition according to Claim 16, in which the compound which improves crop plant tolerance is selected from the following group of compounds:
cloquintocet-mexyl, fenchlorazole-ethyl, isoxadifen-ethyl, mefenpyr-diethyl, furilazole, fenclorim, cumyluron, dymron or the compounds IIe-5 or IIe-11.

18. Method for controlling unwanted vegetation, **characterized in that** a composition according to Claim 16 is allowed to act on the plants or their habitat.

19. Use of a composition according to Claim 16 for controlling unwanted vegetation.

20. Composition according to Claim 16 in which the compound which improves crop plant tolerance is cloquintocet-mexyl or mefenpyr-diethyl.

## Revendications

1. Composés de formule (I) 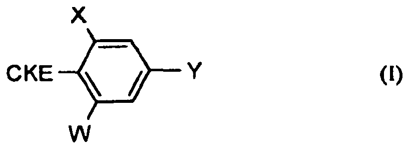 dans laquelle
W représente méthoxy, éthoxy, n-propoxy, méthoxy éthoxy ou cyclopropylméthoxy,
X représente chlore,
Y représente m thyle,
CKE représente un des groupes 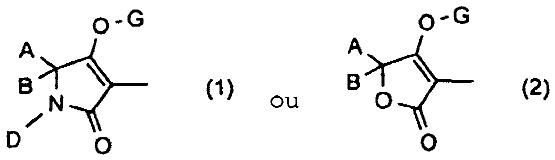
A représente méthyle, i-propyle, i-butyle ou cyclopropyle,
B représente hydrogéne, méthyle ou éthyle,
A, B et l'atome de carbone auquel ils sont liés représentent C₅-C₆-cycloalkyle saturé , dans lequel le cas échéant un atome de cycle est remplacé par oxygéne et qui est le cas échéant monosubstitué par méthyle ou méthoxy,
D représente hydrogéne, méthyle ou éthyle,
G représente hydrogéne (a) ou un des groupes 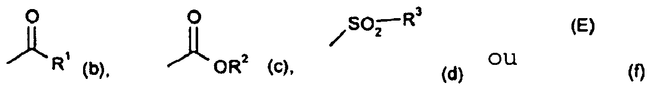
E représente un ion d'ammonium,
R¹ représente C₁-C₆-alkyle, C₁-C₂-alcoxy-C₁-alkyle, C₃-C₆-cycloalkyle, C₁-C₄-alkyle monosubstitué par chlore ou phényle le cas échéant monosubstitué par chlore,
R² représente C₁-C₈-alkyle, C₃-C₆-alcényle ou benzyle,
R³ représente C₁-C₆-alkyle.

2. Procédé pour la préparation de composés de formule (I) selon la revendication 1, **caractérisé en ce que**, pour obtenir
(A) des composés de formule (I-1-a) 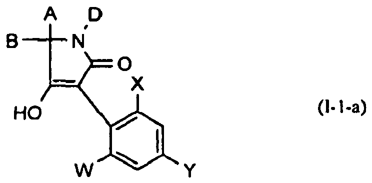 dans laquelle
A, B, D, W, X et Y présentent les significations indiquées ci-dessus,
on condense de manière intramoléculaire des composés de formule (II) 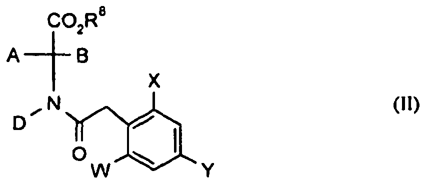 dans laquelle
A, B, D, W, X et Y présentent les significations indiquées ci-dessus, et
R⁸ représente alkyle,
en présence d'un diluant et en présence d'une base,
(B) des composés de formule (I-2-a) 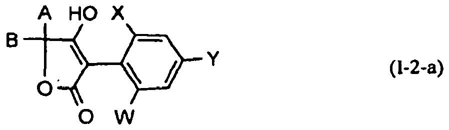 dans laquelle A, B, W, X et Y présentent les significations
indiquées ci-dessus,
on condense de manière intramoléculaire des composés de formule (III) 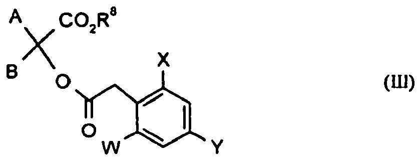 dans laquelle
A, B, W, X, Y et R⁸ présentent les significations indiquées ci-dessus,
en présence d'un diluant et en présence d'une base,
(I) des composés des formules (I-1-b) (I-2-b) représentées ci-dessus, dans lesquelles A, B, D, R¹, W, X et Y présentent les significations indiquées ci-dessus, on transforme des composés des formules (I-1-a) (I-2-a) représentées ci-dessus, dans lesquelles A, B, D, W, X et Y présentent les significations indiquées ci-dessus,
chaque fois
(α) avec des halogénures d'acide de formule (XIII) 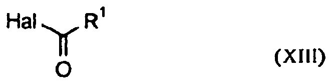 dans laquelle
R¹ présente la signification indiquée ci-dessus et
Hal représente halogéne ou
( ) avec des anhydrides d'acide carboxylique de formule (XIV)
R¹-CO-O-CO-R¹ (XIV) dans laquelle
R¹ présente la signification indiquée ci-dessus,
le cas échéant en présence d'un diluant et le cas échéant en présence d'un liant d'acide ;
(J) des composés des formules (I-1-c) (I-2-c) représentées ci-dessus, dans lesquelles A, B, D, R², M, W, X et Y présentent les significations indiquées ci-dessus et L représente oxygéne, on transforme des composés des formules (I-1-a) (I-2-a) représentées ci-dessus, dans lesquelles A, B, D, W, X et Y présentent les significations indiquées ci-dessus, chaque fois
avec des esters de l'acide chloroformique ou des thioesters de l'acide chloroformique de formule (XV)
R²-M-CO-Cl (XV)
dans laquelle
R² et M présentent les significations indiquées ci-dessus,
le cas échéant en présence d'un diluant et le cas
échéant en présence d'un liant d'acide ;
(L) des composés des formules (I-1-d) (I-2-d) représentées ci-dessus, dans lesquelles A, B, D, R³, W, X et Y présentent les significations indiquées ci-dessus, on transforme des composés des formules (I-1-a) (I-2-a) représentées ci-dessus, dans lesquelles A, B, D, W, X et Y présentent les significations indiquées ci-dessus,
chaque fois
avec des chlorures d'acide sulfonique de formule (XVII)
R³-SO₂-Cl (XVII)
dans laquelle
R³ présente la signification indiquée ci-dessus,
le cas échéant en présence d'un diluant et le cas
échéant en présence d'un liant d'acide,
(N) des composés des formules (I-1-f) (I-2-f) représentées ci-dessus, dans lesquelles A, B, D, E, W, X et Y présentent les significations indiquées ci-dessus, on transforme des composés des formules (I-1-a) (I-2-a) représentées ci-dessus, dans lesquelles A, B, D, W, X et Y présentent les significations indiquées ci-dessus,
chaque fois
avec des composés métalliques ou des amines des formules (XIX) ou (XX)
**Me(OR¹⁰)ₜ** **(XIX)** 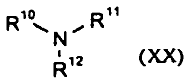
dans lesquelles
Me représente un métal monovalent ou divalent,
t représente le nombre 1 ou 2 et
R¹⁰, R¹¹, R¹² représentent, indépendamment l'un de l'autre, hydrogéne ou alkyle,
le cas échéant en présence d'un diluant,
(P) des composés des formules (I-1-a) (I-2-a) représentées ci-dessus, dans lesquelles A, B, D, W, X et Y présentent la signification indiquée ci-dessus, on transforme des composés des formules (I-1-a') (I-2-a') représentées ci-dessus, dans lesquelles A, B, D, X et Y présentent la signification indiquée ci-dessus et W' représente brome 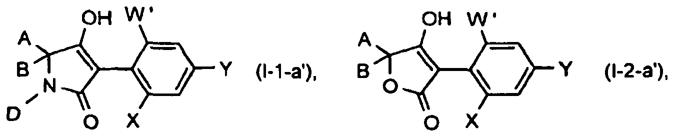 avec des alcools de formule
W-OH
dans laquelle
W présente la signification indiquée ci-dessus, le cas échéant en présence d'un diluant, d'un sel de Cu (I) et d'une base forte.

3. Composés de formule (H) 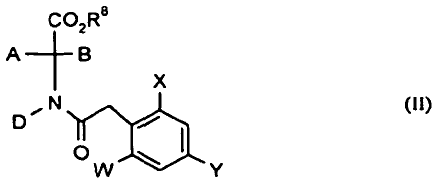 dans laquelle
R⁸, A, B, D, W, X et Y présentent les significations indiquées ci-dessus.

4. Composés de formule (III) 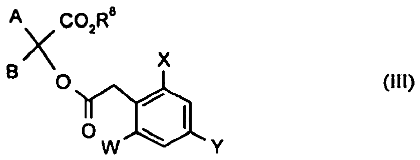 dans laquelle
R⁸, A, B, W, X et Y présentent les significations indiquées ci-dessus.

5. Composés de formule (XXV) 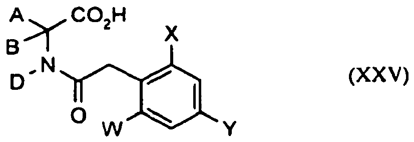 dans laquelle
A, B, D, W, X et Y présentent les significations indiquées ci-dessus.

6. Composés de formule (XXIV) 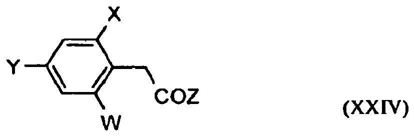 dans laquelle
W, X, Y et Z présentent les significations indiquées ci-dessus.

7. Composés de formule (XXVII) 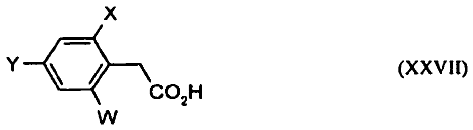 dans laquelle
W, X et Y présentent la signification indiquée ci-dessus.

8. Composés de formule (XXIX) 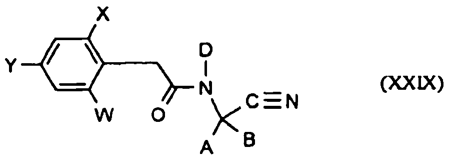 dans laquelle
A, B, D, W, X et Y présentent les significations indiquées ci-dessus.

9. Composés de formule (XXXI) 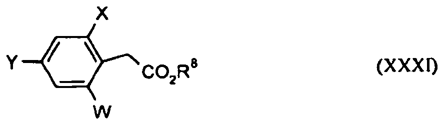 dans laquelle
W, X, Y et R⁸ présentent la signification indiquée ci-dessus.

10. Composés de formule (XLII) 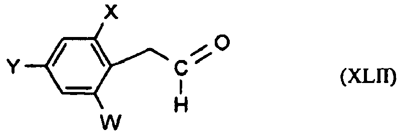 dans laquelle
W, X et Y présentent la signification indiquée ci-dessus.

11. Utilisation de composés de formule (I) selon la revendication 1 pour la préparation d'agents de lutte contre les organismes nuisibles et/ou des herbicides.

12. Agents de lutte contre des organismes nuisibles et/ou herbicides, **caractérisés par** une teneur en au moins un composé de formule (I) selon la revendication 1.

13. Procédé pour lutter contre des organismes nuisibles animaux et/ou une couverture végétale non souhaitée, **caractérisé en ce qu'**on laisse agir des composés de formule (I) selon la revendication 1 sur des organismes nuisibles et/ou leur espace de vie, des procédés de traitement chirurgical ou thérapeutique du corps humain et animal tant exclus.

14. Utilisation de composés de formule (I) selon la revendication 1 pour lutter contre des organismes nuisibles animaux et/ou une couverture végétale non souhaitée, l'utilisation dans des procédés de traitement chirurgical ou thérapeutique du corps humain ou animal tant exclue.

15. Procédé pour la préparation d'agents de lutte contre les organismes nuisibles et/ou d'herbicides, **caractérisé en ce qu'**on mélange des composés de formule (I) selon la revendication 1 avec des agents d'allongement et/ou des substances tensioactives.

16. Agent contenant une teneur active en une combinaison de substances actives comprenant
a') au moins un cétoénol cyclique, substitué de formule (I) selon la revendication 1, dans laquelle CKE, W, X et Y présentent la signification indiquée ci-dessus et
(b') au moins un composé améliorant la tolérance des plantes de culture du groupe suivant de composés :
4-dichloroacétyl-1-oxa-4-azaspiro[4,5]-décane (AD-67, MON-4660), 1-dichloroacétylhexahydro-3,3,8a-triméthylpyrrolo[1,2-a]-pyrimidin-6(2H)-one (Dicyclonon, BAS-145138), 4-dichloroacétyl-3,4-dihydro-3-méthyl-2H-1,4-benzoxazine (Benoxacor), ester 1-méthylhexylique de l'acide 5-chloroquinol in-8-oxyacétique (Cloquintocetmexyl - Cf. également les composés apparentés dans les documents EP A 86 750, EP A 94 349, EP A 191 736, EP A 492 366), 3-(2-chlorobenzyl)-1-(1-méthyl-1-phényl thyl)-urée (Cumyluron), α-(cyanom thoximino)-phénylac tonitrile (Cyometrinil), acide 2,4-dichlorophénoxyacétique (2,4-D), acide 4-(2,4-dichlorophénoxy)-butyrique (2,4-DB), 1-(1-méthyl-1-phényléthyl)-3-(4-méthylphényl)-urée (Daimuron, Dymron), acide 3,6-dichloro-2-méthoxybenzo que (Dicamba), ester S-1-méthyl-1-phényéthylique de l'acide pipéridine-1-thiocarboxylique (Dimepiperate), 2,2-dichloro-N-(2-oxo-2-(2-propénylamino)-éthyl)-N-(2-propényl)-acétamide (DKA-24), 2,2-dichloro-N,N-di-2-propénylacétamide (Dichlormid), 4,6-dichloro-2-phénylpyrimidine (Fenclorim), esteréthylique de l'acide 1-(2,4-dichlorophényl)-5-trichlorométhyl-1H-1,2,4-triazole-3-carboxylique (Fenchloroazole-ethyl - Cf. également les composés apparentés dans les documents EP A 174 562 et EP A 346 620), esteréphénylm thylique de l'acide 2-chloro-4-trifluorom thylthiazole-5-carboxylique (Flurazole), 4-chloro-N-(1,3-dioxolan-2-ylméthoxy)-α-trifluoroacétophénonoxime (Fluxofenim), 3-dichloroacétyl-5-(2-furannyl)-2,2-diméthyloxazolidine (Furilazole, MON-13900), éthyl-4,5-dihydro-5,5-diphényl-3-isoxazolecarboxylate (Isoxadifen-ethyl - Cf. également les composés apparentés dans le document WO A 95/07 897), 3,6-dichloro-2-méthoxybenzoate de 1-( éthoxycarbonyl)- éthyle (Lactidichlor), acide (4-chloro-o-toluyloxy)-acétique (MCPA), acide 2-(4-chloro-o-toluyloxy)-propionique (Mecoprop), 1-(2,4-dichlorophényl)-4,5-dihydro-5-méthyl-1H-pyrazole-3,5-dicarboxylate de diéthyle (Mefenpyr-diethyl - Cf. également les composés apparentés dans le document WO A 91/07 874) 2-dichlorométhyl-2-méthyl-1,3-dioxolane (MG-191), 2-propényl-1-oxa-4-azaspiro[4,5]décane-4-carbodithioate (MG-838), anhydride de l'acide 1,8-naphtalénique, α-(1,3-dioxolan-2-ylméthoximino)-phénylacétonitrile (Oxabetrinil), 2,2-dichloro-N-(1,3-dioxolan-2-ylméthyl)-N-(2-propényl)-acétamide (PPG-1292), 3-dichloroacétyl-2,2-diméthyloxazolidine (R-28725), 3-dichloroacétyl-2,2,5-triméthyloxazolidine (R-29148), acide 4-(4-chloro-o-toluyl)-butyrique, acide 4-(4-chlorophénoxy)-butyrique, acide diphénylméthoxycétique, esteréméthylique de l'acide diphénylméthoxyacétique, ester thylique de l'acide diphénylméthoxyacétique, ester méthylique de l'acide 1-(2-chlorophényl)-5-phényl-1H-pyrazole-3-carboxylique, esteréthylique de l'acide 1-(2,4-dichlorophényl)-5-méthyl-1H-pyrazole-3-carboxylique, esteréthylique de l'acide 1-(2,4-dichlorophényl)-5-isopropyl-1H-pyrazole-3-carboxylique, ester thylique de l'acide 1-(2,4-dichlorophényl)-5-(1,1-diméthyléthyl)-1H-pyrazole-3-carboxylique, esteréthylique de l'acide 1-(2,4-dichlorophényl)-5-phényl-1H-pyrazole-3-carboxylique (Cf. également les composés apparentés dans les documents EP A 269 806 et EP A 333 131), esteré thylique de l'acide 5-(2,4-dichlorobenzyl)-2-isoxazoline-3-carboxylique, esteréthylique de l'acide 5-phényl-2-isoxazoline-3-carboxylique, esteréthylique de l'acide 5-(4-fluorophényl)-5-phényl-2-isoxazoline-3-carboxylique (Cf. également les composés apparentés dans le document WO A 91/08 202), ester 1,3-diméthylbut-1-ylique de l'acide 5-chloroquinol in-8-oxyacétique, ester 4-allyloxybutylique de l'acide 5-chloroquinol ine-8-oxyacétique, ester 1-allyloxyprop-2-ylique de l'acide 5-chloroquinol in-8-oxyacétique, ester méthylique de l'acide 5-chloroquinoxalin-8-oxyacétique, ester thylique de l'acide 5-chloroquinol in-8-oxyacétique, ester allylique de l'acide 5-chloroquinoxalin-8-oxyacétique, ester 2-oxoprop-1-ylique de l'acide 5-chloroquinol in-8-oxyacétique, ester diéthylique de l'acide 5-chloroquinol in-8-oxymalonique, ester diallylique de l'acide 5-chloroquinoxalin-8-oxymalonique, ester diéthylique de l'acide 5-chloroquinol in-8-oxymalonique (Cf. également les composés apparentés dans le document EP A 582 198), acide 4-carboxychroman-4-ylacétique (AC-304415, Cf. le document EP A 613 618), acide 4-chlorophénoxyacétique, 3,3'-diméthyl-4-méthoxybenzophénone, 1-bromo-4-chlorométhylsulfonylbenzène, 1-[4-(N-2-méthoxybenzoylsulfamoyl)-phényl]-3-méthylurée (alias N-(2-méthoxybenzoyl)-4-[(méthylaminocarbonyl)-amino]-benzénesulfonamide), 1-[4-(N-2-méthoxybenzoylsulfamoyl)-phényl]-3,3-diméthylurée, 1-[4-(N-4,5-diméthylbenzoylsulfamoyl)-phényl]-3-méthylurée, 1-[4-(N-naphtylsulfamoyl)-phényl]-3,3-diméthylurée, N-(2-méthoxy-5-méthylbenzoyl)-4-(cyclopropylaminocarbonyl)-benzénesulfonamide,
et/ou un des composés suivants définis par des formules générales de formule générale (IIa) 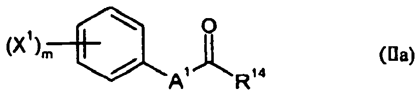 ou de formule générale (IIb) 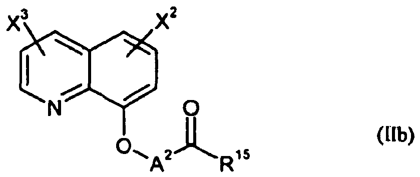 ou de formule (IIc) 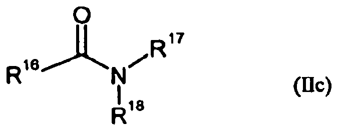
O m représente le nombre 0, 1, 2, 3, 4 ou 5,
A¹ représente un des groupes hétérocycliques divalents esquisses ci-aprés, 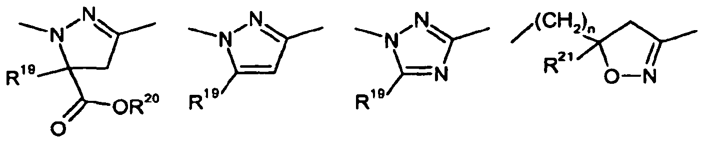
n représente le nombre 0, 1, 2, 3, 4 ou 5,
A² représente alcanediyle, comprenant 1 ou 2 atomes de carbone, le cas échéant substitué par C₁-C₄-alkyle et/ou C₁-C₄-alcoxycarbonyle et/ou C₁-C₄-alcényloxycarbonyle,
R¹⁴ représente hydroxy, mercapto, amino, C₁-C₆-alcoxy, C₁-C₆-alkylthio, C₁-C₆-alkylamino ou di-(C₁-C₄-alkyl)-amino,
R¹⁵ représente hydroxy, mercapto, amino, C₁-C₇-alcoxy, C₁-C₆-alcényloxy, C₁-C₆-alcényloxy-C₁-C₆-alcoxy, C₁-C₆-alkylthio, C₁-C₆-alkylamino ou di-(C₁-C₄-alkyl) - amino,
R¹⁶ représente C₁-C₄-alkyle le cas échéant substitué par fluor, chlore et/ou brome,
R¹⁷ représente hydrogène ; C₁-C₆-alkyle, C₂-C₆-alcényle ou C₂-C₆-alcynyle chaque fois le cas échéant substitué par fluor, chlore et/ou brome ; C₁-C₄-alcoxy-C₁-C₄-alkyle, dioxolanyl-C₁-C₄-alkyle, furyle, furyl-C₁-C₄-alkyle, thiényle, thiazolyle, pipéridinyle ; ou phényle le cas échéant substitué par fluor, chlore et/ou brome ou par C₁-C₄-alkyle,
R¹⁸ représente hydrogène ; C₁-C₆-alkyle, C₂-C₆-alcényle ou C₂-C₆-alcynyle chaque fois le cas échéant substitué par fluor, chlore et/ou brome ; C₁-C₄-alcoxy-C₁-C₄-alkyle, dioxolanyl-C₁-C₄-alkyle, furyle, furyl-C₁-C₄-alkyle, thiényle, thiazolyle, pipéridinyle ; ou phényle le cas échéant substitué par fluor, chlore et/ou brome ou par C₁-C₄-alkyle,
R¹⁷ et R¹⁸ représentent également, ensemble, C₃-C₆-alcanediyle ou C₂-C₅-oxaalcanediyle chaque fois le cas échéant substitué par C₁-C₄-alkyle, phényle, furyle, un cycle benzéne annel ou par deux substituants qui forment, ensemble avec l'atome de carbone auquel ils sont liés, un carbocyclique de 5 ou 6 chaénons,
R¹⁹ représente hydrogéne ; cyano ; halogène ; ou C₁-C₄-alkyle, C₃-C₆-cycloalkyle ou phé nyle chaque fois le cas échéant substitué par fluor, chlore et/ou brome,
R²⁰ représente hydrogène ; C₁-C₆-alkyle, C₃-C₆-cycloalkyle ou tri-(C₁-C₄-alkyl)-silyle chaque fois le cas échéant substitué par hydroxy, cyano, halogène ou C₁-C₄-alcoxy,
R²¹ représente hydrogène ; cyano ; halogène ; ou C₁-C₄-alkyle, C₃-C₆-cycloalkyle ou phé nyle chaque fois le cas échéant substitué par fluor, chlore et/ou brome,
X¹ représente nitro, cyano, halogéne, C₁-C₄-alkyle, C₁-C₄-halogénoalkyle, C₁-C₄-alcoxy ou C₁-C₄-halogénoalcoxy,
X² représente hydrogéne, cyano, nitro, halogène, C₁-C₄-alkyle, C₁-C₄-halog énoalkyle, C₁-C₄-alcoxy ou C₁-C₄-halogénoalcoxy,
X³ représente hydrogène, cyano, nitro, halogène, C₁-C₄-alkyle, C₁-C₄-halogé noalkyle, C₁-C₄-alcoxy ou C₁-C₄-halogénoalcoxy,
et/ou les composés suivants définis par des formules
générales de formule générale (IId) 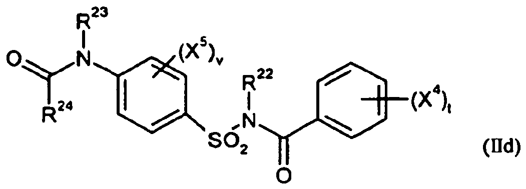
ou de formule générale (IIe) 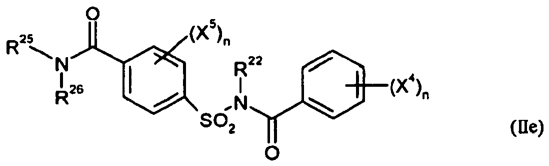
O t représente le nombre 0, 1, 2, 3, 4 ou 5,
v représente le nombre 0, 1, 2, 3, 4 ou 5,
R²² représente hydrogéne ou C₁-C₄-alkyle,
R²³ représente hydrogéne ou C₁-C₄-alkyle,
R²⁴ représente hydrogéne ; C₁-C₆-alkyle, C₁-C₆-alcoxy, C₁-C₆-alkylthio, C₁-C₆-alkylamino ou di- (C₁-C₄-alkyl)-amino chaque fois le cas échéant substitué par cyano, halogène ou C₁-C₄-alcoxy ; ou C₃-C₆-cycloalkyle, C₃-C₆-cycloalkyloxy, C₃-C₆-cycloalkylthio ou C₃-C₆-cycloalkylamino chaque fois substitué par cyano, halogène ou C₁-C₄-alkyle,
R²⁵ représente hydrogène ; C₁-C₆-alkyle le cas échéant substitué par cyano, hydroxy, halogène ou C₁-C₄-alcoxy ; C₃-C₆-alcényle ou C₃-C₆-alcynyle chaque fois le cas échéant substitué par cyano ou halogène ; ou C₃-C₆-cycloalkyle le cas échéant substitué par cyano, halogène ou C₁-C₄-alkyle,
R²⁶ représente hydrogène ; C₁-C₆-alkyle le cas échéant substitué par cyano, hydroxy, halogène ou C₁-C₄-alcoxy ; C₃-C₆-alcényle ou C₃-C₆-alcynyle chaque fois le cas échéant substitué par cyano ou halogène ; C₃-C₆-cycloalkyle le cas échéant substitué par cyano, halogène ou C₁-C₄-alkyle ; ou phényle le cas échéant substitué par nitro, cyano, halogène, C₁-C₄-alkyle, C₁-C₄-halogénoalkyle, C₁-C₄-alcoxy ou C₁-C₄-halogénoalcoxy ; ou, ensemble avec R²⁵, C₂-C₆-alcanediyle ou C₂-C₅-oxaalcanediyle chaque fois le cas échéant substitué par C₁-C₄-alkyle,
X⁴ représente nitro, cyano, carboxy, carbamoyle, formyle, sulfamoyle, hydroxy, amino, halogène, C₁-C₄-alkyle, C₁-C₄-halogénoalkyle, C₁-C₄-alcoxy ou C₁-C₄-halogénoalcoxy, et
X⁵ représente nitro, cyano, carboxy, carbamoyle, formyle, sulfamoyle, hydroxy, amino, halogène, C₁-C₄-alkyle, C₁-C₄-halogénoalkyle, C₁-C₄-alcoxy ou C₁-C₄-halogénoalcoxy.

17. Agent selon la revendication 16, dans lequel le composé améliorant la tolérance des plantes de culture est choisi dans le groupe suivant de composés : Cloquintocetmexyle, Fenchlorazole-ethyl, Isoxadifen-ethyl, Mefenpyr-diethyl, Furilazole, Fenclorim, Cumyluron, Dymron ou les composés IIe-5 ou IIe-11.

18. Procédé pour lutter contre une croissance non souhaitée de végétaux, **caractérisé en ce qu'**on laisse agir un agent selon la revendication 16 sur les végétaux ou leur environnement.

19. Utilisation d'un agent selon la revendication 16 pour lutter contre la croissance non souhaitée de végétaux.

20. Agent selon la revendication 16, dans lequel le composé améliorant la tolérance des plantes de culture est le Cloquintocet-mexyl ou le Mefenpyr-diethyl.
